# EUROPEAN PATENT SPECIFICATION

(11) **EP 4 508 028 B1**
(45) Date of publication and mention of the grant of the patent: **11.03.2026**
(21) Application number: 23720264.3
(22) Date of filing: 14.04.2023
(51) Int. Cl.: C07D 205/04, C07D 207/12, C07D 405/12, C07D 409/12, C07F 5/02, A61P 35/00, A61K 31/40

(54) **NOVEL SUBSTITUTED 4-AMINO-4-OXO-BUT-2-ENYL DERIVATIVES, PROCESSES FOR THEIR PREPARATION AND THERAPEUTIC USES THEREOF**
NEUARTIGE SUBSTITUIERTE 4-AMINO-4-OXO-BUT-2-ENYL-DERIVATE, VERFAHREN ZU IHRER HERSTELLUNG UND THERAPEUTISCHE VERWENDUNGEN DAVON
NOUVEAUX DÉRIVÉS DE 4-AMINO-4-OXO-BUT-2-ENYL SUBSTITUÉS, LEURS PROCÉDÉS DE PRÉPARATION ET LEURS UTILISATIONS THÉRAPEUTIQUES

(30) Priority: 15.04.2022 EP 22305564
(43) Date of publication of application: 19.02.2025
(73) Proprietor: Sanofi, 75017 Paris (FR)
(72) Inventor: EL-AHMAD, Youssef, 75017 Paris (FR); HALLEY, Frank, 75017 Paris (FR); PETIT, Frédéric, 75017 Paris (FR); SLOWINSKI, Franck, 75017 Paris (FR)
(74) Representative: Ipsilon
(86) International application number: PCT/EP2023/059808
(87) International publication number: WO 2023/198901

(56) References cited:
- WO-A1-2017/140669
- WO-A1-2018/091153
- PHILIPP Y. MAXIMOV ET AL: "Influence of the Length and Positioning of the Antiestrogenic Side Chain of Endoxifen and 4-Hydroxytamoxifen on Gene Activation and Growth of Estrogen Receptor Positive Cancer Cells", JOURNAL OF MEDICINAL CHEMISTRY, vol. 57, no. 11, 12 June 2014 (2014-06-12), US, pages 4569 - 4583, XP055667383, ISSN: 0022-2623, DOI: 10.1021/jm500569h

## Description

Disclosed herein are novel substituted 4-amino-4-oxo-but-2-enyl derivatives, the processes for their preparation, as well as the therapeutic uses thereof, in particular as anticancer agents via selective antagonism and degradation of estrogen receptors.

The Estrogen Receptors (ER) belong to the steroid/nuclear receptor superfamily involved in the regulation of eukaryotic gene expression, cellular proliferation and differentiation in target tissues. ERs are in two forms: the estrogen receptor alpha (ERα) and the estrogen receptor beta (ERβ) respectively encoded by the ESR1 and the ESR2 genes. ERα and ERβ are ligand-activated transcription factors which are activated by the hormone estrogen (the most potent estrogen produced in the body is 17β-estradiol). In the absence of hormone, ERs are largely located in the cytosol of the cell. When the hormone estrogen binds to ERs, ERs migrate from the cytosol to the nucleus of the cell, form dimers and then bind to specific genomic sequences called Estrogen Response Elements (ERE). The DNA/ER complex interacts with co-regulators to modulate the transcription of target genes.

ERα is mainly expressed in reproductive tissues such as uterus, ovary, breast, bone and white adipose tissue. Abnormal ERα signaling leads to development of a variety of diseases, such as cancers, metabolic and cardiovascular diseases, neurodegenerative diseases, inflammation diseases and osteoporosis.

ERα is expressed in not more than 10% of normal breast epithelium but approximately 50-80% of breast tumors. Such breast tumors with high level of ERα are classified as ERα-positive breast tumors. The etiological role of estrogen in breast cancer is well established and modulation of ERα signaling remains the mainstay of breast cancer treatment for the majority ERα-positive breast tumors. Currently, several strategies for inhibiting the estrogen axis in breast cancer exist, including: 1- blocking estrogen synthesis by aromatase inhibitors that are used to treat early and advanced ERα-positive breast cancer patients; 2- antagonizing estrogen ligand binding to ERα by tamoxifen which is used to treat ERα-positive breast cancer patients in both pre- and post- menopausal setting; 3-antagonizing and downregulating ERα levels by fulvestrant, which is used to treat breast cancer in patients that have progressed despite endocrine therapies such as tamoxifen or aromatase inhibitors.

Although these endocrine therapies have contributed enormously to reduction in breast cancer development, about more than one-third of ERα-positive patients display de-novo resistance or develop resistance over time to such existing therapies. Several mechanisms have been described to explain resistance to such hormone therapies. For example, hypersensitivity of ERα to low estrogen level in treatment with aromatase inhibitors, the switch of tamoxifen effects from antagonist to agonist effects in tamoxifen treatments or multiple growth factor receptor signaling pathways. Acquired mutations in ERα occurring after initiation of hormone therapies may also play a role in treatment failure and cancer progression. Certain mutations in ERα, particularly those identified in the Ligand Binding Domain (LBD), result in the ability to bind to DNA in the absence of ligand and confer hormone independence in cells harboring such mutant receptors. Further reference may be made to WO2018/091153, WO2017/140669 and Maximov et al., "J. Med. Chem.", 57(11), 2014, pages 4569-4583.

Hence, there is still a need to provide potent compounds suitable as ER-directed therapies.

The inventors have now found novel compounds which are selective estrogen receptors covalent antagonists (SERCA).

Disclosed herein are compounds of the formula (I), or pharmaceutically acceptable salts thereof: 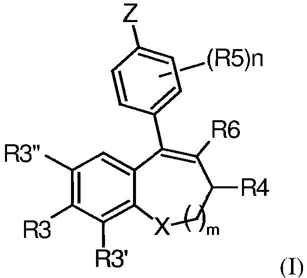 wherein:
- R3 represents a hydrogen atom, a -COOH group or a -OH group;
- R3' and R3" independently represent a hydrogen atom, a methyl group, a methoxy group, a chlorine atom, a fluorine atom or a cyano group;
- R4 represents a hydrogen atom, a (C₁-C₃)alkyl group or a cyclopropyl;
- R5 independently represents a (C₁-C₃)alkyl group such as a methyl group, a halogen atom, such as a fluorine atom, a cyano group, or a (C₁-C₃)fluoroalkyl group, such as a trifluoromethyl;
- R6 represents a group selected from:
   ▪ a phenyl group, said phenyl group being optionally substituted by 1 to 3 substituents independently selected from a halogen atom; a (C₁-C₆)alkyl group, optionally substituted with a cyano group or a -OH group; a (C₁-C₆)fluoroalkyl group; a (C₃-C₆)cycloalkyl group; a (C₁-C₆)alkoxy group; a (C₁-C₆)fluoroalkoxy group; a cyano group; a trifluoromethylsulfonyl group; a (C₁-C₄)alkylthio group; a (C₁-C₄)fluoroalkylthio group; a (C₁-C₄)alkylsulfonyl group and a -OH group;
   ▪ a fused phenyl group, selected from phenyl groups fused with a (C₃-C₆)cycloalkyl, which (C₃-C₆)cycloalkyl ring optionally comprises an unsaturation and, wherein the fused phenyl group is optionally substituted with 1 to 3 substituents independently selected from a (C₁-C₃)alkyl group, a hydroxy group, a halogen atom, a (C₁-C₆)fluoroalkyl group and a (C₁-C₃)alkoxy group;
   ▪ a bicyclic group comprising 5 to 12 carbon atoms, optionally comprising 1 to 2 unsaturations; optionally substituted with 1 to 4 substituents independently selected from: a fluorine atom, a -OH group, a (C₁-C₃)alkyl group, a (C₁-C₃)fluoroalkyl group, a (C₁-C₃)alkoxy group, a (C₁-C₃)fluoroalkoxy group and an oxo group;
   ▪ a heteroaryl group comprising 2 to 9 carbon atoms and comprising from 1 to 3 heteroatoms independently selected from oxygen, nitrogen and sulfur, and at least 5 atoms including carbon atoms and heteroatoms, such as a pyridyl group or a pyrrolyl group, said heteroaryl group being optionally substituted with 1 to 3 substituents independently selected from a halogen atom, a (C₁-C₆)alkyl group, a (C₁-C₆)fluoroalkyl group, a (C₁-C₆)alkoxy group, a (C₁-C₆)fluoroalkoxy group, a cyano group, a carbamoyl group and a -OH group;
   ▪ a cycloalkyl group comprising 3 to 7 carbon atoms, said cycloalkyl group being saturated or partially saturated and being optionally substituted with 1 to 4 substituents independently selected from:
      ∘ a fluorine atom, a -OH group, a (C₁-C₃)alkyl group, a (C₁-C₃)fluoroalkyl group, a (C₁-C₃)alkoxy group, a (C₁-C₃)fluoroalkoxy group, an oxo group,
      ∘ a (C₃-C₆)cycloalkyl group, and a phenyl group, said (C₃-C₆)cycloalkyl or phenyl groups being optionally substituted with one or two halogen atom(s) or (C₁-C₃)alkyl group(s);
   ▪ a (C₃-C₆)cycloalkyl(C₁-C₃)alkyl group, optionally substituted on the cycloalkyl with 1 to 4 substituents independently selected from: a fluorine atom, a -OH group, a (C₁-C₄)alkyl group, a (C₁-C₃)fluoroalkyl group, a (C₁-C₃)fluoroalkoxy group and an oxo group;
   ▪ a 4 to 7 membered-heterocycloalkyl group comprising 1 or 2 heteroatoms independently selected from oxygen, nitrogen and sulfur, such as a tetrahydropyranyl or a tetrahydrofuranyl group, said heterocycloalkyl group being saturated or partially saturated and being optionally substituted with 1 to 3 substituents independently selected from: a fluorine atom, a (C₁-C₃)alkyl group, a (C₁-C₃)fluoroalkyl group, a (C₁-C₃)fluoroalkoxy group, an oxo group, a (C₁-C₃)alkoxy group, and a -OH group;
   ▪ a (C₁-C₆)alkyl group, such as an isobutyl group or an ethylbutyl group, said alkyl group being optionally substituted with 1 to 4 substituents independently selected from: a fluorine atom, a (C₁-C₃)alkoxy group, a (C₁-C₃)fluoroalkoxy group and a -OH group; and
   ▪ a phenyl(C₁-C₂)alkyl group, said phenyl group being optionally substituted with 1 to 3 substituents independently selected from a halogen atom; a (C₁-C₃)alkyl group; a (C₁-C₃)fluoroalkyl group; a (C₁-C₃)alkoxy group; a (C₁-C₃)fluoroalkoxy group; a cyano group; and a -OH group;
- X represents -CH₂-, -O- or -S-;
- n is 0, 1 or 2;
- m is 0 or 1; and
- Z represents a group selected from:
   ▪ 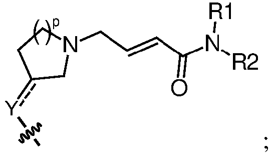 and
   ▪ 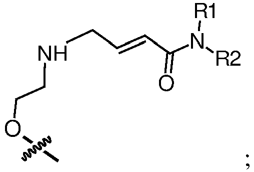 wherein:
      o R1 and R2 independently represent a hydrogen atom, a -CH₃ group, a -CH₂CH₃ group, or a -CH₂CH₂OH group, or R1 and R2 forms a 4 to 6 membered-heterocycloalkyl group with the nitrogen atom to which R1 and R2, are attached, said heterocycloalkyl group optionally comprising an additional heteroatom selected from oxygen, nitrogen and sulfur;
      o Y represents -CH₂-, -CH=, -CR7=, -O- or -NH-, wherein R7 represents a fluorine atom or a (C₁-C₃)alkyl group;
      ∘ 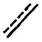 represents a single bond or a double bond; and
      o p is 0 or 1.

Y represents -CH₂-, -O- or -NH- when 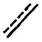 represents a single bond and Y represents -CH= or -CR7= when 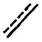 represents a double bond.

The compounds of formula (I) can contain one or more asymmetric carbon atoms. They may therefore exist in the form of enantiomers.

The compounds of formula (I) may be present as well under tautomer forms.

The compounds of formula (I) may exist in the form of bases, acids, zwitterion or of addition salts with acids or bases. Hence, herein are provided compounds of formula (I) or pharmaceutically acceptable salts thereof.

These salts may be prepared with pharmaceutically acceptable acids or bases, although the salts of other acids or bases useful, for example, for purifying or isolating the compounds of formula (I) are also provided.

Among suitable salts of the compounds of formula (I), hydrochloride may be cited.

As used herein, the terms below have the following definitions unless otherwise mentioned throughout the instant specification:
- a halogen atom: a fluorine, a chlorine, a bromine or an iodine atom, and in particular a fluorine and a chlorine atom;
- an oxo: a "=O" group;
- an alkyl group: a linear or branched saturated hydrocarbon-based aliphatic group comprising, unless otherwise mentioned, from 1 to 6 carbon atoms (noted "(C₁-C₆)alkyl"). By way of examples, mention may be made of, but not limited to: methyl, ethyl, propyl, isopropyl, butyl, isobutyl, sec-butyl, tert-butyl, pentyl, isopentyl, hexyl and isohexyl groups, and the like;
- a cycloalkyl group: a monocyclic alkyl group comprising, unless otherwise mentioned, from 3 to 7 carbon atoms, saturated or partially unsaturated and unsubstituted or substituted. By way of examples, mention may be made of, but not limited to: cyclopropyl, cyclobutyl, cyclopentyl, cyclobutenyl, cyclopentenyl, cyclohexyl, cyclohexenyl, cycloheptyl, cycloheptenyl groups and the like, in particular a cyclopentyl, a cyclohexyl, a cycloheptyl, a cycloheptenyl, or a cyclohexenyl;
- a heterocycloalkyl group: a 4 to 7-membered cycloalkyl group, saturated or partially unsaturated, comprising 1 to 2 heteroatoms independently selected from oxygen, nitrogen and sulfur, in particular being oxygen or nitrogen. By way of examples, mention may be made of, but not limited to: morpholinyl, piperazinyl, piperidinyl, pyrrolidinyl, aziridinyl, oxanyl, oxetanyl, tetrahydropyranyl, morpholinyl, tetrahydrofuranyl, oxepanyl, diazepanyl, dioxanyl, tetrahydropyranyl, and tetrahydrothiopyranyl. The heterocycloalkyl is advantageously tetrahydrofuranyl or tetrahydropyranyl.
- a fluoroalkyl group: an alkyl group as previously defined where the alkyl group is substituted with at least one fluorine atom. In other terms, at least one hydrogen atom of the alkyl group is replaced by a fluorine atom. By way of example, mention may be made of -CH₂F, -CHF₂, -CH₂CHF₂, -CH₂CH₂F and the like. When all the hydrogen atoms belonging to the alkyl group are replaced by fluorine atoms, the fluoroalkyl group can be named perfluoroalkyl group. By way of example, mention may be made of trifluoromethyl group or trifluoroethyl group and the like;
- an alkoxy group: an -O-alkyl group where the alkyl group is as previously defined. By way of examples, mention may be made of, but not limited to: methoxy, ethoxy, propoxy, isopropoxy, linear, secondary or tertiary butoxy, isobutoxy, pentoxy or hexoxy groups, and the like;
- a fluoroalkoxy group: an -O-alkyl group where the alkyl group is as previously defined and where the alkyl group is substituted with at least one fluorine atom. In other terms, at least one hydrogen atom of the alkyl group is replaced by a fluorine atom. By way of example, mention may be made of -OCH₂F, -OCHF₂, -OCH₂CH₂F and the like. When all the hydrogen atoms belonging to the alkyl group are replaced by fluorine atoms, the fluoroalkoxy group can be named perfluoroalkoxy group. By way of example, mention may be made of trifluoromethoxy group and the like;
- a (C₁-C₄)alkylthio group also named a (C₁-C₄)alkylsulfanyl group: a -S-alkyl group where the alkyl group is as previously defined. By way of examples, mention may be made of, but not limited to: methylthio, ethylthio, propylthio, isopropylthio, linear, secondary or tertiary butylthio, isobutylthio, and the like;
- a (C₁-C₄)alkylsulfonyl group: a -SO₂-alkyl group where the alkyl group is as previously defined. By way of examples, mention may be made of, but not limited to -SO₂CH₃, -SO₂CH₂CH₃ and the like;
- a (C₁-C₄)fluoroalkylthio group also named a (C₁-C₄)fluoroalkylsulfanyl group: a -S-fluoroalkyl group where the fluoroalkyl group is as previously defined. By way of examples, mention may be made of, but not limited to: fluoromethylthio, difluoromethylthio, trifluoromethylthio and the like;
- a fused phenyl group: a bicyclic radical comprising from 7 to 10 carbon atoms and that contains a phenyl moiety. Said phenyl moiety may be fused to a (C₃-C₆)cycloalkyl group, i.e. the phenyl moiety may share a bond with said (C₃-C₆)cycloalkyl group. The fused phenyl group may be bound to the rest of the molecule by its phenyl moiety. It may be substituted. Examples are, but are not limited to indanyl, bicyclo[4.2.0] octa-1(6),2,4-trienyl, tetrahydronaphthalenyl and the like;
- a heteroaryl group: a cyclic 5 to 10-membered aromatic group containing between 2 and 9 carbon atoms and containing between 1 and 3 heteroatoms, such as nitrogen, oxygen or sulfur. Such nitrogen atom may be substituted with an oxygen atom in order to form a -NO bond. Such -N-O bond can be in a form of a N-oxide (-N⁺-O⁻). Said heteroaryl group may be monocyclic or bicyclic. By way of examples of heteroaryl groups, mention may be made of, but not limited to: thiophene, furan, thiadiazole, thiazole, imidazole, pyridazine, triazine, pyrazine, oxadiazole, pyrazole, isothiazole, oxazole, isoxazole, pyridine, pyrimidine, benzotriazole, benzoxazole, pyrrolo[2,3-b]pyridine, benzimidazole, benzoxadiazole, benzothiazole, benzothiadiazole, benzofuran, indole, isoquinoline, indazole, benzisoxazole, benzisothiazole, pyridone groups and the like. The heteroaryl group is advantageously pyridine, pyrrole, imidazole, pyrazine, furane, thiazole, pyrazole, thiadiazole, pyridazine, pyridone and pyrimidine, and more particularly pyridine, pyridone and pyrrole;
- a bicyclic group, generally comprising 5 to 12 carbon atoms, is a hydrocarbon group selected from groups comprising two rings connected through:
   - a single common atom: a "spirobicyclic ring". Such spiro bicyclic alkyl generally comprises 5 to 11 carbon atoms referring to a "spiro(C₅-C₁₁)bicyclic ring". The rings may be saturated or partially unsaturated. Such spirobicyclic ring may be unsubstituted or substituted, in particular by at least one (C₁-C₃)alkyl group such as methyl or a fluorine. By way of examples of spiro(C₅-C₁₁)bicyclic ring as for the definition of R6, mention may be made of, but not limited to: spiro[2.3]hexane, spiro[3.3]heptane, spiro[3.3]heptene, spiro[2.5]octane and 7-azaspiro[3.5]nonane. The spiro(C₅-C₁₁)bicyclic ring is advantageously spiro[3.3]heptane or spiro[3.3]heptene still for the R6 group.
   - two common atoms: In that case the bicyclic group comprises 7 to 12 carbon atoms and optionally comprises 1 to 2 unsaturations. By way of examples of such bicyclic groups, mention may be made of, but not limited to: cis-1,3a,4,5,6,6a-hexahydropentalenyl group, bicyclo[3.1.0]hexan-1-yl, bicyclo[4.1.0]heptanyl and octahydropentalenyl.
   - three or more common atoms: In that case the bicyclic group comprises 6 to 10 carbon atoms, such bicyclic group may be referred to as a "bridged (C₆-C₁₀)cycloalkyl" group, the rings share three or more atoms and the bridge contains at least one atom, for example 1, 2 or 3 atoms and preferentially 1 atom. By way of examples of such bridged cycloalkyl groups, mention may be made of, but not limited to bicyclo[3.2.1]octan-3-yl and bicyclo[2.2.1]heptan-2-yl.
- a zwitterion means: a globally neutral molecule with a positive and a negative electrical charge and having an acidic group and a basic group.

In another embodiment, in the compounds of formula (I) as defined above, Z represents a group:

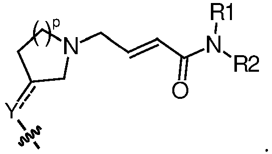

According to this embodiment, the compounds of formula (I) as defined above are of formula (I'):

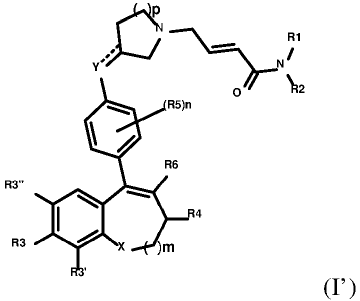

In another embodiment, in the compounds of formula (I) or (I') as defined above, Y represents -CH₂-, -CH=, -O- or -NH-.

In another embodiment, in the compounds of formula (I) or (I') as defined above, Y represents -O-.

In another embodiment, in the compounds of formula (I) or (I') as defined above, Y represents -CH₂-.

In another embodiment, in the compounds of formula (I) or (I') as defined above, Y represents -CH=.

In another embodiment, in the compounds of formula (I) or (I') as defined above, Y represents -NH-.

In another embodiment, in the compounds of formula (I) or (I') as defined above, 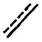 represents a single bond.

In another embodiment, in the compounds of formula (I) or (I') as defined above, 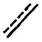 represents a double bond.

In another embodiment, in the compounds of formula (I) or (I') as defined above, p is 1.

In another embodiment, in the compounds of formula (I) or (I') as defined above, p is 0.

In another embodiment, in the compounds of formula (I) as defined above, Z represents a group:

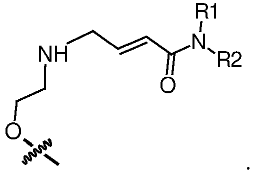

According to this embodiment, the compounds of formula (I) as defined above are of formula (I"):

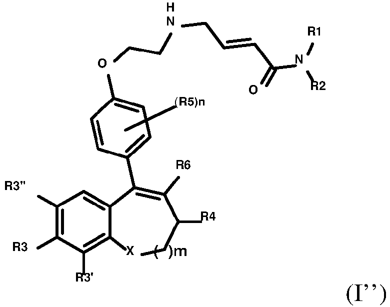

In another embodiment, in the compounds of formula (I), (I') or (I") as defined above, R1 and R2 are a -CH₃ group.

In another embodiment, in the compounds of formula (I), (I') or (I") as defined above, R3 is a -COOH group.

In another embodiment, in the compounds of formula (I), (I') or (I") as defined above, R3 is a -OH group.

In another embodiment, in the compounds of formula (I), (I') or (I") as defined above, R3 is a hydrogen atom.

In another embodiment, in the compounds of formula (I), (I') or (I") as defined above, R3' and R3" represent a hydrogen atom.

In another embodiment, in the compounds of formula (I), (I') or (I") as defined above, X represents -CH₂-.

In another embodiment, in the compounds of formula (I), (I') or (I") as defined above, X represents -O-.

In another embodiment, in the compounds of formula (I), (I') or (I") as defined above, X represents -S-.

In another embodiment, in the compounds of formula (I), (I') or (I") as defined above, R4 represents a hydrogen atom.

In another embodiment, in the compounds of formula (I), (I') or (I") as defined above, n is 0.

In another embodiment, in the compounds of formula (I), (I') or (I") as defined above, m is 1.

In another embodiment, in the compounds of formula (I), (I') or (I") as defined above, m is 0.

In another embodiment, in the compounds of formula (I), (I') or (I") as defined above, R6 represents:
▪ a phenyl group, said phenyl group being optionally substituted by 1 to 3 substituents independently selected from a halogen atom; a (C₁-C₆)alkyl group, optionally substituted with a cyano group or a -OH group; a (C₁-C₆)fluoroalkyl group; a (C₃-C₆)cycloalkyl group; a (C₁-C₆)alkoxy group; a (C₁-C₆)fluoroalkoxy group; a cyano group; a trifluoromethylsulfonyl group; a (C₁-C₄)alkylthio group; a (C₁-C₄)fluoroalkylthio group; a (C₁-C₄)alkylsulfonyl group and a -OH group; or
▪ a heteroaryl group comprising 2 to 9 carbon atoms and comprising from 1 to 3 heteroatoms independently selected from oxygen, nitrogen and sulfur, and at least 5 atoms including carbon atoms and heteroatoms, such as a pyridyl group or a pyrrolyl group, said heteroaryl group being optionally substituted with 1 to 3 substituents independently selected from a halogen atom, a (C₁-C₆)alkyl group, a (C₁-C₆)fluoroalkyl group, a (C₁-C₆)alkoxy group, a (C₁-C₆)fluoroalkoxy group, a cyano group, a carbamoyl group and a -OH group.

In another embodiment, in the compounds of formula (I) , (I') or (I") as defined above, R6 represents a phenyl group, said phenyl group being optionally substituted by 1 to 3 substituents independently selected from a halogen atom; a (C₁-C₆)alkyl group, optionally substituted with a cyano group or a -OH group; a (C₁-C₆)fluoroalkyl group; a (C₃-C₆)cycloalkyl group; a (C₁-C₆)alkoxy group; a (C₁-C₆)fluoroalkoxy group; a cyano group; a trifluoromethylsulfonyl group; a (C₁-C₄)alkylthio group; a (C₁-C₄)fluoroalkylthio group; a (C₁-C₄)alkylsulfonyl group and a -OH group.

In another embodiment, in the compounds of formula (I), (I') or (I") as defined above, R6 represents a phenyl group, said phenyl group being optionally substituted with 1 or 2 substituents independently selected from a chlorine atom, a fluorine atom and a methyl group.

In another embodiment, in the compounds of formula (I), (I') or (I") as defined above, R6 represents a phenyl group.

In another embodiment, in the compounds of formula (I), (I') or (I") as defined above, R6 represents a phenyl group, said phenyl group being substituted with 1 or 2 substituents independently selected from a chlorine atom, a fluorine atom, and a methyl group.

In another embodiment, in the compounds of formula (I), (I') or (I") as defined above, R6 represents heteroaryl group comprising 2 to 9 carbon atoms and comprising from 1 to 3 heteroatoms independently selected from oxygen, nitrogen and sulfur, and at least 5 atoms including carbon atoms and heteroatoms, such as a pyridyl group or a pyrrolyl group, said heteroaryl group being optionally substituted with 1 to 3 substituents independently selected from a halogen atom, a (C₁-C₆)alkyl group, a (C₁-C₆)fluoroalkyl group, a (C₁-C₆)alkoxy group, a (C₁-C₆)fluoroalkoxy group, a cyano group, a carbamoyl group and a -OH group.

In another embodiment, in the compounds of formula (I), (I') or (I") as defined above, R6 represents a pyridyl group, said pyridyl group being substituted by two substituents independently selected from a halogen atom and a (C₁-C₆)alkoxy group, and more particularly selected from a fluorine atom and a methoxy group.

In another embodiment, in the compounds of formula (I) or (I'), R3 is a -COOH group and R6 is a phenyl group substituted with 1 or 2 substituents independently selected from a chlorine atom, a fluorine atom, and a methyl group. In such embodiment, R3' and R3" are in particular hydrogen atoms. Still in such embodiment, R4 is a hydrogen atom, X is a -CH₂- group, m is equal to 1 and n is equal to 0. In such embodiment, R1 and R2 are a -CH₃ group, Y is -O-, 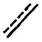 represents a single bond, and p is equal to 1.

In another embodiment, in the compounds of formula (I) or (I"), R3 is a -COOH group, R6 is a phenyl group substituted with 1 or 2 substituents independently selected from a chlorine atom, a fluorine atom and a methyl group. In such embodiment, R3' and R3" are in particular hydrogen atoms. Still in such embodiment, R4 is a hydrogen atom, X is a -CH₂-group, m is equal to 1 and n is equal to 0. In such an embodiment, R1 and R2 are a -CH₃ group.

Among the compounds of formula (I), (I') or (I") described herein, mention may be made in particular of the following compounds or a pharmaceutically acceptable salt thereof, in particular hydrochloride salt thereof:
- (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7] annulene-3-carboxylic acid, (1)
- (S,E)-8-(2-chloro-4-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (2)
- (S,E)-8-(4-chloro-2-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (3)
- (S,E)-8-(2-chloro-3-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (4)
- (S,E)-9-(4-((1-(4-(dimethylamino)-4-oxobut-2-en-1-yl)pyrrolidin-3-yl)oxy)phenyl)-8-(2-fluoro-4-methylphenyl)-6,7-dihydro-5H-benzo[7]annulene-3-carboxylic acid, (5)
- (S,E)-9-(4-((1-(4-(dimethylamino)-4-oxobut-2-en-1-yl)pyrrolidin-3-yl)oxy)phenyl)-8-phenyl-6,7-dihydro-5H-benzo[7]annulene-3-carboxylic acid, (6)
- (S,E)-8-(3-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (7)
- (S,E)-9-(4-((1-(4-(dimethylamino)-4-oxobut-2-en-1-yl)pyrrolidin-3-yl)oxy)phenyl)-8-(2,4-dimethylphenyl)-6,7-dihydro-5H-benzo[7]annulene-3-carboxylic acid, (8)
- (S,E)-8-(2-chlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (9)
- (S,E)-9-(4-((1-(4-(dimethylamino)-4-oxobut-2-en-1-yl)pyrrolidin-3-yl)oxy)phenyl)-8-(2-fluoro-6-methoxypyridin-3-yl)-6,7-dihydro-5H-benzo[7]annulene-3-carboxylic acid, (10)
- (S,E)-8-(2-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (11)
- (E)-8-(2,4-dichlorophenyl)-9-(4-((1-(4-(dimethylamino)-4-oxobut-2-en-1-yl)azetidin-3-yl)oxy)phenyl)-6,7-dihydro-5H-benzo[7]annulene-3-carboxylic acid, (12)
- (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylic acid compound with 2,2,2-trifluoroacetic acid, (13)
- (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylic acid, (14)
- (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (15)
- (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid, (16)
- (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid, (17)
- (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 1, (18)
- (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 2, (19)
- 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylic acid, (20)
- (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylic acid, (21)
- (E)-8-(2,4-dichlorophenyl)-9-(4-(2-((4-(dimethylamino)-4-oxobut-2-en-1-yl)amino)ethoxy)phenyl)-6,7-dihydro-5H-benzo[7]annulene-3-carboxylic acid, (22)
- (S,E)-4-(3-(4-(8-(2,4-dichlorophenyl)-3-hydroxy-6,7-dihydro-5H-benzo[7]annulen-9-yl)phenoxy)pyrrolidin-1-yl)-N,N-dimethylbut-2-enamide, (23)
- (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylic acid, (24)
- (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylic acid, (25)
- (S,E)-4-(3-(4-(8-(2,4-dichlorophenyl)-6,7-dihydro-5H-benzo[7]annulen-9-yl)phenoxy)pyrrolidin-1-yl)-N,N-dimethylbut-2-enamide, (26)
- (S,E)-4-(3-(4-(4-(2,4-dichlorophenyl)-8-hydroxy-2,3-dihydrobenzo[b]thiepin-5-yl)phenoxy)pyrrolidin-1-yl)-N,N-dimethylbut-2-enamide, (27).

Another embodiment is a compound selected from the above list, or a pharmaceutically acceptable salt thereof, for use in therapy, especially as an inhibitor and degrader of estrogen receptors.

Another embodiment is a compound selected from the above list, or a pharmaceutically acceptable salt thereof, for use in the treatment of cancer, especially breast cancer.

Another embodiment is a pharmaceutical composition comprising as active principle an effective dose of a compound selected from the above list, or a pharmaceutically acceptable salt thereof, and also at least one pharmaceutically acceptable excipient.

The compounds of the formula (I), (I') or (I") can be prepared by the following processes.

The compounds of the formula (I), (I') or (I") and other related compounds having different substituents are synthesized using techniques and materials described below or otherwise known by the skilled person in the art. In addition, solvents, temperatures and other reaction conditions presented below may vary as deemed appropriate to the skilled person in the art.

General below methods for the preparation of compounds of formula (I), (I') or (I") optionally modified by the use of appropriate reagents and conditions for the introduction of the various moieties found in the formula (I), (I') or (I") as described below.

The following abbreviations and empirical formulae are used:
- BAST: Deoxo-Fluor
- BOC₂O: di-ter-butyldicarbonate
- Cs₂CO₃: Cesium carbonate
- CO: Carbon monoxide
- DBU: 1,8-Diazabicyclo[5.4.0]undec-7-ene
- DCM: Dichloromethane
- DIEA: Diisopropylethylamine
- DMF: N,N-dimethylformamide
- DMAP: Dimethylaminopyridine
- DMSO: Dimethyl sulfoxide
- Et₂O: Diethyl ether
- EtOAc: Ethyl acetate
- EtOH: Ethanol
- H₂: Hydrogen
- HATU: 2-(3H-[1,2,3]triazolo[4,5-b]pyridin-3-yl)-1,1,3,3-tetramethylisouronium hexafluorophosphate(V)
- HBTU: (2-(1H-benzotriazol-1-yl)-1,1,3,3-tetramethyluronium hexafluorophosphate
- HCl: Hydrochloric acid
- HPLC: High performance liquid chromatography
- K₂CO₃: Potassium carbonate
- KHMDS: Potassium bis(trimethylsilyl)amide
- KOAc: Potassium acetate
- KOH: Potassium hydroxide
- KOPh: Potassium phenolate
- LiHMDS: Lithium bis(trimethylsilyl)amide
- LiOH: Lithium hydroxide
- MeCN: Acetonitrile
- MeOH: Methanol
- MeTHF: 2-Methyltetrahydrofuran
- MgSO₄: MgSO₄
- MTBE: Methyl tert-butyl ether
- n-BuLi: n-Butyllithium
- Na₂SO₄: Sodium sulfate
- NaBH₄: Sodium borohydride
- NaCl: Sodium chloride
- NaH: Sodium hydride
- NaHCO₃: Sodium bicarbonate
- NaHSO₃: Sodium bisulfate
- NaOH: Sodium hydroxide
- NH₄Cl: Ammonium chloride
- NH₄OH: Ammonium hydroxide
- Pd₂(dba)₃: tris(dibenzylideneacetone)dipalladium(0)
- Pd/C: Palladium on carbon
- Pd(dppf)Cl₂: [1,1'-Bis(diphenylphosphino)ferrocene]dichloropalladium(II)
- Pd(OAc)₂: Palladium acetate
- Pd(PPh₃)₂Cl₂: bis (triphenylphosphine) palladium(II) dichloride
- PhOK: Potassium phenolate
- PPh₃: Triphenylphosphine
- RT: Room temperature
- SCX: Strong cation exchange
- SFC: Supercritical Fluid Chromatography
- TEA: Triethylamine
- TFA: Trifluoroacetic acid
- THF: Tetrahydrofuran
- XANTPHOS: (9,9-dimethyl-9H-xanthene-4,5-diyl)bis(diphenylphosphane)

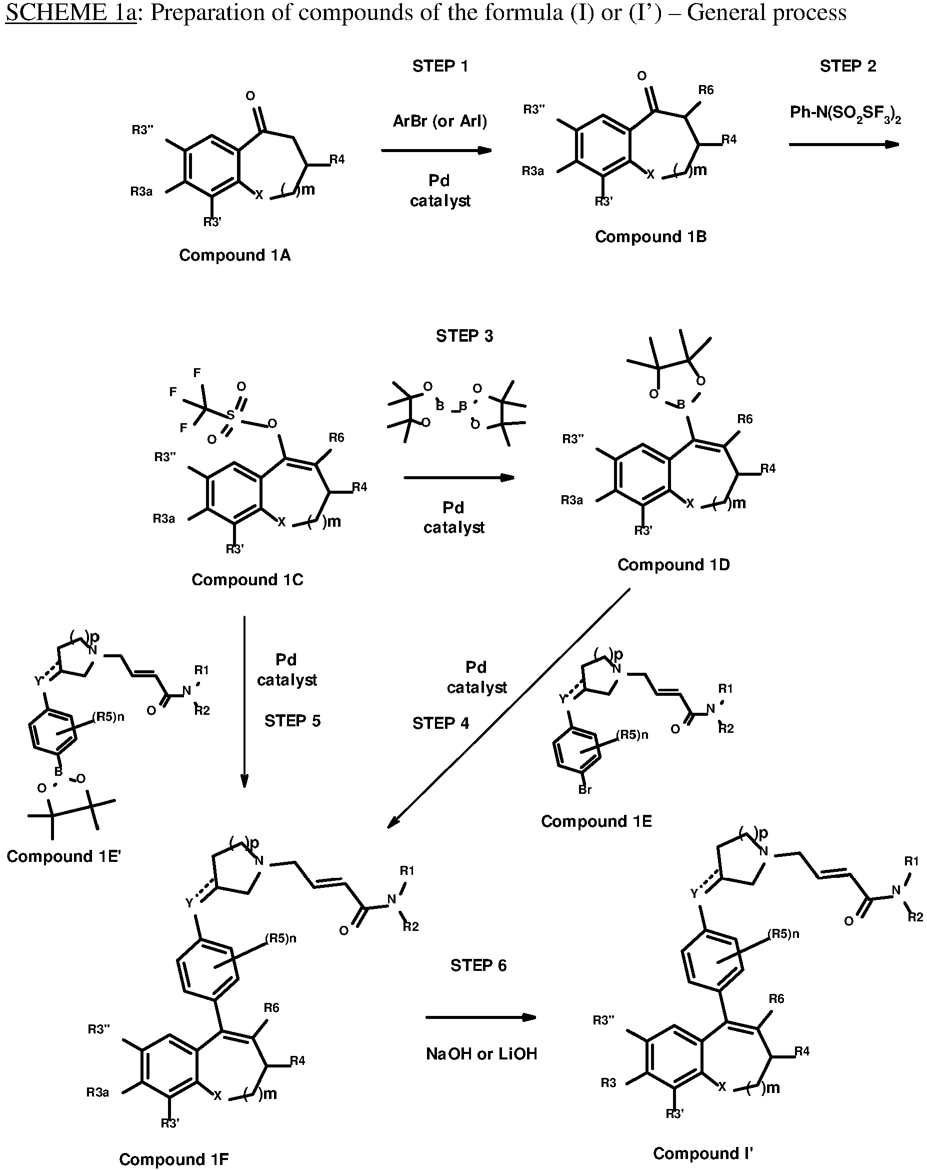

According to SCHEME 1a, in which R3a is a hydrogen atom or a carboxylic ester such as -COOMe, -COOEt, or protected -OH such as O-pivaloyl for example, R1, R2, R3, R3', R3", R4, R5, X, Y, m, n, p and 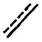 are defined as described above, and R6 is a phenyl or heteroaryl group, compound 1A can be converted in STEP 1 to compound 1B by treatment with aryl or heteroaryl bromide or iodide in the presence of a palladium catalyst, for example tris(dibenzylideneacetone)dipalladium(0) Pd₂(dba)₃, and a phosphine such as (9,9-dimethyl-9H-xanthene-4,5-diyl)bis(diphenylphosphane) (XANTPHOS) in solution in toluene by heating up to reflux of solvent, in presence of a base such as K₂CO₃ or Cs₂CO₃. Alternative way to prepare compound 1B, wherein R6 can be any of the groups defined above for R6 in formula (I) or (I'), is described in SCHEME 1f below.

Compound 1B can be converted in STEP 2 to compound 1C by treatment with N,N-bis(trifluoromethylsulfonyl)aniline in the presence of a base such as DBU or NaH, or KHMDS at -50°C, in a solvent such as MeTHF.

Compound 1C can be converted in STEP 3 to compound 1D by treatment for example with bis(pinacolato)diboron, and with a palladium catalyst, for example bis (triphenylphosphine)palladium(II) dichloride Pd(PPh₃)₂Cl₂, and a phosphine, such as triphenylphosphine, in solution in toluene by heating up to reflux of solvent, in presence of a base such as KOPh.

Compound 1F can be prepared in a Suzuki coupling reaction either between compound 1D and compound 1E in STEP 4 or between compounds 1C and compound 1E' in STEP 5 using for example [1,1'-bis(diphenylphosphino)ferrocene]dichloropalladium(II) (Pd(dppf)Cl₂), complex with DCM, as catalyst, in a mixture of dioxane and water and in the presence of a base, for example cesium carbonate (Cs₂CO₃), by heating up to reflux of solvent.

When R3a is -COOMe, -COOEt, or a protected -OH such as O-pivaloyl, deprotection can be performed in STEP 6 by treatment with an aqueous solution of sodium hydroxide (NaOH) 2N or lithium hydroxide (LiOH) in THF or dioxane. When R3 is -COOH, extraction of the product could give the sodium or lithium salt of compound of formula (I) or (I'). The acidification with an aqueous solution of HCl 2N to pH 6-7 could give the neutral form of compound of formula (I) or (I'). The acidification with an aqueous solution of HCl 2N to pH 1-2 could give the hydrochloride salt of compound (I) or (I'). The purification using HPLC in presence of formic acid or trifluoroacetic acid in the eluent could give the formate or trifluoroacetate salt of compound (I) or (I').

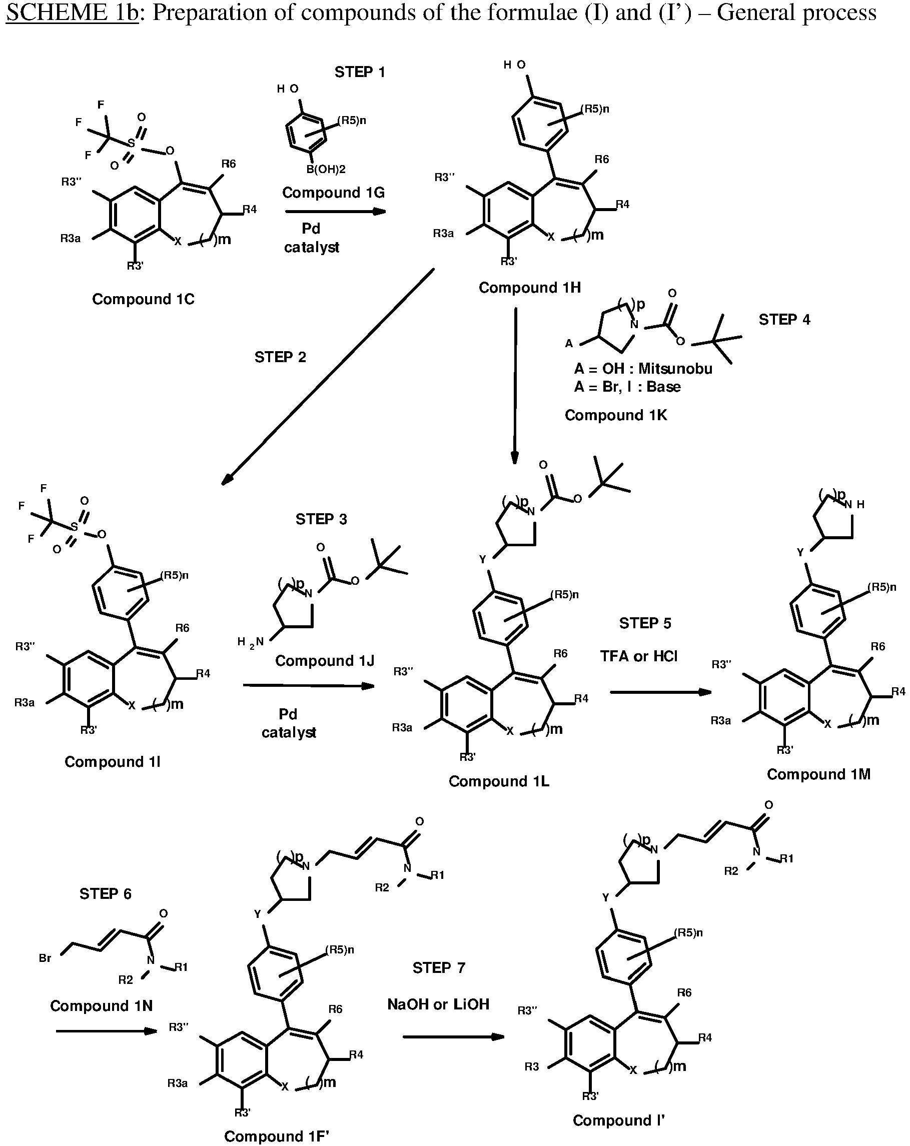

According to SCHEME 1b in which R3a is a hydrogen atom or a carboxylic ester such as -COOMe, -COOEt, or protected -OH such as O-pivaloyl for example, and R1, R2, R3, R3', R3", R4, R5, R6, X, Y, m, n, and p are defined as described above, compound 1C can be converted in STEP 1 to compound 1H by treatment with compound 1G using for example [1,1'-bis(diphenylphosphino)ferrocene]dichloropalladium(II) (Pd(dppf)Cl₂), complex with DCM, as catalyst, in a mixture of dioxane and water and in the presence of a base, for example cesium carbonate (Cs₂CO₃), by heating up to reflux of solvent.

Compound 1H can be converted in STEP 2 to compound 1I by treatment with trifluoromethanesulfonic anhydride in the presence of pyridine in DCM.

Compound 1I can be converted in STEP 3 to compound 1L (Y = -NH-) in a Buchwald coupling reaction with compound 1J in dioxane at 130°C using for example xantphos / palladium acetate (Pd(OAc)₂) as catalytic system.

Compound 1H can be converted in STEP 4 to compound 1L (Y = -O-) by reaction with compound 1K (A = Br or I) in presence of a base such as Cs₂CO₃ in DMF or MeCN, or under Mitsunobu conditions, when (A = -OH), using for example N,N,N',N'-tetramethylazodicarboxamide and triphenylphosphine as coupling agents in THF as a solvent.

Compound 1L can be converted in STEP 5 to compound 1M by treatment with TFA in DCM or HCl in dioxane.

Compound 1M can be converted in STEP 6 to compound 1F' by treatment with compound 1N, in presence of a base such as DIEA in DMF or MeCN.

When R3a is -COOMe, -COOEt, or a protected -OH such as O-pivaloyl, deprotection can be performed in STEP 7 by treatment with an aqueous solution of sodium hydroxide (NaOH) 2N or lithium hydroxide (LiOH) in THF or dioxane. When R3 is -COOH, extraction of the product can give the sodium or lithium salt of compound of formula (I) or (I'). The acidification with an aqueous solution of HCl 2N to pH 6-7 can give the neutral form of compound of formula (I) or (I'). The acidification with an aqueous solution of HCl 2N to pH 1-2 can give the hydrochloride salt of compound of formula (I) or (I'). The purification using HPLC in presence of formic acid or trifluoroacetic acid in the eluent can give the formate or trifluoroacetate salt of compound of formula (I) or (I').

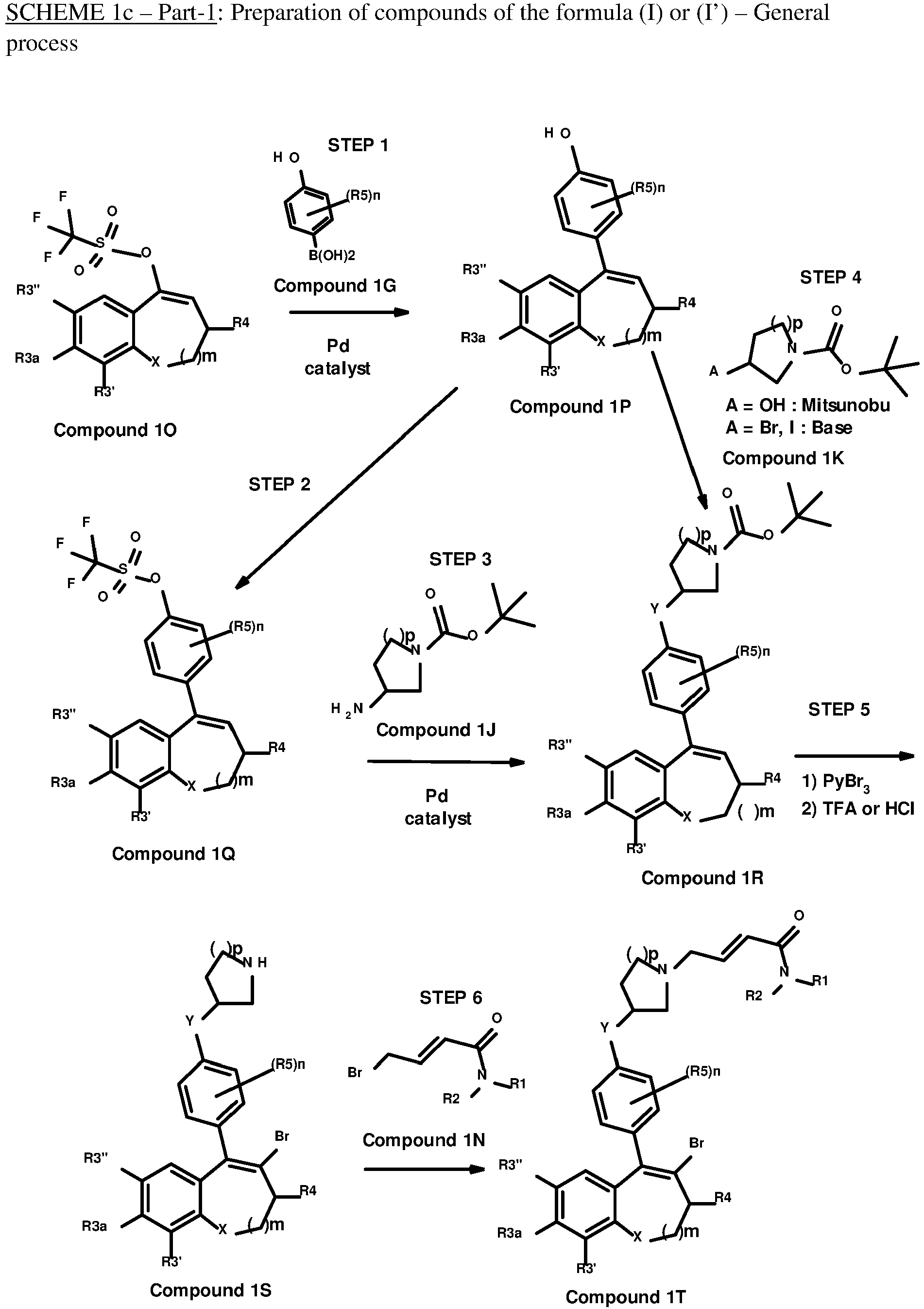

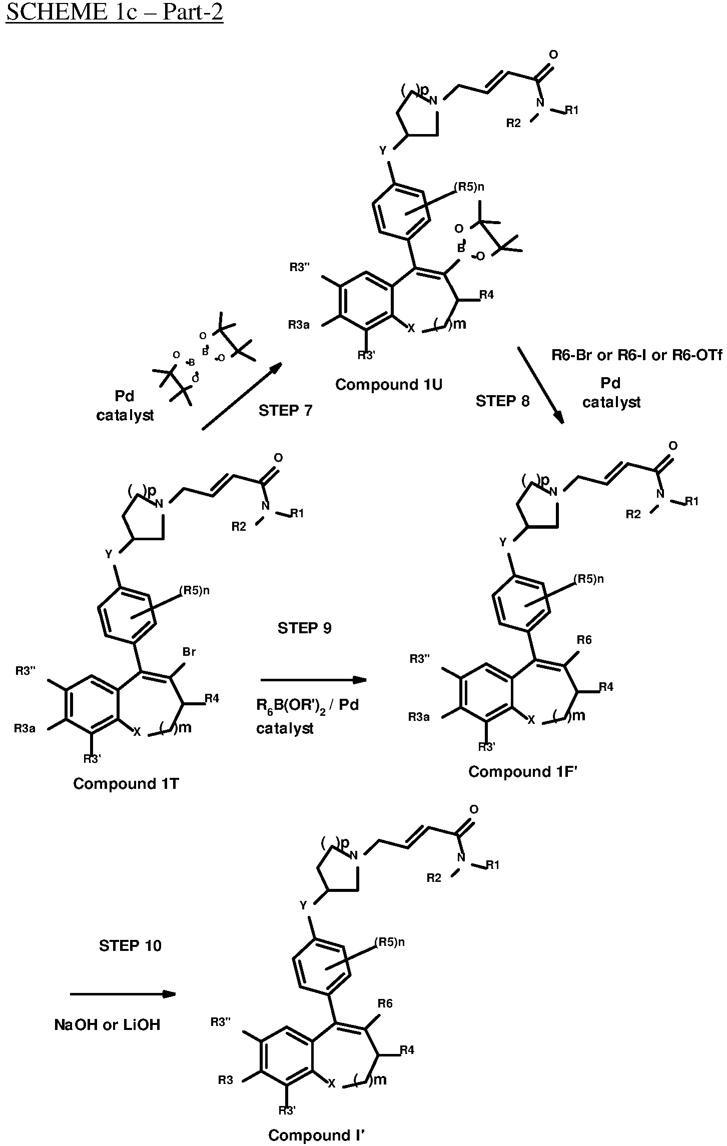

According to SCHEME 1c Part-1 and Part-2 in which R3a is a hydrogen atom or a carboxylic ester such as -COOMe, -COOEt, or protected -OH such as O-pivaloyl for example, and R1, R2, R3, R3', R3", R4, R5, R6, X, Y, m, n and p are defined as described above, compound 1O can be converted in STEP 1 to compound 1P by treatment with compound 1G using for example [1,1'-bis(diphenylphosphino)ferrocene]dichloropalladium(II) (Pd(dppf)Cl₂), complex with DCM, as catalyst, in a mixture of dioxane and water and in the presence of a base, for example cesium carbonate (Cs₂CO₃), by heating up to reflux of solvent.

Compound 1P can be converted in STEP 2 to compound 1Q by treatment with trifluoromethanesulfonic anhydride in the presence of pyridine in DCM.

Compound 1Q can be converted in STEP 3 to compound 1R (Y = -NH-) in a Buchwald coupling reaction with compound 1J in dioxane at 130°C using for example xantphos / palladium acetate (Pd(OAc)₂ as catalytic system.

Compound 1P can be converted in STEP 4 to compound 1R (Y = -O-) by reaction with compound 1K (A = -Br or -I) in presence of a base such as cesium carbonate (Cs₂CO₃) in DMF or MeCN, or under Mitsunobu conditions, when (A = -OH), using for example N,N,N',N'-tetramethylazodicarboxamide and triphenylphosphine as coupling agents in THF as a solvent.

Compound 1R can be converted in STEP 5 to compound 1S by treatment with for example pyridinium tribromide in DCM or THF at room temperature followed by treatment of the obtained residue with TFA in DCM or HCl in dioxane.

Compound 1S can be converted in STEP 6 to compound 1T by treatment with compound 1N, in the presence of a base such as DIEA in DMF or MeCN.

Compound 1T can be converted in STEP 7 to compound 1U by treatment for example with bis(pinacolato)diboron, and with a palladium catalyst, for example bis(triphenylphosphine)palladium(II) dichloride Pd(PPh₃)₂Cl₂, and a phosphine such as triphenylphosphine in solution in toluene by heating up to reflux of solvent in presence of a base such as KOPh or AcOK.

Compound 1F' can be prepared in a Suzuki coupling reaction either between 1U and R6Br or R6I or R6OTf in STEP 8 or between compounds 1T and R6B(OR')₂ or R6BF₃K, wherein -B(OR')₂ is a boronic acid or a pinacolate ester and R6 is as above defined, in STEP 9 using for example [1,1'-bis(diphenylphosphino) ferrocene]dichloropalladium(II) (Pd(dppf)Cl₂), complex with DCM, as catalyst, in a mixture of dioxane and water and in the presence of a base, for example cesium carbonate (Cs₂CO₃), by heating up to reflux of solvent.

When R3a is -COOMe, -COOEt, or a protected -OH such as O-pivaloyl, deprotection can be performed in STEP 10 by treatment with an aqueous solution of sodium hydroxide (NaOH) 2N or lithium hydroxide (LiOH) in THF or dioxane. When R3 is -COOH, extraction of the product could give the sodium or lithium salt of compound of formula (I) or (I'). The acidification with an aqueous solution of HCl 2N to pH 6-7 can give the neutral form of compound of formula (I) or (I'). The acidification with an aqueous solution of HCl 2N to pH 1-2 can give the hydrochloride salt of compound of formula (I) or (I'). The purification using HPLC in presence of formic acid or trifluoroacetic acid in the eluent can give the formate or trifluoroacetate salt of compound of formula (I) or (I').

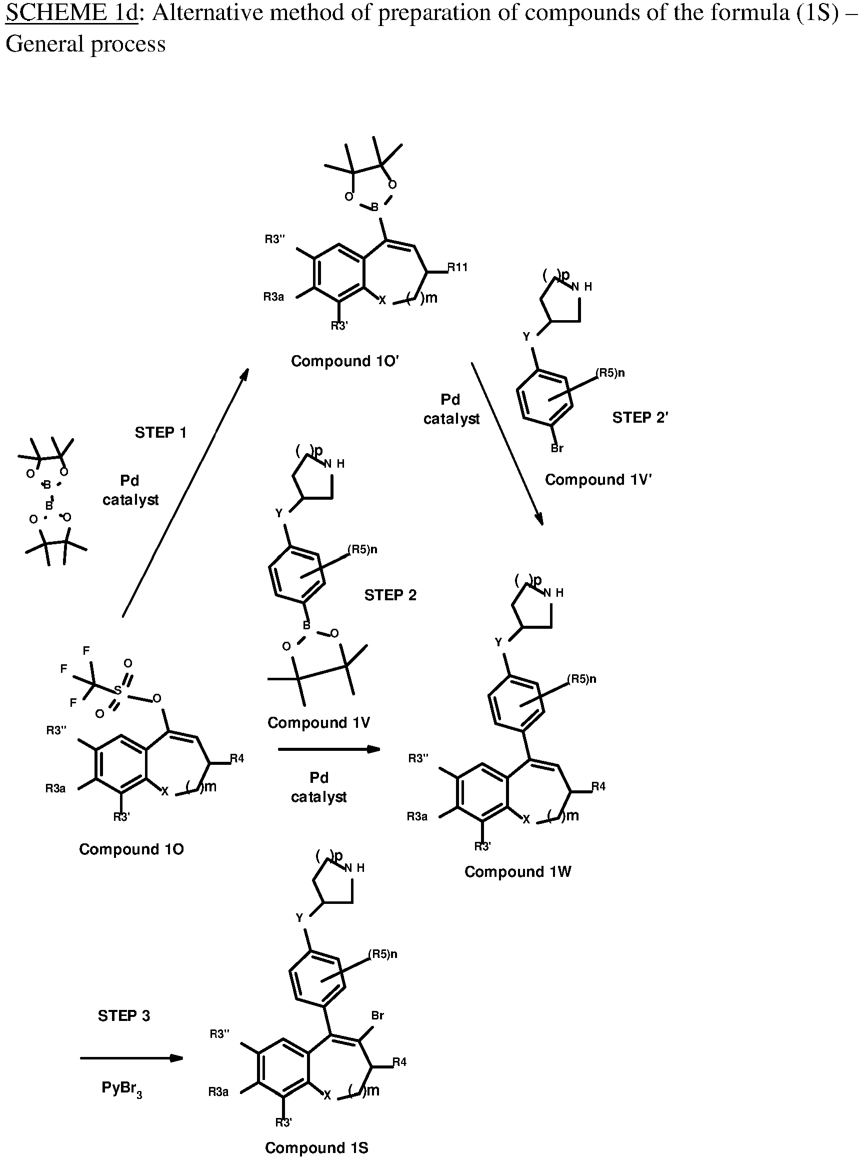

According to SCHEME 1d, in which R3a is a hydrogen atom or a carboxylic ester such as -COOMe, -COOEt, or protected -OH such as O-pivaloyl for example, and R3, R3', R3", R4, R5, X, m, n and p are defined as described above, compound 1O can be converted in STEP 1 to compound 1O' by treatment for example with bis(pinacolato)diboron, and with a palladium catalyst, for example bis(triphenylphosphine)palladium(II) dichloride Pd(PPh₃)₂Cl₂, and a phosphine such as triphenylphosphine in solution in toluene by heating up to reflux of solvent in presence of a base such as KOPh or AcOK.

Compound 1W can be prepared in a Suzuki coupling reaction either between compounds 1O and 1V in STEP 2 or between compounds 1O' and 1V' in STEP 2' in the presence of a palladium catalyst, for example [1,1'-bis(diphenylphosphino)ferrocene]dichloropalladium(II) (Pd(dppf)Cl₂), complex with DCM, in a mixture of dioxane and water and in the presence of a base, for example cesium carbonate (Cs₂CO₃), by heating up to reflux of solvent.

Compound 1W can be converted in STEP 3 to compound 1S by treatment with for example pyridinium tribromide in DCM or THF at room temperature.

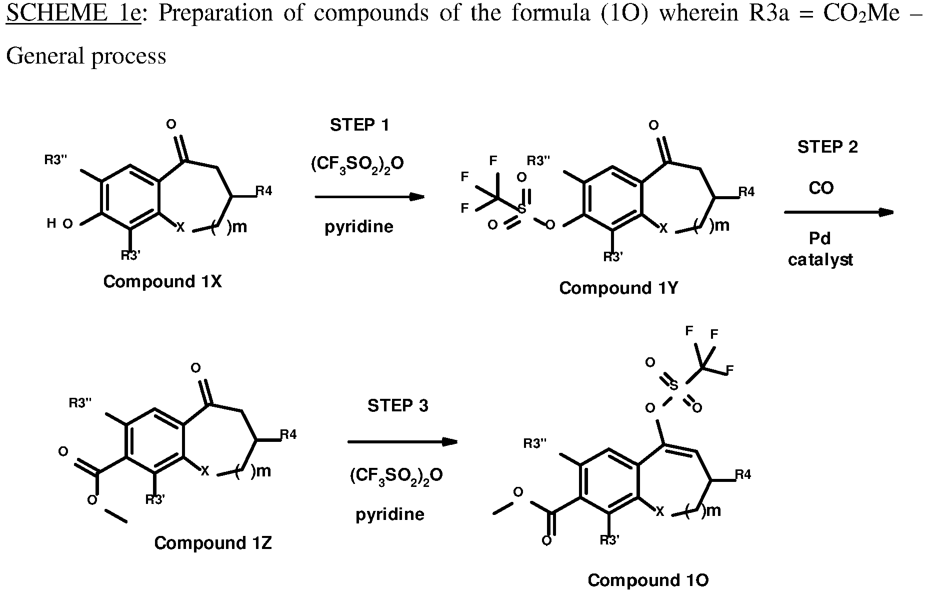

According to SCHEME 1e, in which X, m, R3', R3", R3a = CO₂Me and R4 are defined as described above, compound 1O could be prepared as follows: compound 1X (commercially available or prepared according to WO2017140669 and WO2018091153) can be converted in STEP 1 to compound 1Y by treatment with trifluoromethanesulfonic anhydride, in solution in DCM, in the presence of pyridine as a base.

Compound 1Y can be converted in STEP 2 to compound 1Z by carbonylation with carbon monoxide, in solution in DMF and MeOH, in the presence of a palladium catalyst, for example [1,1'-bis(diphenylphosphino)ferrocene]dichloropalladium(II) (Pd(dppf)Cl₂), complex with DCM.

Compound 1Z can be converted in STEP 3 to compound 1O wherein R3a = CO₂Me by treatment with trifluoromethanesulfonic anhydride, in solution in DCM, in the presence of pyridine as a base.

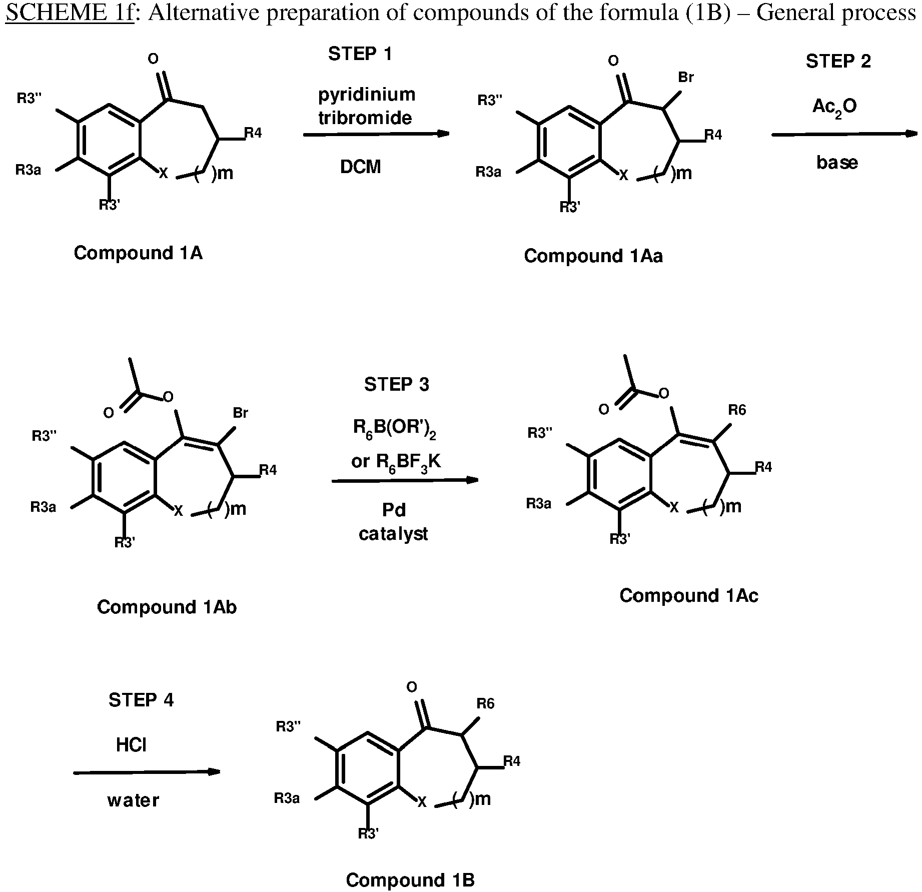

According to SCHEME 1f, in which R3a is a hydrogen atom or a carboxylic ester such as COOMe, COOEt, or protected OH such as O-pivaloyl for example, R3', R3", R, X and m are defined as described above, compound 1B can alternatively be prepared as follows: compound 1A can be converted in STEP 1 to compound 1Aa by treatment with pyridinium tribromide in DCM or THF at room temperature for example.

Compound 1Aa can be converted in STEP 2 to compound 1Ab by deprotonation with a base such as LiHMDS in THF followed by treatment with acetic anhydride.

Compound 1Ac can be prepared in STEP 3 in a Suzuki coupling reaction between compounds 1Ab and R₆B(OR')₂ or R₆BF₃K using for example [1,1'-bis(diphenylphosphino)ferrocene]dichloropalladium(II) (Pd(dppf)Cl₂), complex with DCM, as catalyst, in a mixture of toluene and water and in the presence of a base, for example cesium carbonate (Cs₂CO₃), by heating up to reflux of solvent.

Compound 1Ac can be converted in STEP 4 to compound 1B by hydrolysis with aqueous HCl solution by heating in methanol and DCM for example.

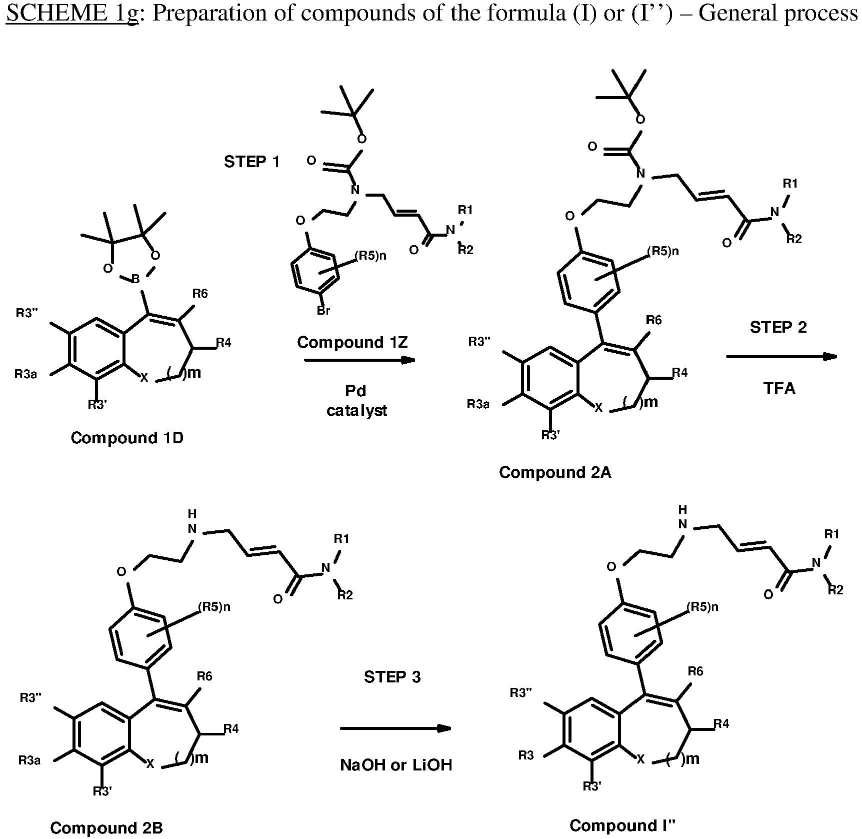

According to SCHEME 1g, in which R3a is a hydrogen atom or a carboxylic ester such as -COOMe, -COOEt, or protected -OH such as O-pivaloyl for example, and R1, R2, R3, R3', R3", R4, R5, R6, X, m and n are defined as described above, compound 1D can be converted in STEP 1 to compound 2A by treatment with compound 1Z using for example [1,1'-bis(diphenylphosphino)ferrocene]dichloropalladium(II) (Pd(dppf)Cl₂), complex with DCM, as catalyst, in a mixture of dioxane and water and in the presence of a base, for example cesium carbonate (Cs₂CO₃), by heating up to reflux of solvent.

Compound 2A can be converted to compound 2B in STEP 2 by treatment with TFA in DCM.

When R3a is -COOMe, -COOEt, or a protected -OH such as O-pivaloyl, deprotection can be performed in STEP 3 by treatment with an aqueous solution of sodium hydroxide (NaOH) 2N or lithium hydroxide (LiOH) in THF or dioxane. When R3 is -COOH, extraction of the product could give the sodium or lithium salt of compound of formula (I) or (I"). The acidification with an aqueous solution of HCl 2N to pH 6-7 could give the neutral form of compound of formula (I) or (I"). The acidification with an aqueous solution of HCl 2N to pH 1-2 could give the hydrochloride salt of compound of formula (I) or (I"). The purification using HPLC in presence of formic acid or trifluoroacetic acid in the eluent could give the formate or trifluoroacetate salt of compound of formula (I) or (I").

Herein is also provided a process for preparing a compound of formula (I) or (I') as defined above, wherein a compound of formula 1F: 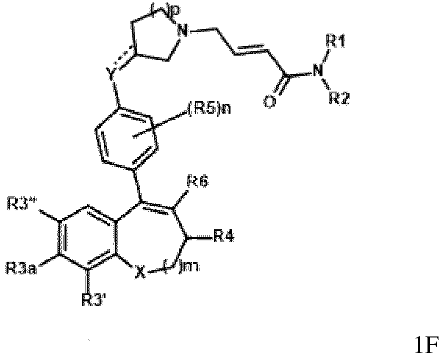 wherein R1, R2, R3', R3", R4, R5, R6, m, n, p, X, Y and 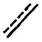 are as defined above and R3a is a hydrogen atom or a carboxylic ester such as -COOMe, -COOEt, or protected -OH such as O-pivaloyl for example, is converted to compound of formula (I) or (I'), in presence of a source of hydroxide ions, such as NaOH or LiOH in solution in THF or dioxane.

Herein is also provided a process for preparing a compound of formula (I) or (I') as described above, wherein a compound of formula 1F': 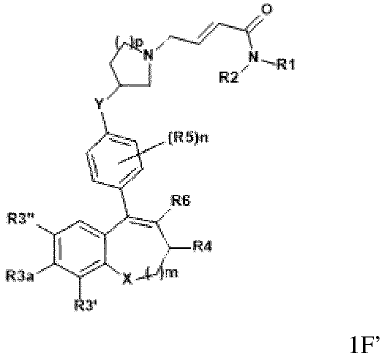 wherein R1, R2, R3, R3', R3", R4, R5, R6, m, n, p, X and Y are as described above, and R3a is a hydrogen atom or a carboxylic ester such as -COOMe, -COOEt, or protected -OH such as O-pivaloyl for example, is converted to compound of formula (I) or (I'), in presence of a source of hydroxide ions, such as NaOH or LiOH in solution in THF or dioxane , said step being optionally preceded by a step for obtaining compound 1F', wherein a compound of formula 1T: 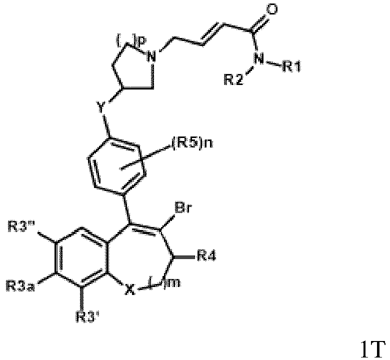
wherein R1, R2, R3', R3", R4, R5, m, n, p, X and Y are as described above,
is submitted to a Suzuki coupling with a boronic reagent R6B(OR')₂, wherein -B(OR')₂ is a boronic acid or a pinacolate ester and R6 is defined above.

Herein are also provided the intermediates compounds selected from compounds of formula 1T, 1F, 1F' and 2B, or any of its pharmaceutically acceptable salt, 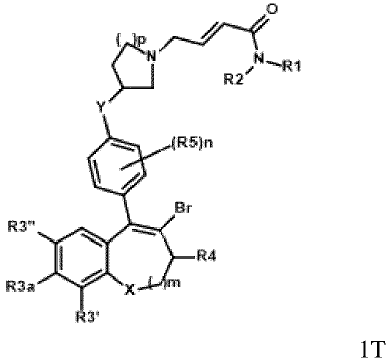 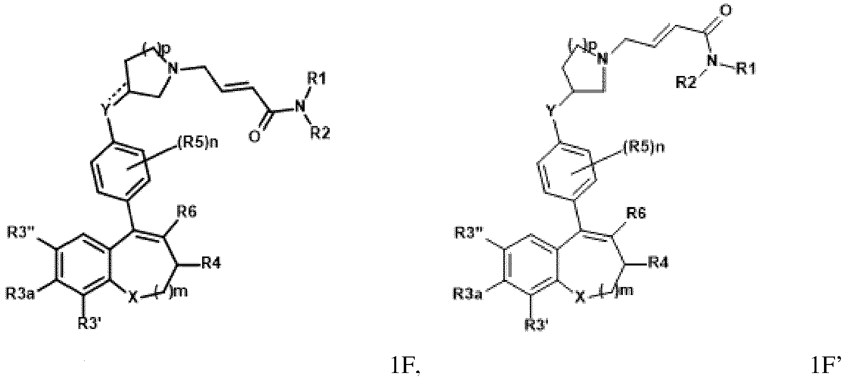 and 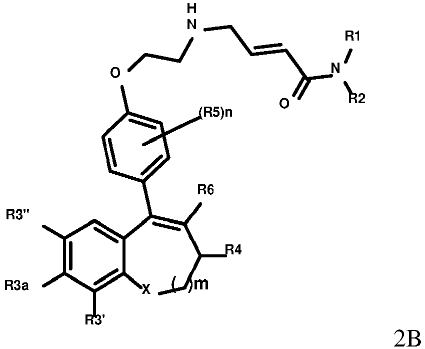 wherein R1, R2, R3', R3", R4, R5, R6, m, n, p, X, Y and 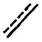 are as defined above and R3a is a hydrogen atom or a carboxylic ester such as -COOMe, -COOEt, or protected -OH such as O-pivaloyl.

Herein is further provided the intermediate compound of formula 1E, or any of its pharmaceutically acceptable salt: 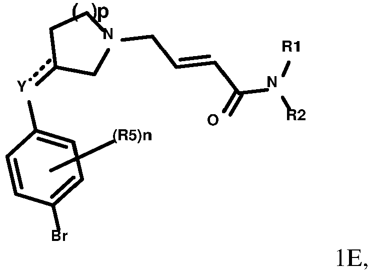 wherein R1, R2, R5, Y, n, p and 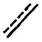 are as described above.

The present application also describes the intermediate compound of formula 1D, or any of its pharmaceutically acceptable salt: 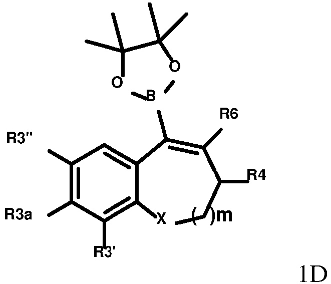 wherein R3a, R3', R3", X, m, R4 and R6 are as described above.

In another aspect, herein is also provided a process for the preparation of a compound of formula (I), wherein R3 is a -COOH group, comprising a deprotection step of a compound of formula IF as defined above, optionally followed by a purification step.

Said purification step may for example consist, as illustrated in step 2 of example 1 herein after, in an acidification step, for example with an aqueous solution of hydrochloric acid.

The ¹H NMR Spectra at 400 and 500 MHz were performed on a Bruker Avance DRX-400 and Bruker Avance DPX-500 spectrometer, respectively, with the chemical shifts (δ in ppm) in the solvent dimethyl sulfoxide-d6 (d6-DMSO) referenced at 2.5 ppm at a temperature of 303 K. Coupling constants (J) are given in Hertz.

The liquid chromatography/mass spectra (LC/MS) were obtained on a UPLC Acquity Waters instrument, light scattering detector Sedere and SQD Waters mass spectrometer using UV detection DAD 210-400 nm and flash Acquity UPLC CSH C18 1.7 µm, dimension 2.1x30 mm, mobile phase H₂O + 0.1% HCO₂H / CH₃CN + 0.1% HCO₂H.

**Table 1a: (the first column Ex corresponds to the compound and example number)**

| **Ex** | **Structure** | **Name** |
|---|---|---|
| 1 | 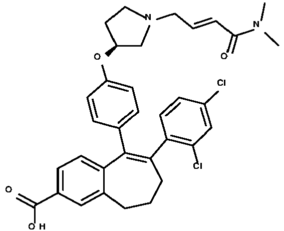 | (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 2 | 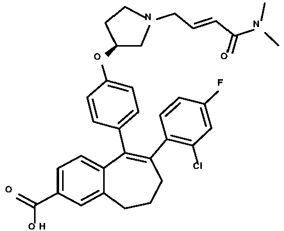 | (S,E)-8-(2-chloro-4-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 3 | 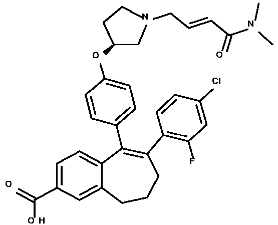 | (S,E)-8-(4-chloro-2-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 4 | 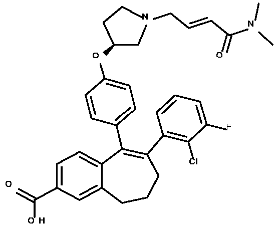 | (S,E)-8-(2-chloro-3-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 5 | 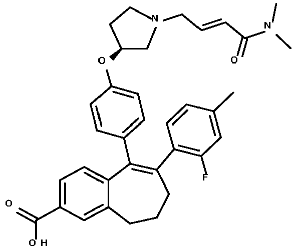 | (S,E)-9-(4-((1-(4-(dimethylamino)-4-oxobut-2-en-1-yl)pyrrolidin-3-yl)oxy)phenyl)-8-(2-fluoro-4-methylphenyl)-6,7-dihydro-5H-benzo[7]annulene-3-carboxylic acid |
| 6 | 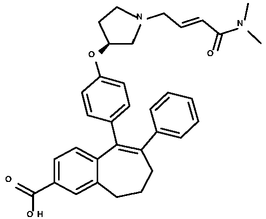 | (S,E)-9-(4-((1-(4-(dimethylamino)-4-oxobut-2-en-1-yl)pyrrolidin-3-yl)oxy)phenyl)-8-phenyl-6,7-dihydro-5H-benzo[7]annulene-3-carboxylic acid |
| 7 | 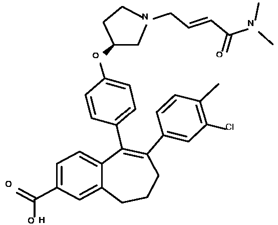 | (S,E)-8-(3-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 8 | 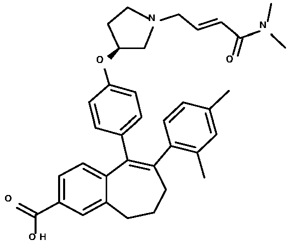 | (S,E)-9-(4-((1-(4-(dimethylamino)-4-oxobut-2-en-1-yl)pyrrolidin-3-yl)oxy)phenyl)-8-(2,4-dimethylphenyl)-6,7-dihydro-5H-benzo[7]annulene-3-carboxylic acid |
| 9 | 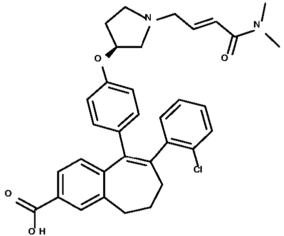 | (S,E)-8-(2-chlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 10 | 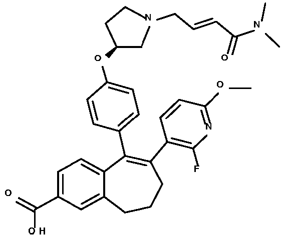 | (S,E)-9-(4-((1-(4-(dimethylamino)-4-oxobut-2-en-1-yl)pyrrolidin-3-yl)oxy)phenyl)-8-(2-fluoro-6-methoxypyridin-3-yl)-6,7-dihydro-5H-benzo[7]annulene-3-carboxylic acid |
| 11 | 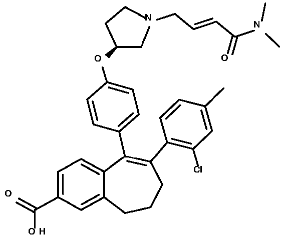 | (S,E)-8-(2-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 12 | 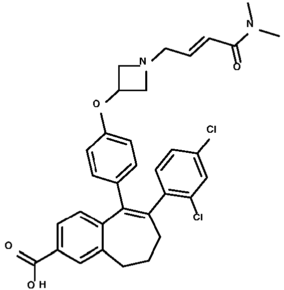 | (E)-8-(2,4-dichlorophenyl)-9-(4-((1-(4-(dimethylamino)-4-oxobut-2-en-1-yl)azetidin-3-yl)oxy)phenyl)-6,7-dihydro-5H-benzo[7]annulene-3-carboxylic acid |
| 13 | 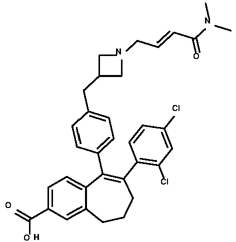 | (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylic acid compound with 2,2,2-trifluoroacetic acid (1:1) |
| 14 | 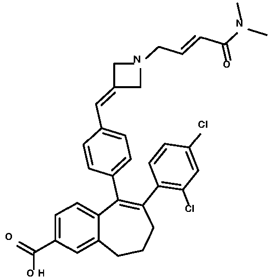 | (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylic acid |
| 15 | 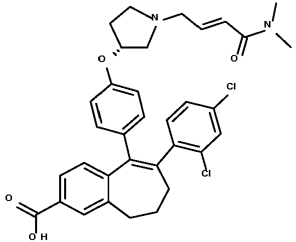 | (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid |
| 16 | 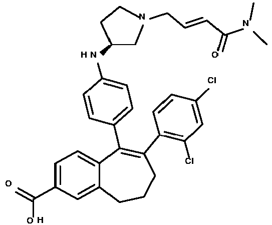 | (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid |
| 17 | 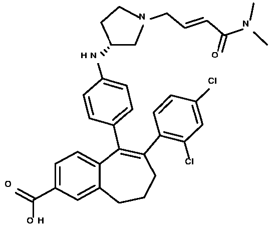 | (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid |
| 18 | 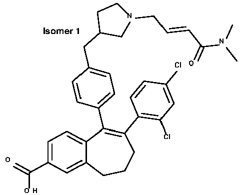 | (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 1 |
| 19 | 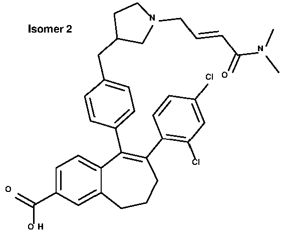 | (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 2 |
| 20 | 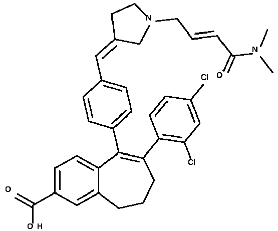 | 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylic acid |
| 21 | 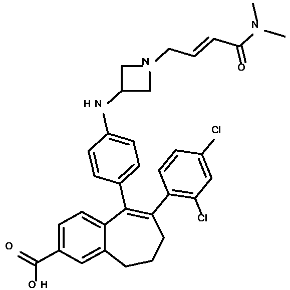 | (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylic acid |
| 22 | 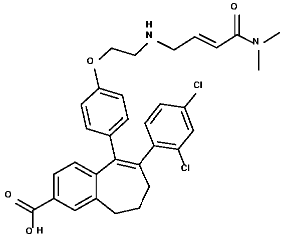 | (E)-8-(2,4-dichlorophenyl)-9-(4-(2-((4-(dimethylamino)-4-oxobut-2-en-1-yl)amino)ethoxy)phenyl)-6,7-dihydro-5H-benzo[7]annulene-3-carboxylic acid |
| 23 | 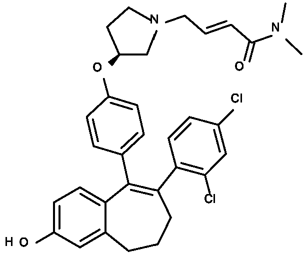 | (S,E)-4-(3-(4-(8-(2,4-dichlorophenyl)-3-hydroxy-6,7-dihydro-5H-benzo[7]annulen-9-yl)phenoxy)pyrrolidin-1-yl)-N,N-dimethylbut-2-enamide |
| 24 | 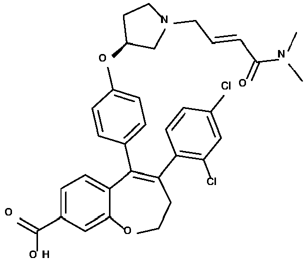 | (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylic acid |
| 25 | 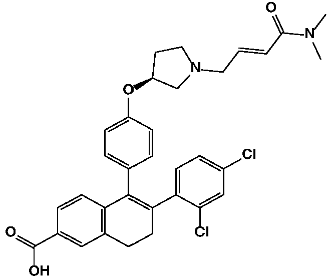 | (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylic acid |
| 26 | 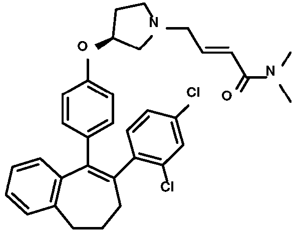 | (S,E)-4-(3-(4-(8-(2,4-dichlorophenyl)-6,7-dihydro-5H-benzo[7]annulen-9-yl)phenoxy)pyrrolidin-1-yl)-N,N-dimethylbut-2-enamide |
| 27 | 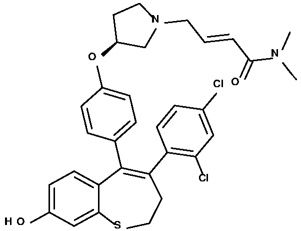 | (S,E)-4-(3-(4-(4-(2,4-dichlorophenyl)-8-hydroxy-2,3-dihydrobenzo[b]thiepin-5-yl)phenoxy)pyrrolidin-1-yl)-N,N-dimethylbut-2-enamide |

**Table 1b:**

| **Ex** | **Preparation Method** | **NMR** | **MASS:** LC/MS (m/z, MH+): |
|---|---|---|---|
| 1 | A | 1H NMR (400 MHz, CDCl₃) δ ppm 2.03 (m, 1 H), 2.14 - 2.37 (m, 5 H), 2.80 - 2.97 (m, 4 H), 3.00 (s, 3 H), 3.05 (s, 3 H), 3.09 - 3.21 (m, 2 H), 3.41 - 3.49 (m, 2 H), 4.78 (br t, J=6 Hz, 1 H), 6.49 - 6.58 (m, 3 H), 6.74 (d, J=8 Hz, 2 H), 6.84 - 7.08 (m, 4 H), 7.34 (d, J=2 Hz, 1 H), 7.84 (dd, J=8, 2 Hz, 1 H), 7.98 (d, J=2 Hz, 1 H) | 605 |
| 2 | A | 1H NMR (400 MHz, CDCl₃) δ ppm 2.02 (m, 1 H), 2.13 - 2.38 (m, 5 H), 2.75 - 2.96 (m, 4 H), 3.00 (s, 3 H), 3.05 (s, 3 H), 3.11 (m, 2 H), 3.34 - 3.52 (m, 2 H), 4.78 (m, 1 H), 6.45 - 6.59 (m, 3 H), 6.68 - 6.92 (m, 4 H), 6.96 (br d, J=8 Hz, 1 H), 6.99 - 7.14 (m, 2 H), 7.84 (br d, J=8 Hz, 1 H), 7.98 (s, 1 H) | 589 |
| 3 | A | 1H NMR (400 MHz, CDCl₃) δ ppm 1.87 - 2.54 (m, 6 H), 2.69 - 3.16 (m, 12 H), 3.32 - 3.54 (m, 2 H), 4.79 (m, 1 H), 6.48 - 6.59 (m, 3 H), 6.75 (d, J=9 Hz, 2 H), 6.84 - 7.05 (m, 5 H), 7.83 (dd, J=8, 2 Hz, 1 H), 7.97 (d, J=1 Hz, 1 H) | 589 |
| 4 | A | 1H NMR (400 MHz, CDCl₃) δ ppm 2.00 (m, 1 H), 2.12 - 2.36 (m, 5 H), 2.74 - 3.17 (m, 12 H), 3.34 - 3.51 (m, 2 H), 4.76 (br s, 1 H), 6.46 - 6.56 (m, 3 H), 6.75 (d, J=8 Hz, 2 H), 6.84 - 7.07 (m, 5 H), 7.85 (d, J=8 Hz, 1 H), 7.99 (s, 1 H) | 589 |
| 5 | A | 1H NMR (400 MHz, CDCl₃) δ ppm 2.02 (m, 1 H), 2.11 - 2.31 (m, 8 H), 2.75 - 3.18 (m, 12 H), 3.34 - 3.52 (m, 2 H), 4.78 (br t, J=6 Hz, 1 H), 6.44 - 6.60 (m, 3 H), 6.71 - 6.82 (m, 4 H), 6.87 (dt, J=15, 6 Hz, 1 H), 6.92 - 7.07 (m, 2 H), 7.83 (d, J=8 Hz, 1 H), 7.97 (d, J=1 Hz, 1 H) | 569 |
| 6 | A | 1H NMR (400 MHz, CDCl₃) δ ppm 2.01 (m, 1 H), 2.12 - 2.44 (m, 5 H), 2.64 - 3.23 (m, 12 H), 3.33 - 3.51 (m, 2 H), 4.78 (m, 1 H), 6.43 - 6.61 (m, 3 H), 6.69 - 6.80 (m, 2 H), 6.88 (dt, J=15, 6 Hz, 1 H), 6.95 (d, J=8 Hz, 1 H), 7.08 - 7.23 (m, 5 H), 7.82 (s, 1 H), 7.97 (d, J=1 Hz, 1 H) | 537 |
| 7 | A | 1H NMR (400 MHz, CDCl₃) δ ppm 2.03 (m, 1 H), 2.13 - 2.36 (m, 8 H), 2.79 - 2.98 (m, 5 H), 3.00 (s, 3 H), 3.06 (s, 3 H), 3.11 (m, 1 H), 3.31 - 3.56 (m, 2 H), 4.80 (m, 1 H), 6.48 - 6.61 (m, 3 H), 6.76 (d, J=8 Hz, 2 H), 6.85 - 7.01 (m, 4 H), 7.15 (d, J=2 Hz, 1 H), 7.83 (dd, J=8, 2 Hz, 1 H), 7.96 (d, J=2 Hz, 1 H) | 585 |
| 8 | A | 1H NMR (400 MHz, CDCl₃) δ ppm 2.01 (m, 1 H), 2.09 - 2.15 (m, 3 H), 2.16 - 2.29 (m, 8 H), 2.70 - 2.95 (m, 5 H), 3.00 (s, 3 H), 3.03 - 3.15 (m, 4 H), 3.32 - 3.48 (m, 2 H), 4.75 (m, 1 H), 6.43 - 6.58 (m, 3 H), 6.69 (d, J=9 Hz, 2 H), 6.79 - 7.05 (m, 5 H), 7.83 (dd, J=8, 2 Hz, 1 H), 7.97 (d, J=2 Hz, 1 H) | 565 |
| 9 | A | 1H NMR (400 MHz, CDCl₃) δ ppm 2.01 (m, 1 H), 2.15 - 2.37 (m, 5 H), 2.75 - 2.96 (m, 5 H), 3.00 (s, 3 H), 3.05 (s, 3 H), 3.11(m, 1 H), 3.31 - 3.51 (m, 2 H), 4.76 (m, 1 H), 6.48 - 6.56 (m, 3 H), 6.76 (d, J=8 Hz, 2 H), 6.87 (dt, J=15, 6 Hz, 1 H), 6.98 (d, J=8 Hz, 1 H), 7.04 - 7.13 (m, 3 H), 7.32 (d, J=9 Hz, 1 H), 7.84 (dd, J=8, 2 Hz, 1 H), 7.98 (d, J=2 Hz, 1 H) | 571 |
| 10 | A | 1H NMR (400 MHz, CDCl₃) δ ppm 2.02 (m, 1 H), 2.13 - 2.33 (m, 5 H), 2.78 - 2.96 (m, 5 H), 3.00 (s, 3 H), 3.05 (s, 3 H), 3.10 (br dd, J=11, 6 Hz, 1 H), 3.35 - 3.51 (m, 2 H), 3.88 (s, 3 H), 4.79 (m, 1 H), 6.45 (dd, J=8, 1 Hz, 1 H), 6.51 (d, J=15 Hz, 1 H), 6.55 - 6.62 (m, 2 H), 6.75 - 6.82 (m, 2 H), 6.88 (dt, J=16, 6 Hz, 1 H), 6.95 (d, J=8 Hz, 1 H), 7.35 (m, 1 H), 7.83 (dd, J=8, 2 Hz, 1 H), 7.97 (d, J=2 Hz, 1 H) | 586 |
| 11 | A | 1H NMR (400 MHz, CDCl₃) δ ppm 2.01 (m, 1 H), 2.28 (s, 8 H), 2.80 - 3.18 (m, 12 H), 3.32 - 3.53 (m, 2 H), 4.77 (m, 1 H), 6.46 - 6.56 (m, 3 H), 6.77 (d, J=9 Hz, 2 H), 6.83 - 6.92 (m, 2 H), 6.92 - 7.00 (m, 2 H), 7.14 (s, 1 H), 7.83 (dd, J=8, 2 Hz, 1 H), 7.97 (d, J=2 Hz, 1 H) | 585 |
| 12 | B | 1H NMR (500 MHz, DMSO-d6) δ ppm 2.07 - 2.22 (m, 4 H), 2.85 (s, 3 H), 2.89 (br d, J=4 Hz, 2 H), 2.92 - 2.98 (m, 2 H), 3.02 (s, 3 H), 3.21 (d, J=2 Hz, 2 H), 3.68 - 3.76 (m, 2 H), 4.71 (quin, J=6 Hz, 1 H), 6.48 (s, 2 H), 6.61 (d, J=9 Hz, 2 H), 6.75 (d, J=9 Hz, 2 H), 6.78 (br d, J=8 Hz, 1 H), 7.19 (d, J=8 Hz, 1 H), 7.27 (dd, J=8, 2 Hz, 1 H), 7.59 (d, J=2 Hz, 1 H), 7.74 (br d, J=7 Hz, 1 H), 7.89 (s, 1 H) | 591 |
| 13 | B | 1H NMR (400 MHz, DMSO-d6) δ ppm 2.08 - 2.26 (m, 4 H), 2.76 - 2.86 (m, 2 H), 2.88 (s, 3 H), 2.92 - 3.01 (m, 3 H), 3.04 (s, 3 H), 3.73 - 3.85 (m, 2 H), 3.91 (br s, 2 H), 4.12 (br s, 2 H), 6.43 (m, 1 H), 6.73 - 6.86 (m, 4 H), 6.98 (d, J=8 Hz, 2 H), 7.18 - 7.28 (m, 2 H), 7.59 (d, J=2 Hz, 1 H), 7.75 (dd, J=8, 2 Hz, 1 H), 7.93 (d, J=2 Hz, 1 H), 10.01 (br s, 1 H), 12.89 (br s, 1 H) | 589 |
| 14 | B | 1H NMR (400 MHz, DMSO-d6) δ ppm 2.06 - 2.29 (m, 4 H), 2.86 (s, 3 H), 2.89 - 2.98 (m, 2 H), 3.03 (s, 3 H), 3.19 - 3.25 (m, 2 H), 3.94 (br s, 2 H), 4.09 (br s, 2 H), 6.09 (t, J=2 Hz, 1 H), 6.47 - 6.57 (m, 2 H), 6.78 - 6.92 (m, 5 H), 7.19 - 7.29 (m, 2 H), 7.58 (d, J=2 Hz, 1 H), 7.74 (dd, J=8, 2 Hz, 1 H), 7.91 (d, J=2 Hz, 1 H) | 587 |
| 15 | B | 1H NMR (400 MHz, DMSO-d6) δ ppm 1.72 (m, 1 H), 2.00 - 2.28 (m, 5 H), 2.39 - 3.25 (m, 12 H), 3.36(m hidden, 2 H), 4.77 (br t, J=6 Hz, 1 H), 6.51 - 6.60 (m, 2 H), 6.63 - 6.69 (m, 2 H), 6.71 - 6.77 (m, 2 H), 6.88 (d, J=8 Hz, 1 H), 7.20 (d, J=9 Hz, 1 H), 7.28 (d, J=8 Hz, 1 H), 7.59 (d, J=2 Hz, 1 H), 7.76 (dd, J=8, 2 Hz, 1 H), 7.91 (d, J=2 Hz, 1 H), 12.20 (br s, 1 H) | 605 |
| 16 | B | 1H NMR (400 MHz, DMSO-d6) δ ppm 0.84 (m, 1 H), 1.28 (m, 1 H), 2.00 - 2.27 (m, 4 H), 2.51 - 3.35 (m partially hidden, 14 H), 4.13 (m hidden , 1 H), 5.75 - 6.13 (m, 1 H), 6.36 (d, J=9 Hz, 2 H), 6.54 - 6.66 (m, 4 H), 6.90 (d, J=8 Hz, 1 H), 7.20 (d, J=8 Hz, 1 H), 7.29 (dd, J=8, 2 Hz, 1 H), 7.59 (d, J=2 Hz, 1 H), 7.75 (dd, J=8, 2 Hz, 1 H), 7.90 (d, J=2 Hz, 1 H), 12.85 (br s, 1 H) | 604 |
| 17 | B | 1H NMR (400 MHz, DMSO-d6) δ ppm 1.51 (m, 1 H), 2.05 - 2.20 (m, 5 H), 2.34 (m, 1 H), 2.43 (m, 1 H), 2.60 (m, 1 H), 2.72 (m, 1 H), 2.80 - 2.91 (m, 5 H), 3.00 (s, 3 H), 3.13 - 3.23 (m, 2 H), 3.76 (m, 1 H), 5.74 (br d, J=7 Hz, 1 H), 6.29 (d, J=9 Hz, 2 H), 6.50 - 6.63 (m, 4 H), 6.91 (d, J=8 Hz, 1 H), 7.19 (d, J=8 Hz, 1 H), 7.27 (dd, J=8, 2 Hz, 1 H), 7.57 (d, J=2 Hz, 1 H), 7.74 (dd, J=8, 2 Hz, 1 H), 7.87 (d, J=2 Hz, 1 H), 11.29 - 13.81 (m, 1 H) | 604 |
| 18 | B | 1H NMR (400 MHz, DMSO-d6) δ ppm 1.50 (m, 1 H), 1.86 (m, 1 H), 2.11 - 2.23 (m, 4 H), 2.42 - 3.91 (m, 9 H), 2.86 (s, 3 H), 2.90 - 2.96 (m, 2 H), 3.02 (s, 3 H), 6.57 (td, J=15, 6 Hz, 1 H), 6.66 - 6.78 (m, 1 H), 6.78 (d, J=8 Hz, 2 H), 6.86 (d, J=8 Hz, 1 H), 6.99 (d, J=8 Hz, 2 H), 7.18 (d, J=8 Hz, 1 H), 7.25 (dd, J=8, 2 Hz, 1 H), 7.57 (d, J=2 Hz, 1 H), 7.75 (dd, J=8, 2 Hz, 1 H), 7.91 (d, J=2 Hz, 1 H), 10.58 - 13.98 (m, 1 H) | 603 |
| 19 | B | 1H NMR (400 MHz, DMSO-d6) δ ppm 1.35 (m, 1 H), 1.77 (m, 1 H), 2.01 - 2.22 (m, 5 H), 2.25 - 2.55 (m, 6 H), 2.85 (s, 3 H), 2.86 - 2.95 (m, 2 H), 3.00 (s, 3 H), 3.07 - 3.21 (m, 2 H), 6.51 (d, J=15 Hz, 1 H), 6.56 (td, J=15, 6 Hz, 1 H), 6.75 (d, J=8 Hz, 2 H), 6.80 (br d, J=8 Hz, 1 H), 6.96 (d, J=8 Hz, 2 H), 7.17 (d, J=8 Hz, 1 H), 7.24 (dd, J=8, 2 Hz, 1 H), 7.58 (s, 1 H), 7.72 (br d, J=7 Hz, 1 H), 7.89 (br s, 1 H) | 603 |
| 20 | B | 1H NMR (400 MHz, DMSO-d6) δ ppm 2.07 - 2.26 (m, 4 H), 2.55 - 2.61 (m, 4 H), 2.86 (s, 3 H), 2.90 - 2.97 (m, 2 H), 3.01 (s, 3 H), 3.27 (m partially hidden, 2 H), 3.35 (m partially hidden, 2 H), 6.22 (br s, 1 H), 6.53 - 6.65 (m, 2 H), 6.81 (d, J=8 Hz, 2 H), 6.87 (d, J=8 Hz, 1 H), 6.98 (d, J=9 Hz, 2 H), 7.20 (m, 1 H), 7.29 (m, 1 H), 7.58 (d, J=2 Hz, 1 H), 7.75 (dd, J=8, 2 Hz, 1 H), 7.92 (d, J=2 Hz, 1 H), 12.20 - 13.74 (m, 1 H) | 601 |
| 21 | B | 1H NMR (400 MHz, DMSO-d6) δ ppm 2.06 - 2.21 (m, 4 H), 2.72 - 2.81 (m, 2 H), 2.83 - 2.92 (m, 5 H), 3.02 (s, 3 H), 3.12 - 3.21 (m, 2 H), 3.63 (br t, J=7 Hz, 2 H), 3.88 (m, 1 H), 6.04 (d, J=7 Hz, 1 H), 6.25 (d, J=9 Hz, 2 H), 6.43 - 6.50 (m, 2 H), 6.50 - 6.56 (m, 2 H), 6.88 (d, J=8 Hz, 1 H), 7.18 (d, J=8 Hz, 1 H), 7.27 (dd, J=8, 2 Hz, 1 H), 7.57 (d, J=2 Hz, 1 H), 7.72 (dd, J=8, 2 Hz, 1 H), 7.87 (d, J=2 Hz, 1 H), 11.52 - 13.92 (m, 1 H) | 590 |
| 22 | B | 1H NMR (400 MHz, DMSO-d6) δ ppm 2.11 - 2.24 (m, 4 H), 2.88 (s, 3 H), 2.95 (t, J=8 Hz, 2 H), 3.05 (s, 3 H), 3.15 - 3.21 (m, 2 H), 3.69 (m, 2 H), 4.08 - 4.21 (m, 2 H), 6.60 (m, 1 H), 6.73 - 6.84 (m, 5 H), 6.87 (d, J=8 Hz, 1 H), 7.22 - 7.32 (m, 2 H), 7.60 (d, J=2 Hz, 1 H), 7.76 (dd, J=8, 2 Hz, 1 H), 7.92 (s, 1 H), 8.30 - 9.69 (m, 1 H) | 597 |
| 23 | C | 1H NMR (400 MHz, DMSO-d6) δ ppm 1.71 (m, 1 H), 2.02 - 2.11 (m, 2 H), 2.13 - 2.28 (m, 3 H), 2.37 - 2.44 (m, 1 H), 2.57 (br d, J=2 Hz, 1 H), 2.64 - 2.82 (m, 4 H), 2.86 (s, 3 H), 3.00 (s, 3 H), 3.19 (d, J=5 Hz, 2 H), 4.76 (m, 1 H), 6.49 - 6.77 (m, 9 H), 7.14 (d, J=9 Hz, 1 H), 7.23 (br d, J=8 Hz, 1 H), 7.54 (d, J=2 Hz, 1 H), 9.42 (s, 1 H) | 577 |
| 24 | A | 1H NMR (400 MHz, DMSO-d6) δ ppm 1.71 (m, 1 H), 2.22 (m, 1 H), 2.37 - 2.43 (m, 2 H), 2.60 (m, 1 H), 2.61 - 2.73 (m, 2 H), 2.79 (m, 1 H), 2.85 (s, 3 H), 3.00 (s, 3 H), 3.20 (d, J=6 Hz, 2 H), 4.49 - 4.63 (m, 2 H), 4.77 (br s, 1 H), 6.52 - 6.65 (m, 2 H), 6.69 (d, J=9 Hz, 2 H), 6.77 (d, J=9 Hz, 2 H), 6.87 (d, J=9 Hz, 1 H), 7.09 (d, J=8 Hz, 1 H), 7.27 (br d, J=8 Hz, 1 H), 7.59 - 7.66 (m, 3 H), 12.29 - 14.22 (m, 1 H) | |
| 25 | A | 1H NMR (400 MHz, DMSO-d6) δ ppm 1.74 (m, 1 H), 2.24 (m, 1 H), 2.36 - 2.74 (m partially hidden, 5 H), 2.82 (m, 1 H), 2.86 (s, 3 H), 2.99 - 3.07 (m, 5 H), 3.21 (d, J=5 Hz, 2 H), 4.80 (br s, 1 H), 6.53 - 6.62 (m, 2 H), 6.76 (m, 3 H), 6.94 (br d, J=8 Hz, 2 H), 7.12 (d, J=8 Hz, 1 H), 7.23 (m, 1 H), 7.55 (d, J=1 Hz, 1 H), 7.68 (br d, J=8 Hz, 1 H), 7.82 (s, 1 H), 11.58 - 13.62 (m, 1 H) | |
| 26 | C | 1H NMR (400 MHz, DMSO-d6) δ ppm 1.71 (m, 1 H), 2.04 - 2.28 (m, 5 H), 2.42 (m, 1 H), 2.56 (m, 1 H), 2.63 - 2.72 (m, 2 H), 2.74 - 2.91 (m, 5 H), 3.00 (s, 3 H), 3.19 (d, J=5 Hz, 2 H), 4.75 (m, 1 H), 6.49 - 6.61 (m, 2 H), 6.64 (d, J=10 Hz, 2 H), 6.73 (d, J=8 Hz, 2 H), 6.77 (dd, J=8, 1 Hz, 1 H), 7.13 - 7.29 (m, 4 H), 7.32 (dd, J=7, 1 Hz, 1 H), 7.57 (d, J=2 Hz, 1 H) | 450 |
| 27 | C | 1H NMR (400 MHz, DMSO-d6) δ ppm 1.69 (m, 1 H), 2.19 (m, 1 H), 2.35 - 2.44 (m, 2 H), 2.56 (m, 1 H), 2.62 - 2.73 (m, 2 H), 2.78 (m, 1 H), 2.85 (s, 3 H), 3.00 (s, 3 H), 3.08 (m, 1 H), 3.19 (d, J=5 Hz, 2 H), 3.44 (m, 1 H), 4.74 (m, 1 H), 6.34 (d, J=3 Hz, 1 H), 6.52 - 6.63 (m, 2 H), 6.64 - 6.76 (m, 5 H), 7.09 (d, J=8 Hz, 1 H), 7.25 (dt, J=8, 2 Hz, 1 H), 7.43 (d, J=8 Hz, 1 H), 7.64 (d, J=2 Hz, 1 H), 9.55 (s, 1 H) | 595 |

The examples which follow describe the preparation of some compounds of formula (I) described herein. The numbers of the compounds exemplified below match those given in the Tables 1a and 1b above. All reactions are performed under inert atmosphere, unless otherwise stated.

In the following examples, when the source of the starting products is not specified, it should be understood that said products are known compounds.

### Intermediates:

### Intermediate 1: Methyl (S,E)-8-bromo-9-(4-((1-(4-(dimethylamino)-4-oxobut-2-en-1-yl)pyrrolidin-3-yl)oxy)phenyl)-6,7-dihydro-5H-benzo[7]annulene-3-carboxylate

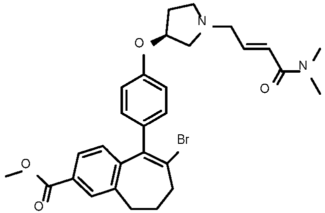

### Step 1: Methyl 9-(4-hydroxyphenyl)-6,7-dihydro-5H-benzo[7]annulene-3-carboxylate

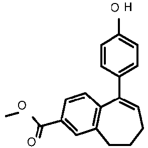

To a mixture of methyl 9-(((trifluoromethyl)sulfonyl)oxy)-6,7-dihydro-5H-benzo[7]annulene-3-carboxylate (23.8 g, 67.94 mmol) (prepared according to WO2017140669), (4-hydroxyphenyl)boronic acid (10.85 g, 74.73 mmol) and [1,1'-bis(diphenylphosphino)ferrocene]dichloropalladium(II), complex with DCM (4.48 g, 6.11 mmol) in dioxane (200 ml) and water (70 ml) was added Cs₂CO₃ (44.32 g, 135.88 mmol). The reaction mixture was stirred for 30 minutes at RT. Water (500 ml) and EtOAc (500 ml) were added. The organic phase was dried over MgSO₄, filtered, and concentrated under reduced pressure. The residue was purified by flash chromatography eluting with a mixture of EtOAc and n-heptane (20/80; v/v) to give 17.36 g (87%) of methyl 9-(4-hydroxyphenyl)-6,7-dihydro-5H-benzo[7]annulene-3-carboxylate. LC/MS (m/z, MH+): 295

### Step 2: Tert-butyl (S)-3-(4-(3-(methoxycarbonyl)-6,7-dihydro-5H-benzo[7]annulen-9-yl)phenoxy)pyrrolidine-1-carboxylate

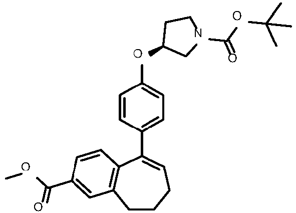

To a mixture of methyl 9-(4-hydroxyphenyl)-6,7-dihydro-5H-benzo[7]annulene-3-carboxylate (5 g, 17 mmol), tert-butyl (R)-3-hydroxypyrrolidine-1-carboxylate (3.82 g, 20.38 mmol) and N,N,N',N'-tetramethylazodicarboxamide (5.97 g, 33.97 mmol) in THF (100 ml) was added PPh₃ (8.91 g, 33.97 mmol). The reaction mixture was stirred at RT for 18 hours. EtOAc (500 ml) and water (250 ml) were added. The organic phase was separated, dried over Na₂SO₄, filtered and concentrated under reduced pressure and the residue obtained was purified by flash chromatography eluting with a gradient of DCM / MeOH (100/00 to 98/02), to give 5.9 g (75%) of tert-butyl (S)-3-(4-(3-(methoxycarbonyl)-6,7-dihydro-5H-benzo[7]annulen-9-yl)phenoxy)pyrrolidine-1-carboxylate.

LC/MS (m/z, MH+): 464

### Step 3: Methyl (S)-8-bromo-9-(4-(pyrrolidin-3-yloxy)phenyl)-6,7-dihydro-5H-benzo[7]annulene-3-carboxylate

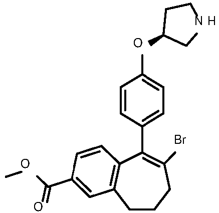

To a solution of tert-butyl (S)-3-(4-(3-(methoxycarbonyl)-6,7-dihydro-5H-benzo[7]annulen-9-yl)phenoxy)pyrrolidine-1-carboxylate (5.9 g, 12.73 mmol) in DCM (340 ml) was added pyridinium tribromide (4.48 g, 14 mmol). The reaction mixture was stirred at RT for 18 hours. A concentrated aqueous solution of NaHCO₃ was added. After decantation, the organic phase was dried over Na₂SO₄, filtered, concentrated under reduced pressure. To the residue obtained was added dioxane (100 ml) and HCl in dioxane 4 N (30.42 ml, 121.67 mmol). After 30 minutes of stirring at RT, the reaction mixture was concentrated under reduced pressure and the residue obtained was purified on strong cation exchange (SCX) column to give 4.95 g (92%) of methyl (S)-8-bromo-9-(4-(pyrrolidin-3-yloxy)phenyl)-6,7-dihydro-5H-benzo[7]annulene-3-carboxylate

LC/MS (m/z, MH+): 442

### Step 4: Methyl (S,E)-8-bromo-9-(4-((1-(4-(tert-butoxy)-4-oxobut-2-en-1-yl)pyrrolidin-3-yl)oxy)phenyl)-6,7-dihydro-5H-benzo[7]annulene-3-carboxylate

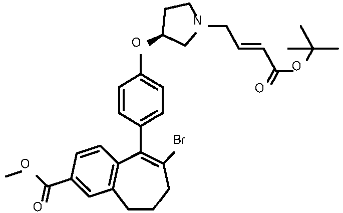

A mixture of methyl (S)-8-bromo-9-(4-(pyrrolidin-3-yloxy)phenyl)-6,7-dihydro-5H-benzo[7]annulene-3-carboxylate (2.34 g, 5.29 mmol), DIEA (1.37 g, 10.58 mmol) and tert-butyl (E)-4-bromobut-2-enoate (1.29 g, 5.29 mmol) in THF (40 ml) was stirred at RT for 18 hours. EtOAc (200 ml) and concentrated aqueous solution of NaCl (50 ml) were added. After decantation, the organic phase was dried over Na₂SO₄, filtered, concentrated under reduced pressure and the residue obtained was purified by flash chromatography eluting with a gradient of DCM / MeOH (100/00 to 98/02), to give 1.6 g (52%) of methyl (S,E)-8-bromo-9-(4-((1-(4-(tert-butoxy)-4-oxobut-2-en-1-yl)pyrrolidin-3-yl)oxy)phenyl)-6,7-dihydro-5H-benzo [7]annulene-3-carboxylate.

LC/MS (m/z, MH+): 582

### Step 5: (S,E)-4-(3-(4-(8-Bromo-3-(methoxycarbonyl)-6,7-dihydro-5H-benzo[7]annulen-9-yl)phenoxy)pyrrolidin-1-yl)but-2-enoic acid

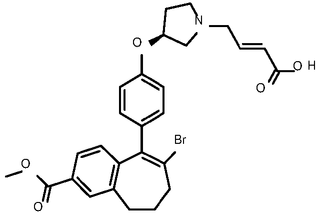

To a solution of methyl (S,E)-8-bromo-9-(4-((1-(4-(tert-butoxy)-4-oxobut-2-en-1-yl)pyrrolidin-3-yl)oxy)phenyl)-6,7-dihydro-5H-benzo[7]annulene-3-carboxylate (2.4 g, 4.12 mmol) in DCM (4 ml) was added TFA (3.17 ml, 41.2 mmol). The reaction mixture was stirred at RT for 18 hours. The reaction mixture was concentrated under reduced pressure and the residue obtained was purified by flash chromatography eluting with a gradient of DCM / MeOH (100/00 to 90/10), to give 1.5 g (69%) of (S,E)-4-(3-(4-(8-bromo-3-(methoxycarbonyl)-6,7-dihydro-5H-benzo[7]annulen-9-yl)phenoxy)pyrrolidin-1-yl)but-2-enoic acid.

LC/MS (m/z, MH+): 526

### Step 6: Methyl (S,E)-8-bromo-9-(4-((1-(4-(dimethylamino)-4-oxobut-2-en-1-yl)pyrrolidin-3-yl)oxy)phenyl)-6,7-dihydro-5H-benzo[7]annulene-3-carboxylate

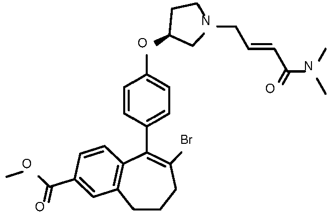

A mixture of (S,E)-4-(3-(4-(8-bromo-3-(methoxycarbonyl)-6,7-dihydro-5H-benzo[7]annulen-9-yl)phenoxy)pyrrolidin-1-yl)but-2-enoic acid (1.5 g, 2.85 mmol) DMF (10 ml), DIEA (1.1 g, 8.53 mmol) and HATU (1.3 g, 3.42 mmol) was stirred at RT for 10 minutes. Dimethylamine 2M in THF (2.14 ml, 4.27 mmol) was added and the reaction mixture was stirred at RT for 2 hours. EtOAc (50 ml) and water (50 ml) were added. After decantation, the organic phase was dried over MgSO₄, filtered, and concentrated under reduced pressure and the residue obtained was purified by flash chromatography eluting with DCM/acetone 25/75 to give 1.58 g (100%) of methyl (S,E)-8-bromo-9-(4-((1-(4-(dimethylamino)-4-oxobut-2-en-1-yl)pyrrolidin-3-yl)oxy)phenyl)-6,7-dihydro-5H-benzo[7]annulene-3-carboxylate.

LC/MS (m/z, MH+): 553

### Intermediate 2: (E)-4-Bromo-N,N-dimethyl-but-2-enamide

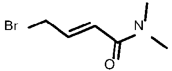

To a mixture of (E)-4-bromobut-2-enoic acid (20 g, 121.23 mmol) in DCM (50 ml) cooled at 0°C were added DIEA (17.24 g, 133.35 mmol) and HBTU (46.71 g, 145.47 mmol). The reaction mixture was stirred for 30 minutes at 0°C. The reaction mixture was cooled at - 10°C, then was added dropwise dimethylamine 2M in THF (75.77 ml, 151.53 mmol). The reaction mixture was stirred at 0°C for 2 hours. DCM (150 ml) and water (150 ml) were added. After decantation, the organic phase was dried over MgSO₄, filtered, and concentrated under reduced pressure and the residue obtained was purified by flash chromatography eluting with a gradient of heptane/EtOAc from 100/00 to 00/100 to give 9.33 g (40%) of (E)-4-bromo-N,N-dimethyl-but-2-enamide.

LC/MS (m/z, MH+): 192

### Intermediate 3: Methyl 8-(2,4-dichlorophenyl)-9-(4,4,5,5-tetramethyl-1,3,2-dioxaborolan-2-yl)-6,7-dihydro-5H-benzo[7]annulene-3-carboxylate

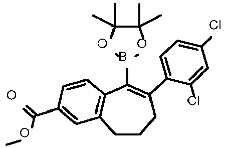

A mixture of methyl 8-(2,4-dichlorophenyl)-9-(((trifluoromethyl)sulfonyl)oxy)-6,7-dihydro-5H-benzo[7]annulene-3-carboxylate (prepared according to WO2020/049153) (15 g, 30.29 mmol) in toluene (300 ml), Pd(PPh₃)₂Cl₂ (0.87 g, 1.21 mmol), PPh₃ (0.64 g, 2.42 mmol), bis(pinacolato)diboron (19.23 g, 75.71 mmol) and PhOK (8.43 g, 60.57 mmol) was heated to 75°C for 24 h. After cooling to RT, EtOAc (300 mL) and water (300 mL) were added. After decantation, the organic phase was dried over Na₂SO₄, filtered, concentrated under reduced pressure and the residue obtained was purified by flash chromatography eluting with a gradient of heptane / EtOAc (from 100/00 to 90/10) to give 11.73 g (82%) of methyl 8-(2,4-dichlorophenyl)-9-(4,4,5,5-tetramethyl-1,3,2-dioxaborolan-2-yl)-6,7-dihydro-5H-benzo[7]annulene-3-carboxylate as a white solid.

LC/MS (m/z, MH+): 473

### Intermediate 4: (E)-4-[3-(4-Bromophenoxy)azetidin-1-yl]-N,N-dimethyl-but-2-enamide

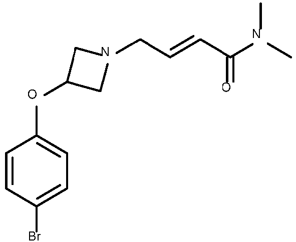

### Step 1: Tert-butyl 3-(4-bromophenoxy)azetidine-1-carboxylate

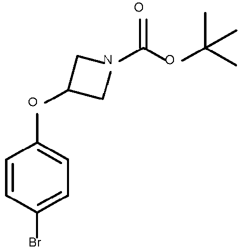

A mixture of 4-bromphenol (5 g, 29 mmol), 1-boc-3-iodoazetidine (12 g, 43 mmol) and Cs₂CO₃ (19 g, 58 mmol) in DMF (50 ml) was heated at 65°C for 6 hours. After cooling to RT, addition of EtOAc (200 ml) and water (200 ml). After decantation, the organic phase was dried over MgSO₄, filtered, and concentrated under reduced pressure and the residue obtained was purified by flash chromatography eluting with a gradient of DCM/MeOH from 100/00 to 99/01 to give 9.33 g (98%) of tert-butyl 3-(4-bromophenoxy)azetidine-1-carboxylate.

LC/MS (m/z, MH+): 328

### Step 2: 3-(4-Bromophenoxy)azetidine

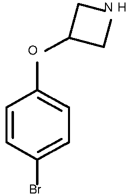

To a solution of tert-butyl 3-(4-bromophenoxy)azetidine-1-carboxylate (5.85 g, 17.8 mmol) in DCM (60 ml) was added dropwise TFA (13.2 ml, 178 mmol). The reaction mixture was stirred at RT for 3 hours. The reaction mixture was concentrated under reduced pressure and the residue obtained was purified by flash chromatography eluting with a gradient of DCM/MeOH/NH₄OH from 100/00/00 to 38/17/2 to give 3.51 g (83%) of 3-(4-bromophenoxy)azetidine.

LC/MS (m/z, MH+): 228

### Step 3: (E)-4-[3-(4-Bromophenoxy)azetidin-1-yl]-N,N-dimethyl-but-2-enamide

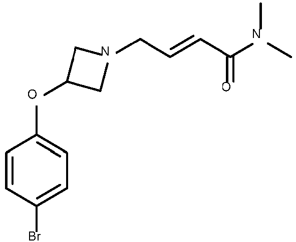

A mixture of 3-(4-bromophenoxy)azetidine (151 mg, 0.66 mmol), DIEA (171 mg, 1.32 mmol) and (E)-4-bromo-N,N-dimethyl-but-2-enamide (Intermediate 2) (127 mg, 0.66 mmol) in MeCN (4 ml) was stirred at RT for 3 hours. The reaction mixture was concentrated under reduced pressure. To the residue obtained, EtOAc (20 ml) and water (5 ml) were added. After decantation, the organic phase was dried over Na₂SO₄, filtered, concentrated under reduced pressure and the residue obtained was purified by flash chromatography eluting with a gradient of DCM / MeOH (100/00 to 95/05), to give 151 mg (68%) of (E)-4-[3-(4-bromophenoxy)azetidin-1-yl]-N,N-dimethyl-but-2-enamide.

LC/MS (m/z, MH+): 339

### Intermediate 5: Methyl (E)-8-(2,4-dichlorophenyl)-9-(4-((1-(4-(dimethylamino)-4-oxobut-2-en-1-yl)azetidin-3-yl)oxy)phenyl)-6,7-dihydro-5H-benzo[7]annulene-3-carboxylate

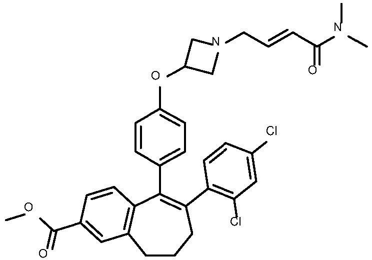

A mixture of methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) (116 mg, 0.24 mmol), (E)-4-[3-(4-bromophenoxy)azetidin-1-yl]-N,N-dimethyl-but-2-enamide (Intermediate 4) (75 mg, 0.22 mmol), [1,1'- bis(diphenylphosphino)ferrocene]dichloropalladium(II), complex with DCM (21 mg, 0.029 mmol), Cs₂CO₃ (185 mg, 0.57 mmol) in dioxane (4 ml) and water (1 ml) was heated to reflux for 4 hours. The organic phase was dried over MgSO₄, filtered, and concentrated under reduced pressure. After cooling to RT, EtOAc (20 ml) and water (5 ml) were added. After decantation, the organic phase was dried over Na₂SO₄, filtered, concentrated under reduced pressure and the residue obtained was purified by flash chromatography eluting with with a gradient of DCM / MeOH (100/00 to 95/05) to give 41.6 mg (31%) methyl (E)-8-(2,4-dichlorophenyl)-9-(4-((1-(4-(dimethylamino)-4-oxobut-2-en-1-yl)azetidin-3-yl)oxy)phenyl)-6,7-dihydro-5H-benzo[7]annulene-3-carboxylate as a yellow solid.

LC/MS (m/z, MH+): 605

### Intermediate 6: Methyl (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylate

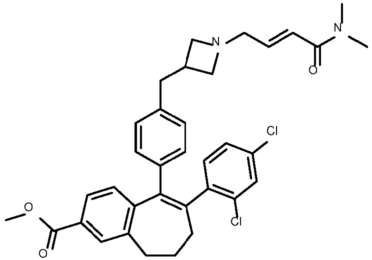

### Step 1: Methyl 9-(4-(azetidin-3-ylmethyl)phenyl)-8-(2,4-dichlorophenyl)-6,7-dihydro-5H-benzo[7]annulene-3-carboxylate

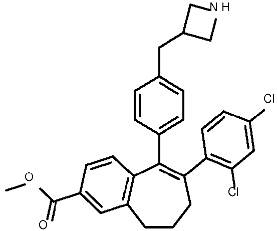

Step 1 of Intermediate 6 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and 3-[(4-bromophenyl)methyl]azetidine, 4-methylbenzenesulfonic acid to give 350 mg (34%) methyl 9-(4-(azetidin-3-ylmethyl)phenyl)-8-(2,4-dichlorophenyl)-6,7-dihydro-5H-benzo[7]annulene-3-carboxylate.

LC/MS (m/z, MH+): 492

### Step 2: Methyl (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylate

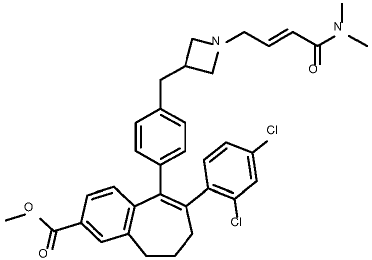

A mixture of methyl 9-(4-(azetidin-3-ylmethyl)phenyl)-8-(2,4-dichlorophenyl)-6,7-dihydro-5H-benzo[7]annulene-3-carboxylate (200 mg, 0.41 mmol), DIEA (68 mg, 0.53 mmol) and (E)-4-bromo-N,N-dimethyl-but-2-enamide (Intermediate 2) (78 mg, 0.41 mmol) in DMF (4 ml) was stirred at RT for 3 hours. EtOAc (10 ml), diethyl ether (10 ml) and water (20 ml) were added. After decantation, the organic phase was dried over Na₂SO₄, filtered, concentrated under reduced pressure and the residue obtained was purified by flash chromatography eluting with a gradient of cyclohexane / EtOAc (100/00 to 00/100), then a gradient of EtOAc / MeOH (100/00 to 90/10) and then DCM / MeOH (90/10) to give 110 mg (45%) of methyl (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylate.

LC/MS (m/z, MH+): 603

### Intermediate 7: Methyl (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylate

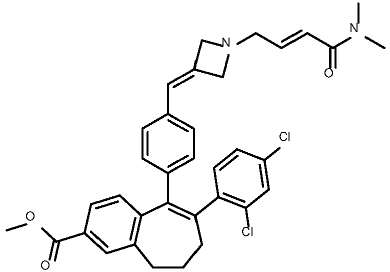

### Step 1: Tert-butyl 3-(4-bromobenzylidene)azetidine-1-carboxylate

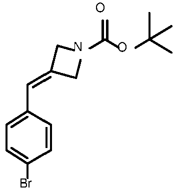

### Method 1:

To a solution of (4-bromobenzyl)triphenylphosphonium bromide (79.2 g, 155 mmol) in DMF (400 mL) was added NaH (6.18 g, 155 mmol, 60% purity in weight) at 0°C. The mixture was stirred at 0°C for 15 min. To this reaction mixture was added a solution of tert-butyl 3-oxoazetidine-1-carboxylate (24.1 g, 141 mmol) in DMF (160 mL). The reaction mixture was stirred at 20°C for 9 hours. The reaction mixture was quenched by addition of saturated aqueous solution of NH₄Cl (100 mL) at 0°C. The reaction mixture was filtered and the filtrate was concentrated under reduced pressure to give 60.0 g (crude) of tert-butyl 3-(4-bromobenzylidene)azetidine-1-carboxylate as a yellow solid.

LC/MS (m/z, MH+): 324

### Method 2:

A mixture of tert-butyl 3-methyleneazetidine-1-carboxylate (12.5 g, 73.9 mmol), 1-bromo-4-iodobenzene (23 g, 81.3 mmol), K₂CO₃ (20.4 g, 148 mmol), tetrabutylammonium bromide (23.8 g, 73.9 mmol) and palladium(II) acetate (1.66 g, 7.39 mmol) in DMF (125 mL) was heated to 60°C for 16 hours. After cooling to RT, EtOAc (500 ml) and water (500 mL) were added. After decantation, the organic phase was washed twice with water (500 ml), dried over MgSO₄, filtered, concentrated under reduced pressure and purified by flash chromatography, eluting with DCM to give 20.7 g (86%) of tert-butyl 3-(4-bromobenzylidene)azetidine-1-carboxylate as a beige solid.

LC/MS (m/z, MH+): 324

### Step 2: Tert-butyl 3-(4-(8-(2,4-dichlorophenyl)-3-(methoxycarbonyl)-6,7-dihydro-5H-benzo[7]annulen-9-yl)benzylidene)azetidine-1-carboxylate

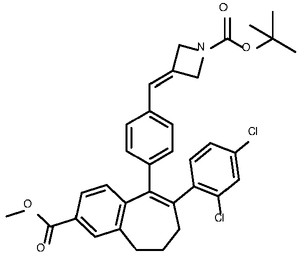

Step 2 of Intermediate 7 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl 3-(4-bromobenzylidene)azetidine-1-carboxylate to give 1.46 g (80%) of tert-butyl 3-(4-(8-(2,4-dichlorophenyl)-3-(methoxycarbonyl)-6,7-dihydro-5H-benzo[7]annulen-9-yl)benzylidene)azetidine-1-carboxylate.

LC/MS (m/z, MH+): 590

### Step 3: Methyl 9-(4-(azetidin-3-ylidenemethyl)phenyl)-8-(2,4-dichlorophenyl)-6,7-dihydro-5H-benzo[7]annulene-3-carboxylate, 2,2,2-trifluoroacetic acid

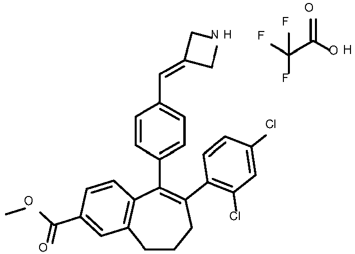

Step 3 of Intermediate 7 was prepared following a similar procedure to that of Step 2 of Intermediate 4 from tert-butyl 3-(4-(8-(2,4-dichlorophenyl)-3-(methoxycarbonyl)-6,7-dihydro-5H-benzo[7]annulen-9-yl)benzylidene)azetidine-1-carboxylate to give 1.58 g (crude) of methyl 9-(4-(azetidin-3-ylidenemethyl)phenyl)-8-(2,4-dichlorophenyl)-6,7-dihydro-SH-benzo[7]annulene-3-carboxylate, 2,2,2-trifluoroacetic acid which was used as such in the next step.

LC/MS (m/z, MH+): 490

### Step 4: Methyl (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylate

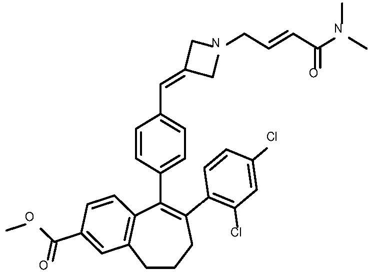

To a mixture of methyl 9-(4-(azetidin-3-ylidenemethyl)phenyl)-8-(2,4-dichlorophenyl)-6,7-dihydro-SH-benzo[7]annulene-3-carboxylate, 2,2,2-trifluoroacetic acid (303 mg, 0.50 mol), (E)-4-bromo-N,N-dimethyl-but-2-enamide (Intermediate 2) (106 mg, 0.55 mmol) in DCM (4 ml) was added NaOH 0.5 N (2 ml, 1.13 mmol). The reaction mixture was stirred at RT for 18 hours. After decantation, the organic phase was purified by flash chromatography using a gradient of EtOAc / MeOH (from 100/00 to 90/10) to give 126 mg (42%) of methyl (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylate.

LC/MS (m/z, MH+): 601

### Intermediate 8: Methyl (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate

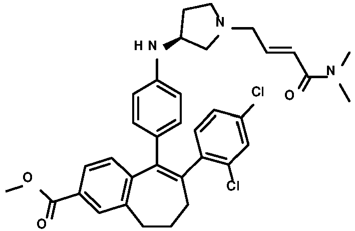

### Step 1: Tert-butyl (3S)-3-(4-bromoanilino)pyrrolidine-1-carboxylate

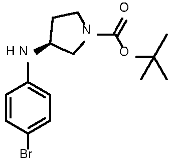

A mixture of 1-bromo-4-iodo-benzene (5 g, 18 mmol), (S)-1-Boc-3-aminopyrrolidine (6.6 g, 35 mmol), palladium(II) acetate (400 mg, 1.8 mmol), 4,5-bis(diphenylphosphino)-9,9-dimethylxanthene (1.4 g, 2.5 mmol) and Cs₂CO₃ (17 g, 53 mmol) in dioxane (50 ml) was heated at reflux for 5 hours. After cooling to RT, EtOAc (100 ml) and water (100 ml) were added. After decantation, the organic phase was dried over MgSO₄, filtered, concentrated under reduced pressure and purified by flash chromatography, eluting with a gradient of cyclohexane / EtOAc (from 88/12 to 00/100) to give 5 g (82%) of tert-butyl (3S)-3-(4-bromoanilino)pyrrolidine-1-carboxylate.

LC/MS (m/z, MH+): 341

### Step 2: Tert-butyl (S)-3-((4-(8-(2,4-dichlorophenyl)-3-(methoxycarbonyl)-6,7-dihydro-5H-benzo[7]annulen-9-yl)phenyl)amino)pyrrolidine-1-carboxylate

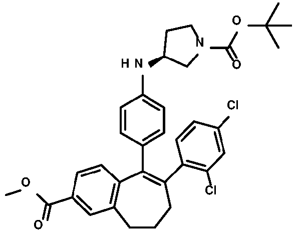

Step 2 of Intermediate 8 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl (3S)-3-(4-bromoanilino)pyrrolidine-1-carboxylate to give 98 mg (11%) of tert-butyl (S)-3-((4-(8-(2,4-dichlorophenyl)-3-(methoxycarbonyl)-6,7-dihydro-5H-benzo[7]annulen-9-yl)phenyl)amino)pyrrolidine-1-carboxylate.

LC/MS (m/z, MH+): 607

### Step 3: Methyl (S)-8-(2,4-dichlorophenyl)-9-(4-(pyrrolidin-3-ylamino)phenyl)-6,7-dihydro-5H-benzo[7]annulene-3-carboxylate

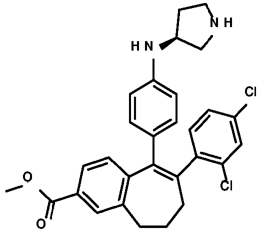

Step 3 of Intermediate 8 was prepared following a similar procedure to that of Step 2 of Intermediate 4 from tert-butyl (S)-3-((4-(8-(2,4-dichlorophenyl)-3-(methoxycarbonyl)-6,7-dihydro-5H-benzo[7]annulen-9-yl)phenyl)amino)pyrrolidine-1-carboxylate to give 49 mg (43%) of methyl (S)-8-(2,4-dichlorophenyl)-9-(4-(pyrrolidin-3-ylamino)phenyl)-6,7-dihydro-5H-benzo[7]annulene-3-carboxylate.

LC/MS (m/z, MH+): 507

### Step 4: Methyl (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate

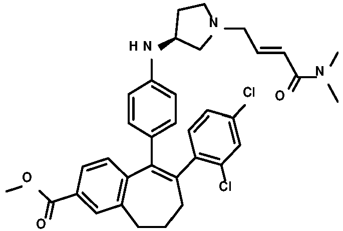

Step 4 of Intermediate 8 was prepared following a similar procedure to that of Step 3 of Intermediate 4 from methyl (S)-8-(2,4-dichlorophenyl)-9-(4-(pyrrolidin-3-ylamino)phenyl)-6,7-dihydro-5H-benzo[7]annulene-3-carboxylate and (E)-4-bromo-N,N-dimethyl-but-2-enamide (Intermediate 2) to give 39 mg (72%) of methyl (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate.

LC/MS (m/z, MH+): 618

### Intermediate 9: Methyl (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylate

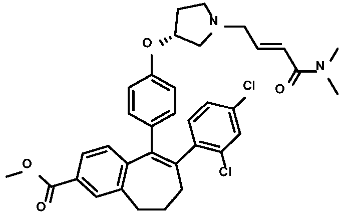

### Step 1: Tert-butyl (3R)-3-(4-bromophenoxy)pyrrolidine-1-carboxylate

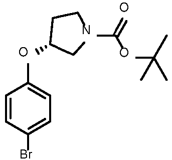

Step 1 of Intermediate 9 was prepared following a similar procedure to that of Step 2 of Intermediate 1 from 4-bromophenol and tert-butyl (3S)-3-hydroxypyrrolidine-1-carboxylate to give 9.26 g (93%) of tert-butyl (3R)-3-(4-bromophenoxy)pyrrolidine-1-carboxylate.

LC/MS (m/z, MH+): 342

### Step 2: Tert-butyl (R)-3-(4-(8-(2,4-dichlorophenyl)-3-(methoxycarbonyl)-6,7-dihydro-5H-benzo[7]annulen-9-yl)phenoxy)pyrrolidine-1-carboxylate

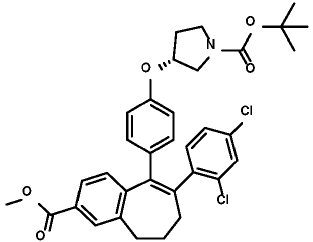

Step 2 of Intermediate 9 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl (3R)-3-(4-bromophenoxy)pyrrolidine-1-carboxylate to give 321 mg (36%) of tert-butyl (R)-3-(4-(8-(2,4-dichlorophenyl)-3-(methoxycarbonyl)-6,7-dihydro-5H-benzo[7]annulen-9-yl)phenoxy)pyrrolidine-1-carboxylate.

LC/MS (m/z, MH+): 608

### Step 3: Methyl (R)-8-(2,4-dichlorophenyl)-9-(4-(pyrrolidin-3-yloxy)phenyl)-6,7-dihydro-5H-benzo[7]annulene-3-carboxylate

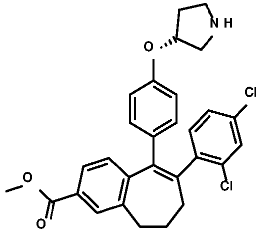

Step 3 of Intermediate 9 was prepared following a similar procedure to that of Step 2 of Intermediate 4 from tert-butyl (R)-3-(4-(8-(2,4-dichlorophenyl)-3-(methoxycarbonyl)-6,7-dihydro-5H-benzo[7]annulen-9-yl)phenoxy)pyrrolidine-1-carboxylate to give 220 mg (85%) of methyl (R)-8-(2,4-dichlorophenyl)-9-(4-(pyrrolidin-3-yloxy)phenyl)-6,7-dihydro-5H-benzo[7]annulene-3-carboxylate.

LC/MS (m/z, MH+): 508

### Step 4: Methyl (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylate

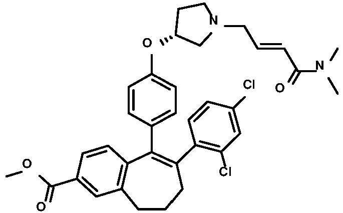

Step 4 of Intermediate 9 was prepared following a similar procedure to that of Step 3 of Intermediate 4 from methyl (R)-8-(2,4-dichlorophenyl)-9-(4-(pyrrolidin-3-yloxy)phenyl)-6,7-dihydro-5H-benzo[7]annulene-3-carboxylate and (E)-4-bromo-N,N-dimethyl-but-2-enamide (Intermediate 2) to give 113 mg (43%) of methyl (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylate.

LC/MS (m/z, MH+): 619

### Intermediate 10: Methyl 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylate

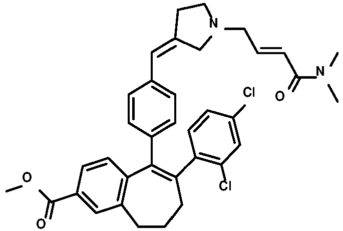

### Step 1: Tert-butyl 3-[(4-bromophenyl)-hydroxy-methyl]pyrrolidine-1-carboxylate

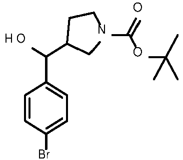

To a mixture of commercially available tert-butyl 3-(4-bromobenzoyl)pyrrolidine-1-carboxylate (5.82 g, 16.44 mmol) in MeOH (180ml) was added NaBH₄ (1.866 g, 49.33 mmol) and the mixture was stirred at RT for 2 hrs. A concentrated aqueous solution of NH₄Cl (100 ml) and EtOAc (300 ml) were added. After decantation, the organic phase was washed with brine (200 ml), dried over Na₂SO₄ filtered and concentrated under reduced pressure to give 5.8 g (crude) of tert-butyl 3-[(4-bromophenyl)-hydroxy-methyl]pyrrolidine-1-carboxylate.

LC/MS (m/z, MH+): 356

### Step 2: (3Z)-3-[(4-bromophenyl)methylene]pyrrolidine

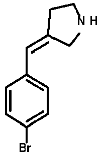

To a mixture of tert-butyl 3-[(4-bromophenyl)-hydroxy-methyl]pyrrolidine-1-carboxylate (5.8 g, 16.29 mmol) and water (12 ml) at 0°C was dropwise added sulfuric acid (42 ml). The cooling bath was removed, and the reaction mixture was stirred for 1 hour at RT. The reaction mixture was poured into a mixture of ice and water (500 ml). Powder NaHCO₃ was added to pH 9 followed by EtOAc (300 ml) and the organic phase was separated and dried over Na₂SO₄, filtered, concentrated under reduced pressure to give 1.97 g (51%) 3-[(4-bromophenyl)methylene]pyrrolidine as a mixture of (Z) and (E) isomers.

The mixture of (Z) and (E) isomers of 3-[(4-bromophenyl)methylene]-1-(3-fluoropropyl)pyrrolidine was separated on column Chiralcel OZ 20 µm (0.46x25cm), eluting with heptane/EtOH/TEA (95/5/0.1) to give 641 mg of (Z) isomer (3Z)-3-[(4-bromophenyl)methylene]pyrrolidine and 953 mg of (E) isomer (3E)-3-[(4-bromophenyl)methylene]pyrrolidine.

LC/MS (m/z, MH+): 238

### Step 3: Methyl (Z)-8-(2,4-dichlorophenyl)-9-(4-(pyrrolidin-3-ylidenemethyl)phenyl)-6,7-dihydro-5H-benzo[7]annulene-3-carboxylate

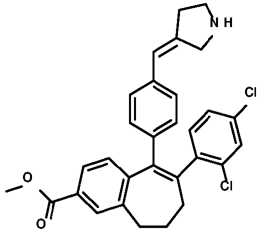

Step 3 of Intermediate 10 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and (3Z)-3-[(4-bromophenyl)methylene]pyrrolidine to give 97 mg (30%) of methyl (Z)-8-(2,4-dichlorophenyl)-9-(4-(pyrrolidin-3-ylidenemethyl)phenyl)-6,7-dihydro-5H-benzo[7]annulene-3-carboxylate.

LC/MS (m/z, MH+): 504

### Step 4: Methyl 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylate

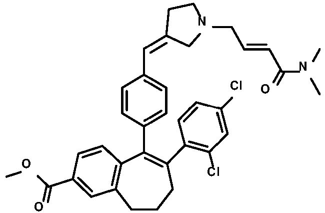

Step 4 of Intermediate 10 was prepared following a similar procedure to that of Step 3 of Intermediate 4 from methyl (Z)-8-(2,4-dichlorophenyl)-9-(4-(pyrrolidin-3-ylidenemethyl)phenyl)-6,7-dihydro-5H-benzo[7]annulene-3-carboxylate and (E)-4-bromo-N,N-dimethyl-but-2-enamide (Intermediate 2) to give 106 mg (49%) of methyl 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylate.

LC/MS (m/z, MH+): 615

### Intermediate 11: Methyl 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylate

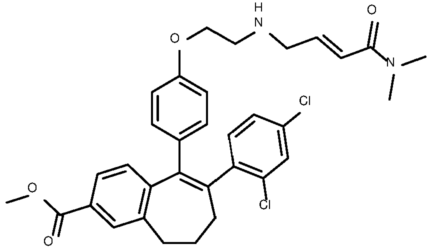

### Step 1: (E)-4-[2-(4-Bromophenoxy)ethylamino]-N,N-dimethyl-but-2-enamide

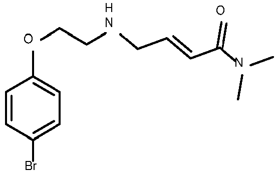

Step 1 of Intermediate 11 was prepared following a similar procedure to that of Step 3 of Intermediate 4 from 2-(4-bromophenoxy)ethanamine, hydrochloride and (E)-4-bromo-N,N-dimethyl-but-2-enamide (Intermediate 2) to give 327 mg (36%) of (E)-4-[2-(4-bromophenoxy)ethylamino]-N,N-dimethyl-but-2-enamide.

LC/MS (m/z, MH+): 327

### Step 2: Tert-butyl N-[2-(4-bromophenoxy)ethyl]-N-[(E)-4-(dimethylamino)-4-oxo-but-2-enyl]carbamate

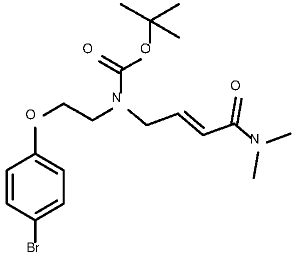

To a mixture of (E)-4-[2-(4-bromophenoxy)ethylamino]-N,N-dimethyl-but-2-enamide (460 mg, 1.40 mmol) and DIEA (0.48 ml, 2.81 mmol) in DCM (5 ml) cooled at 0°C was added di-tert-butyl dicarbonate (368 mg, 1.69 mmol). The cooling bath was removed and the reaction mixture was stirred for 5 hours at RT. Water (5 ml) and DCM (10 ml) were added. After decantation, the organic phase was dried over Na₂SO₄, filtered, concentrated under reduced pressure and the residue obtained was purified by flash chromatography eluting with a gradient of DCM/MeOH from 100/00 to 98/02 to give 414 mg (69%) tert-butyl N-[2-(4-bromophenoxy)ethyl]-N-[(E)-4-(dimethylamino)-4-oxo-but-2-enyl]carbamate.

LC/MS (m/z, MH+): 427

### Step 3: Methyl (E)-9-(4-(2-((tert-butoxycarbonyl)(4-(dimethylamino)-4-oxobut-2-en-1-yl)amino)ethoxy)phenyl)-8-(2,4-dichlorophenyl)-6,7-dihydro-5H-benzo[7]annulene-3-carboxylate

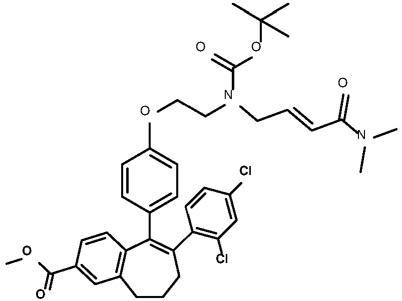

Step 3 of Intermediate 11 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl N-[2-(4-bromophenoxy)ethyl]-N-[(E)-4-(dimethylamino)-4-oxo-but-2-enyl]carbamate to give 261 mg (39%) of methyl (E)-9-(4-(2-((tert-butoxycarbonyl)(4-(dimethylamino)-4-oxobut-2-en-1-yl)amino)ethoxy)phenyl)-8-(2,4-dichlorophenyl)-6,7-dihydro-5H-benzo[7]annulene-3-carboxylate.

LC/MS (m/z, MH+): 693

### Step 4: Methyl (E)-8-(2,4-dichlorophenyl)-9-(4-(2-((4-(dimethylamino)-4-oxobut-2-en-1-yl)amino)ethoxy)phenyl)-6,7-dihydro-5H-benzo[7]annulene-3-carboxylate

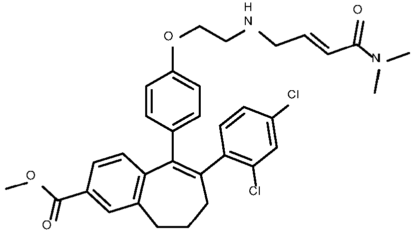

Step 4 of Intermediate 11 was prepared following a similar procedure to that of Step 2 of Intermediate 4 from methyl (E)-9-(4-(2-((tert-butoxycarbonyl)(4-(dimethylamino)-4-oxobut-2-en-1-yl)amino)ethoxy)phenyl)-8-(2,4-dichlorophenyl)-6,7-dihydro-5H-benzo[7]annulene-3-carboxylate to give 114 mg (52%) of methyl (E)-8-(2,4-dichlorophenyl)-9-(4-(2-((4-(dimethylamino)-4-oxobut-2-en-1-yl)amino)ethoxy)phenyl)-6,7-dihydro-5H-benzo[7]annulene-3-carboxylate.

LC/MS (m/z, MH+): 593

### Intermediate 12: Methyl 8-(2,4-dichlorophenyl)-9-(4-(((trifluoromethyl)sulfonyl)oxy)phenyl)-6,7-dihydro-5H-benzo[7]annulene-3-carboxylate

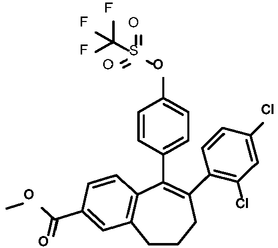

### Step 1: Methyl 8-(2,4-dichlorophenyl)-9-(4-hydroxyphenyl)-6,7-dihydro-5H-benzo [7] annulene-3-carboxylate

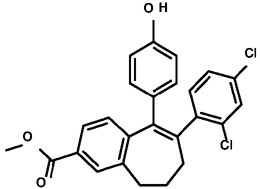

To a mixture of methyl 8-(2,4-dichlorophenyl)-9-(((trifluoromethyl)sulfonyl)oxy)-6,7-dihydro-5H-benzo[7]annulene-3-carboxylate (prepared according to WO2020/049153) (5 g, 10 mmol), 4-(4,4,5,5-tetramethyl-1,3,2-dioxaborolan-2-yl)phenol (2.7 g, 12 mmol mmol) and [1,1'-bis(diphenylphosphino)ferrocene]dichloropalladium(II), complex with DCM (0.74 g, 1 mmol) in dioxane (40 ml) and water (10 ml) was added Cs₂CO₃ (6.6 g, 20 mmol). The reaction mixture was heated to reflux for 24 hours. Water (100 ml) and EtOAc (200 ml) were added. The organic phase was dried over MgSO₄, filtered, and concentrated under reduced pressure. The residue was purified by flash chromatography eluting with a gradient of cyclohexane/EtOAc (from 89/11 to 00/100) to give 3.8 g (87%) of methyl 8-(2,4-dichlorophenyl)-9-(4-hydroxyphenyl)-6,7-dihydro-5H-benzo[7]annulene-3-carboxylate.

LC/MS (m/z, MH+): 439

### Step 2: Methyl 8-(2,4-dichlorophenyl)-9-(4-(((trifluoromethyl)sulfonyl)oxy)phenyl)-6,7-dihydro-5H-benzo[7]annulene-3-carboxylate

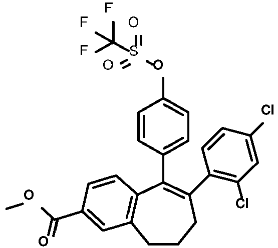

To a mixture of methyl 8-(2,4-dichlorophenyl)-9-(4-hydroxyphenyl)-6,7-dihydro-5H-benzo[7]annulene-3-carboxylate (2 g, 4.6 mmol) in DCM (20 ml) cooled at 0°C were added pyridine (0.65 ml, 8.2 mmol) and trifluoromethanesulfonic anhydride (2.3 g, 8.1 mmol). The reaction mixture was stirred at 0°C for 15 minutes and RT for 2 hours. DCM (50 ml) and water (20 ml) were added. After decantation, the organic phase was dried over MgSO₄, filtered, concentrated under reduced pressure, and purified by flash chromatography, eluting with a gradient of cyclohexane / EtOAc (from 86/14 to 90/10) to give 2.31 g (89%) of methyl 8-(2,4-dichlorophenyl)-9-(4-(((trifluoromethyl)sulfonyl)oxy)phenyl)-6,7-dihydro-5H-benzo[7]annulene-3-carboxylate.

LC/MS (m/z, MH+): 571

### Intermediate 13: Methyl (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate

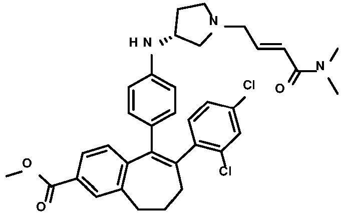

### Step 1: Tert-butyl (R)-3-((4-(8-(2,4-dichlorophenyl)-3-(methoxycarbonyl)-6,7-dihydro-5H-benzo[7]annulen-9-yl)phenyl)amino)pyrrolidine-1-carboxylate

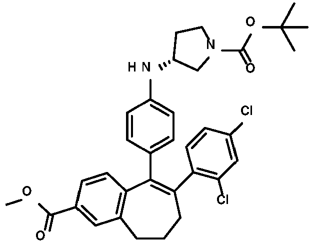

A mixture of methyl 8-(2,4-dichlorophenyl)-9-(4-(((trifluoromethyl)sulfonyl)oxy)phenyl)-6,7-dihydro-5H-benzo[7]annulene-3-carboxylate (Intermediate 12) (0.715 g, 1.25 mmol), (R)-1-Boc-3-aminopyrrolidine (0.589 g, 3.16 mmol), palladium(II) acetate (28 mg, 0.125 mmol), 4,5-bis(diphenylphosphino)-9,9-dimethylxanthene (101 mg, 0.175 mmol) and Cs₂CO₃ (1.22 g, 3.75 mmol) in dioxane (10 ml) was heated to reflux for 5 hours. After cooling to RT, EtOAc (50 ml) and water (50 ml) were added. After decantation, the organic phase was dried over MgSO₄, filtered, concentrated under reduced pressure and purified by flash chromatography, eluting with a gradient of cyclohexane / EtOAc (from 87/13 to 00/100) to give 207 mg (19%) of tert-butyl (R)-3-((4-(8-(2,4-dichlorophenyl)-3-(methoxycarbonyl)-6,7-dihydro-5H-benzo[7]annulen-9-yl)phenyl)amino)pyrrolidine- 1-carboxylate.

LC/MS (m/z, MH+): 607

### Step 2: Methyl (R)-8-(2,4-dichlorophenyl)-9-(4-(pyrrolidin-3-ylamino)phenyl)-6,7-dihydro-5H-benzo[7]annulene-3-carboxylate

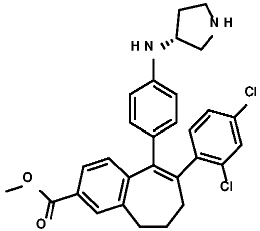

Step 2 of Intermediate 13 was prepared following a similar procedure to that of Step 2 of Intermediate 4 from tert-butyl (R)-3-((4-(8-(2,4-dichlorophenyl)-3-(methoxycarbonyl)-6,7-dihydro-5H-benzo[7]annulen-9-yl)phenyl)amino)pyrrolidine-1-carboxylate to give 136 mg (82%) of methyl (R)-8-(2,4-dichlorophenyl)-9-(4-(pyrrolidin-3-ylamino)phenyl)-6,7-dihydro-5H-benzo[7]annulene-3-carboxylate.

LC/MS (m/z, MH+): 507

### Step 3: Methyl (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate

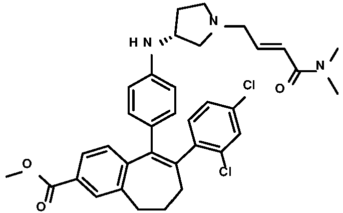

Step 3 of Intermediate 13 was prepared following a similar procedure to that of Step 3 of Intermediate 4 from methyl (R)-8-(2,4-dichlorophenyl)-9-(4-(pyrrolidin-3-ylamino)phenyl)-6,7-dihydro-5H-benzo[7]annulene-3-carboxylate and (E)-4-bromo-N,N-dimethyl-but-2-enamide (Intermediate 2) to give 80 mg (51%) of methyl (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate.

LC/MS (m/z, MH+): 618

### Intermediate 14: Methyl (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylate

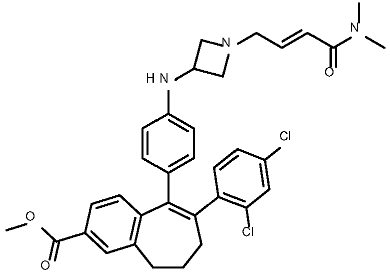

### Step 1: Tert-butyl 3-((4-(8-(2,4-dichlorophenyl)-3-(methoxycarbonyl)-6,7-dihydro-5H-benzo[7]annulen-9-yl)phenyl)amino)azetidine-1-carboxylate

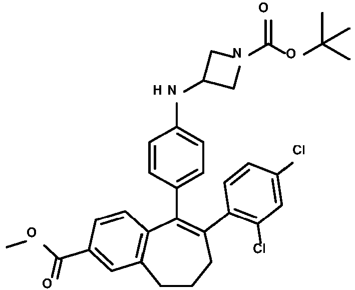

Step 1 of Intermediate 14 was prepared following a similar procedure to that of Step 1 of Intermediate 13 from methyl 8-(2,4-dichlorophenyl)-9-(4-(((trifluoromethyl)sulfonyl)oxy)phenyl)-6,7-dihydro-5H-benzo[7]annulene-3-carboxylate (Intermediate 12) and tert-butyl 3-aminoazetidine-1-carboxylate to give 372 mg (60%) of 1-tert-butyl 3-((4-(8-(2,4-dichlorophenyl)-3-(methoxycarbonyl)-6,7-dihydro-5H-benzo[7]annulen-9-yl)phenyl)amino)azetidine-1-carboxylate.

LC/MS (m/z, MH+): 594

### Step 2: Methyl 9-(4-(azetidin-3-ylamino)phenyl)-8-(2,4-dichlorophenyl)-6,7-dihydro-5H-benzo[7]annulene-3-carboxylate

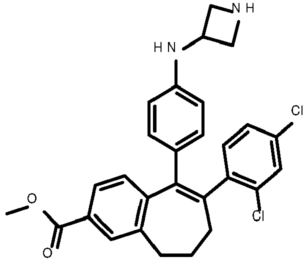

Step 2 of Intermediate 14 was prepared following a similar procedure to that of Step 2 of Intermediate 4 from tert-butyl 3-((4-(8-(2,4-dichlorophenyl)-3-(methoxycarbonyl)-6,7-dihydro-5H-benzo[7]annulen-9-yl)phenyl)amino)azetidine-1-carboxylate to give 226 mg (74%) of methyl 9-(4-(azetidin-3-ylamino)phenyl)-8-(2,4-dichlorophenyl)-6,7-dihydro-5H-benzo[7]annulene-3-carboxylate.

LC/MS (m/z, MH+): 494

### Step 3: Methyl (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylate

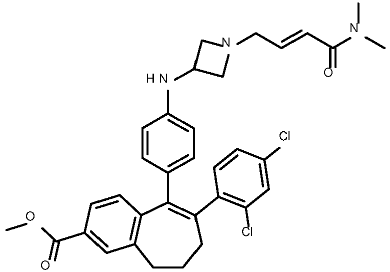

Step 3 of Intermediate 14 was prepared following a similar procedure to that of Step 3 of Intermediate 4 from methyl 9-(4-(azetidin-3-ylamino)phenyl)-8-(2,4-dichlorophenyl)-6,7-dihydro-5H-benzo[7]annulene-3-carboxylate and (E)-4-bromo-N,N-dimethyl-but-2-enamide (Intermediate 2) to give 107 mg (39%) of methyl (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylate.

LC/MS (m/z, MH+): 604

### Intermediate 15: methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 1

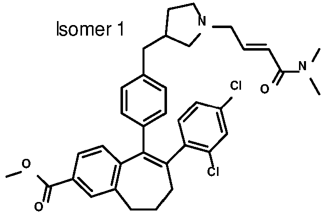

### Step 1: Tert-butyl 3-[(4-bromophenyl)methyl]pyrrolidine-1-carboxylate Isomer 1 and Isomer 2

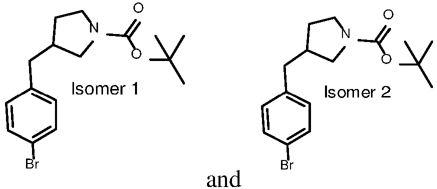

To a solution of commercially available 3-[(4-bromophenyl)methyl]pyrrolidine (35.0 g, 145 mmol) and TEA (29.5 g, 292 mmol) in DCM (250 ml) was added DMAP (1.78 g, 14.57 mmol) and Boc₂O (33.4 g, 153 mmol) at RT and the suspension was stirred at RT for 12 hours. The reaction mixture was quenched by addition of water (300 ml) and extracted with DCM (100 ml). The organic phase was washed with brine (200 ml), dried over Na₂SO₄ filtered and concentrated under reduced pressure and the residue obtained was purified by flash chromatography, eluting with a gradient of petroleum ether / ethyl acetate: from 98/02 to 50/50 to give 37 g (73%) of racemic tert-butyl 3-[(4-bromophenyl)methyl]pyrrolidine-1-carboxylate as a light yellow oil.

The racemic tert-butyl 3-[(4-bromophenyl)methyl]pyrrolidine-1-carboxylate (37 g, 107 mmol) was separated by preparative SFC (column: DAICEL CHIRALCEL OJ (250x50mm,10µm); CO₂ mobile phase with 20% of a solution of 0.1% NH₄OH in EtOH) to give 17 g of tert-butyl 3-[(4-bromophenyl)methyl]pyrrolidine-1-carboxylate Isomer 1 and 17 g of tert-butyl 3-[(4-bromophenyl)methyl]pyrrolidine-1-carboxylate Isomer 2.

LC/MS (m/z, MH+): 340

### Step 2: tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 1

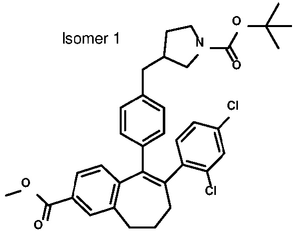

Step 2 of Intermediate 15 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl 3-(4-bromobenzyl)pyrrolidine-1-carboxylate, Isomer 1 to give 470 mg (53%) of tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 1.

LC/MS (m/z, MH+): 606

### Step 3: methyl 8-(2,4-dichlorophenyl)-9-(4-(pyrrolidin-3-ylmethyl)phenyl)-6,7-dihydro-5H-benzo[7]annulene-3-carboxylate , Isomer 1

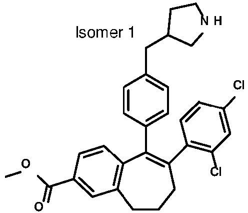

Step 3 of Intermediate 15 was prepared following a similar procedure to that of Step 2 of Intermediate 4 from tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 1 to give 341 mg (87%) of methyl 8-(2,4-dichlorophenyl)-9-(4-(pyrrolidin-3-ylmethyl)phenyl)-6,7-dihydro-5H-benzo[7]annulene-3-carboxylate , Isomer 1.

LC/MS (m/z, MH+): 506

### Step 4: methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 1

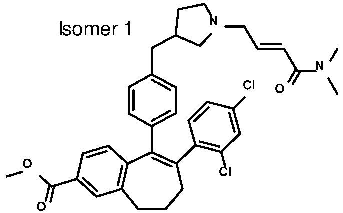

Step 4 of Intermediate 15 was prepared following a similar procedure to that of Step 3 of Intermediate 4 from methyl 8-(2,4-dichlorophenyl)-9-(4-(pyrrolidin-3-ylmethyl)phenyl)-6,7-dihydro-5H-benzo[7]annulene-3-carboxylate, Isomer 1 and (E)-4-bromo-N,N-dimethyl-but-2-enamide (Intermediate 2) to give 229 mg (56%) of methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 1.

LC/MS (m/z, MH+): 617

### Intermediate 16: methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 2

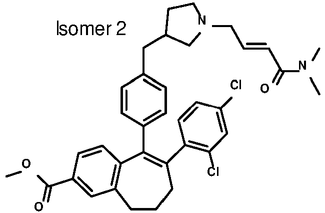

### Step 1: tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 2

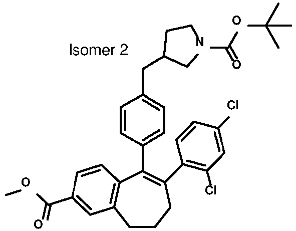

Step 1 of Intermediate 16 was prepared following a similar procedure to that of Intermediate 5 from methyl 8-(2,4-dichlorophenyl)-9-(4,4,5,5-tetramethyl-1,3,2-dioxaborolan-2-yl)-6,7-dihydro-5H-benzo[7]annulene-3-carboxylate (Intermediate 3) and tert-butyl 3-(4-bromobenzyl)pyrrolidine-1-carboxylate, Isomer 2 to give 390 mg (44%) of tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 2.

LC/MS (m/z, MH+): 606

### Step 2: methyl 8-(2,4-dichlorophenyl)-9-(4-(pyrrolidin-3-ylmethyl)phenyl)-6,7-dihydro-5H-benzo[7]annulene-3-carboxylate , Isomer 2

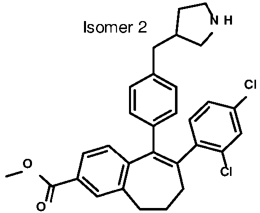

Step 3 of Intermediate 16 was prepared following a similar procedure to that of Step 2 of Intermediate 4 from tert-butyl 3-(4-(8-(2,4-dichlorophenyl)-3-(methoxycarbonyl)-6,7-dihydro-5H-benzo[7]annulen-9-yl)benzyl)pyrrolidine-1-carboxylate , Isomer 2 to give 320 mg (98%) of methyl 8-(2,4-dichlorophenyl)-9-(4-(pyrrolidin-3-ylmethyl)phenyl)-6,7-dihydro-5H-benzo[7]annulene-3-carboxylate , Isomer 2.

LC/MS (m/z, MH+): 506

### Step 3: methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 2

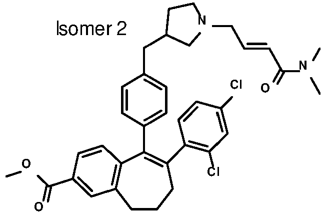

Step 3 of Intermediate 16 was prepared following a similar procedure to that of Step 3 of Intermediate 4 from methyl 8-(2,4-dichlorophenyl)-9-(4-(pyrrolidin-3-ylmethyl)phenyl)-6,7-dihydro-5H-benzo[7]annulene-3-carboxylate, Isomer 2 and (E)-4-bromo-N,N-dimethyl-but-2-enamide (Intermediate 2) to give 23 mg (53%) of methyl (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylate , Isomer 2.

LC/MS (m/z, MH+): 617

### Intermediate 17: Methyl (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylate

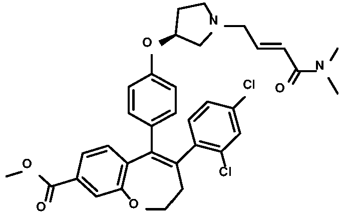

### Step 1: 5-Oxo-2,3,4,5-tetrahydrobenzo[b]oxepine-8-yl trifluoromethanesulfonate

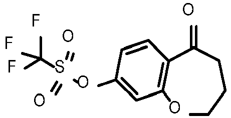

To a mixture of 8-hydroxy-3,4-dihydrobenzo[b]oxepine-5(2H)-one (4.2 g, 23.57 mmol) (prepared according to WO2018091153) and pyridine (2.82 g, 2.89 ml, 35.36 mmol) in DCM (120 ml) cooled at -20°C was dropwise added trifluoromethanesulfonic anhydride (8.14 g, 6 ml, 28.28 mmol). The reaction mixture was stirred at 0°C for 30 minutes. Water (50 ml) was added. The organic phase was separated and washed with an aqueous saturated solution of NaHCO₃ (50 ml). The organic phase was dried over MgSO₄, filtered, and concentrated under reduced pressure to give 7.30 g (100%) of 5-oxo-2,3,4,5-tetrahydrobenzo[b]oxepine-8-yl trifluoromethanesulfonate.

LC/MS (m/z, MH+): 311

### Step 2: Methyl 5-oxo-2,3,4,5-tetrahydrobenzo[b]oxepine-8-carboxylate

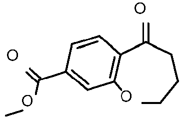

To a solution of compound 5-oxo-2,3,4,5-tetrahydrobenzo[b]oxepine-8-yl trifluoromethanesulfonate (7.4 g, 23.85 mmol) in DMF (30 ml) and MeOH (15 ml) was added DIEA (3.15 g, 4.16 ml, 23.85 mmol) and Pd(dppf)Cl₂ complex with DCM (1.10 g, 1.43 mmol), the suspension was degassed and purged three times with CO. The mixture was stirred under CO (5 bars) at 75 °C for 2 hours. The reaction was filtered through celite. The filtrate was diluted with water (400 ml) and extracted three times with EtOAc (300 ml). The combined organic phases were dried over Na₂SO₄, filtered, concentrated under reduced pressure, and the residue obtained was purified by flash chromatography eluting with a gradient of heptane/ethyl acetate: from 85/15 to 80/20 to give 4.6 g (88%) methyl 5-oxo-2,3,4,5-tetrahydrobenzo[b]oxepine-8-carboxylate.

LC/MS (m/z, MH+): 221

### Step 3: Methyl 4-(2,4-dichlorophenyl)-5-oxo-2,3,4,5-tetrahydrobenzo[b]oxepine-8-carboxylate

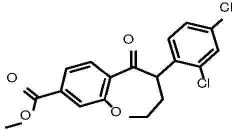

A mixture of 1-bromo-2,4-difluoro-benzene (4.21 g, 18.1 mmol), methyl 5-oxo-2,3,4,5-tetrahydrobenzo[b]oxepine-8-carboxylate (2 g, 9.1 mmol), Cs₂CO₃ (11.92 g, 36.58 mmol) in toluene (12 ml). After addition of XANTPHOS (1.1 g, 1.8 mmol) and Pd₂(dba)₃ (0.83 g, 0.91 mmol), the reaction mixture was heated to reflux for 18 hours. After cooling to RT, water (250 ml) and DCM (20 ml) were added. After decantation, the organic phase was dried over MgSO₄, filtered and concentrated under reduced pressure. The residue obtained was purified by flash chromatography, eluting with a gradient of Heptane/EtOAc from 100/00 to 90/10 to give 1.85 g (56%) of methyl 4-(2,4-dichlorophenyl)-5-oxo-2,3,4,5-tetrahydrobenzo[b]oxepine-8-carboxylate.

LC/MS (m/z, MH+): 365

### Step 4: Methyl 4-(2,4-dichlorophenyl)-5-(((trifluoromethyl)sulfonyl)oxy)-2,3-dihydrobenzo[b]oxepine-8-carboxylate

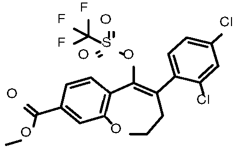

To a suspension of NaH (361 mg, 9.04 mmol, 60% purity in wheight) in Me-THF (17 ml) cooled at -2°C was added DBU (183 mg, 0.18 ml, 1.21 mmol) followed by a solution of methyl 4-(2,4-dichlorophenyl)-5-oxo-2,3,4,5-tetrahydrobenzo[b]oxepine-8-carboxylate (2.2 g, 6.02 mmol) and N,N-bis(trifluoromethylsulfonyl)aniline (2.80 g, 7.83 mmol) in THF (15 ml). The cooling bath was removed to allow the temperature to warm up to RT. The reaction mixture was stirred at RT for 4 hours. A mixture of acetic acid (0.14 ml) and water (20 ml) was dropwise added, followed by water (60 ml) and EtOAc (60 ml). After decantation, the organic phase was dried over MgSO₄, filtered and concentrated under reduced pressure. The residue obtained was purified by flash chromatography, eluting with DCM/heptane 70/30 to give 1.31 g (44%) of methyl 4-(2,4-dichlorophenyl)-5-(((trifluoromethyl)sulfonyl)oxy)-2,3-dihydrobenzo[b]oxepine-8-carboxylate.

LC/MS (m/z, MH+): 496

### Step 5: Methyl (S)-4-(2,4-dichlorophenyl)-5-(4-(pyrrolidin-3-yloxy)phenyl)-2,3-dihydrobenzo[b]oxepine-8-carboxylate

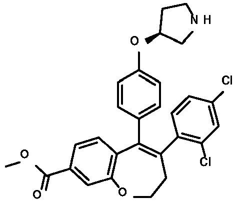

Step 5 of Intermediate 17 was prepared following a similar procedure to that of Step 1 of Intermediate 12 from methyl 4-(2,4-dichlorophenyl)-5-(((trifluoromethyl)sulfonyl)oxy)-2,3-dihydrobenzo[b]oxepine-8-carboxylate and (3S)-3-[4-(4,4,5,5-tetramethyl-1,3,2-dioxaborolan-2-yl)phenoxy]pyrrolidine (prepared according to WO2017140669) to give 530 mg (67%) of methyl (S)-4-(2,4-dichlorophenyl)-5-(4-(pyrrolidin-3-yloxy)phenyl)-2,3-dihydrobenzo[b]oxepine-8-carboxylate.

LC/MS (m/z, MH+): 510

### Step 6: Methyl (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylate

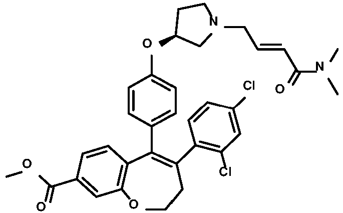

Step 6 of Intermediate 17 was prepared following a similar procedure to that of Step 3 of Intermediate 4 from methyl (S)-4-(2,4-dichlorophenyl)-5-(4-(pyrrolidin-3-yloxy)phenyl)-2,3-dihydrobenzo[b]oxepine-8-carboxylate and (E)-4-bromo-N,N-dimethyl-but-2-enamide (Intermediate 2) to give 423 mg (66%) of (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylate.

LC/MS (m/z, MH+): 621

### Intermediate 18: Methyl (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylate

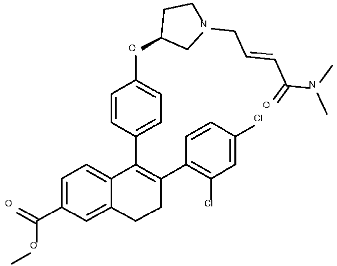

### Step 1: Methyl 2-(2,4-dichlorophenyl)-1-oxo-tetralin-6-carboxylate

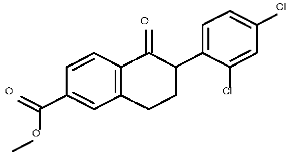

Step 1 of Intermediate 18 was prepared following a similar procedure to that of step 3 of Intermediate 17 from methyl 1-oxotetralin-6-carboxylate to give 459 mg (19%) of methyl 4-(2,4-dichlorophenyl)-5-oxo-2,3,4,5-tetrahydrobenzo[b]oxepine-8-carboxylate.

LC/MS (m/z, MH+): 349

### Step 2: Methyl 6-(2,4-dichlorophenyl)-5-(trifluoromethylsulfonyloxy)-7,8-dihydronaphthalene-2-carboxylate

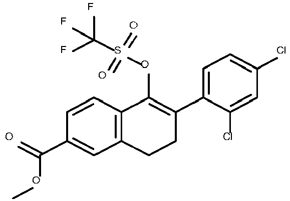

Step 2 of Intermediate 18 was prepared following a similar procedure to that of step 4 of Intermediate 17 from methyl 2-(2,4-dichlorophenyl)-1-oxo-tetralin-6-carboxylate to give 0.48 g (31%) of methyl 6-(2,4-dichlorophenyl)-5-(trifluoromethylsulfonyloxy)-7,8-dihydronaphthalene-2-carboxylate.

LC/MS (m/z, MH+): 481

### Step 3: Methyl (S)-6-(2,4-dichlorophenyl)-5-(4-(pyrrolidin-3-yloxy)phenyl)-7,8-dihydronaphthalene-2-carboxylate

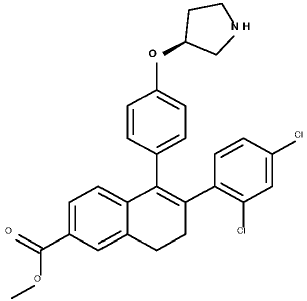

Step 3 of Intermediate 18 was prepared following a similar procedure to that of Step 1 of Intermediate 12 from methyl 6-(2,4-dichlorophenyl)-5-(trifluoromethylsulfonyloxy)-7,8-dihydronaphthalene-2-carboxylate and (3S)-3-[4-(4,4,5,5-tetramethyl-1,3,2-dioxaborolan-2-yl)phenoxy]pyrrolidine (prepared according to WO2017140669) to give 290 mg (45%) of methyl (S)-6-(2,4-dichlorophenyl)-5-(4-(pyrrolidin-3-yloxy)phenyl)-7,8-dihydronaphthalene-2-carboxylate.

LC/MS (m/z, MH+): 494

### Step 4: Methyl (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylate

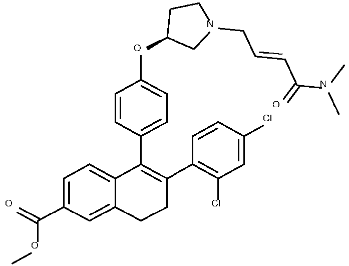

Step 4 of Intermediate 18 was prepared following a similar procedure to that of Step 3 of Intermediate 4 from methyl (S)-6-(2,4-dichlorophenyl)-5-(4-(pyrrolidin-3-yloxy)phenyl)-7,8-dihydronaphthalene-2-carboxylate and (E)-4-bromo-N,N-dimethyl-but-2-enamide (Intermediate 2) to give 127 mg (36%) of methyl (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylate.

LC/MS (m/z, MH+): 605

### Method A

### Example 1: (S,E)-8-(2,4-Dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid

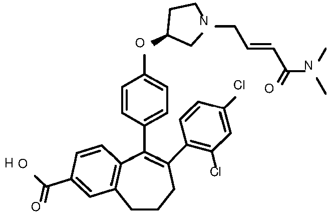

### Step 1: Methyl (R)-8-(2,4-dichlorophenyl)-9-(3-((1-(3-fluoropropyl)pyrrolidin-3-yl)oxy)phenyl)-6,7-dihydro-5H-benzo[7]annulene-3-carboxylate

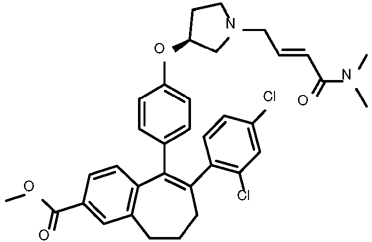

A mixture of methyl (S,E)-8-bromo-9-(4-((1-(4-(dimethylamino)-4-oxobut-2-en-1-yl)pyrrolidin-3-yl)oxy)phenyl)-6,7-dihydro-5H-benzo[7]annulene-3-carboxylate (Intermediate 1) (250 mg, 0.45 mmol), 2,4-dichlorophenylboronic acid (103 mg, 0.54 mmol), Cs₂CO₃ (309 mg, 0.95 mmol), Pd(dppf)Cl₂ complex with DCM (33 mg, 45.17 umol), in dioxane (3.36 ml) and water (0.84 ml) was heated to 100°C under microwaved irradiation for 30 minutes. After cooling to RT, DCM (10 ml) and water (2 ml) were added. After decantation, the organic phase was dried over Na₂SO₄ filtered and concentrated under reduced pressure and the residue obtained was purified by flash chromatography, eluting with a gradient of DCM/acetone from 75/25 to 50/50 to give 119 mg (43%) methyl (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylate.

LC/MS (m/z, MH+): 619

### Step 2: (S,E)-8-(2,4-Dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid

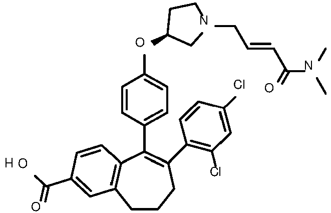

To a mixture of methyl (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylate (115 mg, 0.186 mmol) in dioxane (2 ml) and water (2 ml) was added LiOH (12 mg, 0.50 mmol) and the reaction mixture was stirred at RT for 18 hours. DCM (5 ml) and HCl 1N were added till pH 7. After decantation, the organic phase was dried over Na₂SO₄ filtered and concentrated under reduced pressure to give 73 mg (65%) of (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid.

### Method B

### Example 16: (S,E)-8-(2,4-Dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid

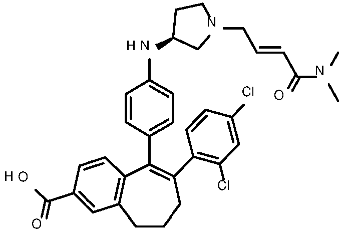

To a mixture of methyl (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylate (Intermediate 8) (35 mg, 0.057 mmol) in dioxane (0.3 ml) was added LiOH 1N (0.15 ml, 0.15 mmol) and the reaction mixture was stirred at RT for 5 hours. DCM (5 ml) and a saturated solution of NH4Cl (5 ml) were added. After decantation, the organic phase was dried over Na₂SO₄ filtered and concentrated under reduced pressure to give 21 mg (61%) of (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid.

### Method C

### Example 23: (S,E)-4-(3-(4-(8-(2,4-Dichlorophenyl)-3-hydroxy-6,7-dihydro-5H-benzo[7]annulen-9-yl)phenoxy)pyrrolidin-1-yl)-N,N-dimethylbut-2-enamide

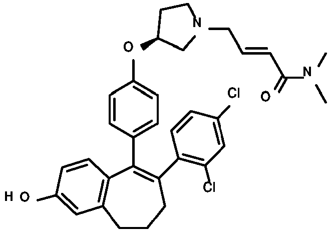

### Step 1: (S)-9-(4-(Pyrrolidin-3-yloxy)phenyl)-6,7-dihydro-5H-benzo[7]annulen-3-yl pivalate

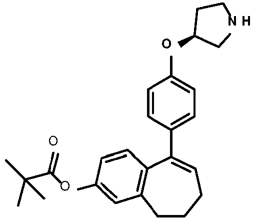

Step 1 of Example 23 was prepared following a similar procedure to that of Step 1 of Intermediate 12 from 9-(((trifluoromethyl)sulfonyl)oxy)-6,7-dihydro-5H-benzo[7]annulen-3-yl pivalate (prepared according to WO2017140669) and (3S)-3-[4-(4,4,5,5-tetramethyl-1,3,2-dioxaborolan-2-yl)phenoxy]pyrrolidine (prepared according to WO2017140669) to give 640 mg (65%) of (S)-9-(4-(pyrrolidin-3-yloxy)phenyl)-6,7-dihydro-5H-benzo[7]annulen-3-yl pivalate.

LC/MS (m/z, MH+): 406

### Step 2: (S)-8-Bromo-9-(4-(pyrrolidin-3-yloxy)phenyl)-6,7-dihydro-5H-benzo[7]annulen-3-yl pivalate

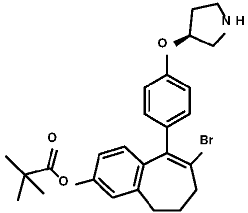

To a solution of (S)-9-(4-(pyrrolidin-3-yloxy)phenyl)-6,7-dihydro-5H-benzo[7]annulen-3-yl pivalate (640 mg, 1.58 mmol) in DCM (20 ml) was added pyridinium tribromide (656 mg, 2.05 mmol). The reaction mixture was stirred at RT for 2 hours. A concentrated aqueous solution of NaHCO₃ was added. After decantation, the organic phase was dried over Na₂SO₄, filtered, concentrated under reduced pressure and the residue obtained was purified by flash chromatography, eluting with a gradient of DCM/MeOH from 95/05 to 90/10 to give 550 mg (72%) of (S)-8-bromo-9-(4-(pyrrolidin-3-yloxy)phenyl)-6,7-dihydro-5H-benzo[7]annulen-3-yl pivalate.

LC/MS (m/z, MH+): 484

### Step 3: (S)-8-(2,4-Dichlorophenyl)-9-(4-(pyrrolidin-3-yloxy)phenyl)-6,7-dihydro-5H-benzo[7]annulen-3-yl pivalate

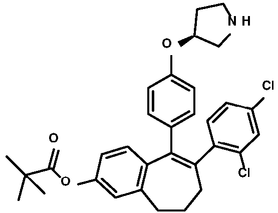

Step 3 of Example 23 was prepared following a similar procedure to that of Step 1 of Example 1 from (S)-8-bromo-9-(4-(pyrrolidin-3-yloxy)phenyl)-6,7-dihydro-5H-benzo[7]annulen-3-yl pivalate and 2,4-dichlorophenylboronic acid to give 200 mg (32%) of (S)-8-(2,4-dichlorophenyl)-9-(4-(pyrrolidin-3-yloxy)phenyl)-6,7-dihydro-5H-benzo[7]annulen-3-yl pivalate.

LC/MS (m/z, MH+): 550

### Step 4: (S,E)-8-(2,4-Dichlorophenyl)-9-(4-((1-(4-(dimethylamino)-4-oxobut-2-en-1-yl)pyrrolidin-3-yl)oxy)phenyl)-6,7-dihydro-5H-benzo[7]annulen-3-yl pivalate

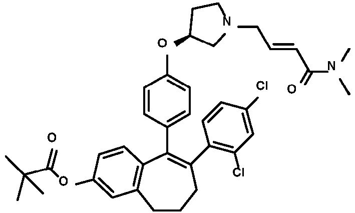

Step 4 of Example 23 was prepared following a similar procedure to that of Step 3 of Intermediate 4 from (S)-3-(4-(8-(2,4-dichlorophenyl)-6,7-dihydro-5H-benzo[7]annulen-9-yl)phenoxy)pyrrolidine and (E)-4-bromo-N,N-dimethyl-but-2-enamide (Intermediate 2) to give 110 mg (40%) of (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulen-3-yl pivalate.

LC/MS (m/z, MH+): 661

### Step 5: (S,E)-4-(3-(4-(8-(2,4-Dichlorophenyl)-3-hydroxy-6,7-dihydro-5H-benzo[7]annulen-9-yl)phenoxy)pyrrolidin-1-yl)-N,N-dimethylbut-2-enamide

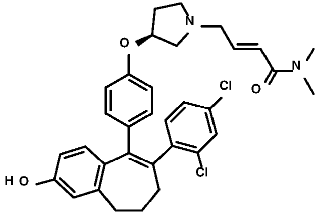

Step 5 of Example 23 was prepared following a similar procedure to that of Example 16 from (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulen-3-yl pivalate to give 15 mg (16%) of (S,E)-4-(3-(4-(8-(2,4-dichlorophenyl)-3-hydroxy-6,7-dihydro-5H-benzo[7]annulen-9-yl)phenoxy)pyrrolidin-1-yl)-N,N-dimethylbut-2-enamide.

### Example 26: (S,E)-4-(3-(4-(8-(2,4-Dichlorophenyl)-6,7-dihydro-5H-benzo[7]annulen-9-yl)phenoxy)pyrrolidin-1-yl)-N,N-dimethylbut-2-enamide

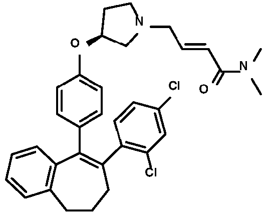

### Step 1: 6,7-Dihydro-5H-benzo[7]annulen-9-yl trifluoromethanesulfonate

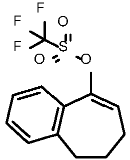

To a mixture of 6,7,8,9-tetrahydro-5H-benzo[7]annulen-5-one (1.3 g, 8.1 mmol) in DCM (30 ml) cooled at 0°C were added pyridine (0.98 ml, 12 mmol) and trifluoromethanesulfonic anhydride (4.6 g, 16 mmol). The reaction mixture was stirred at 0°C for 15 minutes and RT for 2 hours. DCM (50 ml) and cold water (30 ml) were added. After decantation, the organic phase was dried over MgSO₄, filtered, concentrated under reduced pressure to give 2.4 g (99%) of 6,7-dihydro-5H-benzo[7]annulen-9-yl trifluoromethanesulfonate.

LC/MS (m/z, MH+): 293

### Step 2: (S)-3-(4-(6,7-Dihydro-5H-benzo[7]annulen-9-yl)phenoxy)pyrrolidine

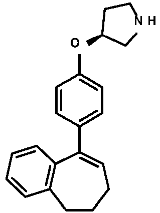

Step 2 of Example 26 was prepared following a similar procedure to that of Step 1 of Intermediate 12 from 6,7-dihydro-5H-benzo[7]annulen-9-yl trifluoromethanesulfonate and (3S)-3-[4-(4,4,5,5-tetramethyl-1,3,2-dioxaborolan-2-yl)phenoxy]pyrrolidine (prepared according to WO2017140669) to give 1.2 g (49%) of (S)-3-(4-(6,7-dihydro-5H-benzo[7]annulen-9-yl)phenoxy)pyrrolidine.

LC/MS (m/z, MH+): 306

### Step 3: (S)-3-(4-(8-Bromo-6,7-dihydro-5H-benzo[7]annulen-9-yl)phenoxy)pyrrolidine

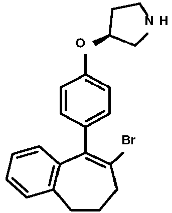

Step 3 of Example 26 was prepared following a similar procedure to that of Step 2 of Example 23 from (S)-3-(4-(6,7-dihydro-5H-benzo[7]annulen-9-yl)phenoxy)pyrrolidine and pyridinium tribromide to give 1.48 g (98%) of (S)-3-(4-(8-bromo-6,7-dihydro-5H-benzo[7]annulen-9-yl)phenoxy)pyrrolidine.

LC/MS (m/z, MH+): 384

### Step 4: (S)-3-(4-(8-(2,4-Dichlorophenyl)-6,7-dihydro-5H-benzo[7]annulen-9-yl)phenoxy)pyrrolidine

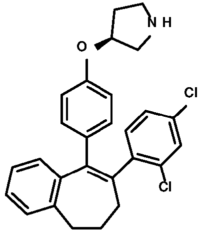

Step 4 of Example 26 was prepared following a similar procedure to that of Step 1 of Example 1 from (S)-3-(4-(8-bromo-6,7-dihydro-5H-benzo[7]annulen-9-yl)phenoxy)pyrrolidine and 2,4-dichlorophenylboronic acid to give 1.2 g (69%) of (S)-3-(4-(8-(2,4-dichlorophenyl)-6,7-dihydro-5H-benzo[7]annulen-9-yl)phenoxy)pyrrolidine.

LC/MS (m/z, MH+): 450

### Step 5: (S,E)-4-(3-(4-(8-(2,4-Dichlorophenyl)-6,7-dihydro-5H-benzo[7]annulen-9-yl)phenoxy)pyrrolidin-1-yl)-N,N-dimethylbut-2-enamide

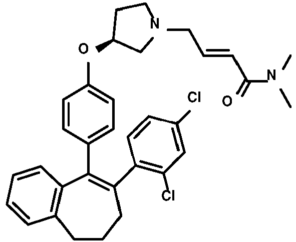

Step 5 of Example 26 was prepared following a similar procedure to that of Step 3 of Intermediate 4 from (S)-3-(4-(8-(2,4-dichlorophenyl)-6,7-dihydro-5H-benzo[7]annulen-9-yl)phenoxy)pyrrolidine and (E)-4-bromo-N,N-dimethyl-but-2-enamide (Intermediate 2) to give 370 mg (46%) of (S,E)-4-(3-(4-(8-(2,4-dichlorophenyl)-6,7-dihydro-5H-benzo[7]annulen-9-yl)phenoxy)pyrrolidin-1-yl)-N,N-dimethylbut-2-enamide.

### Example 27: (S,E)-4-(3-(4-(4-(2,4-Dichlorophenyl)-8-hydroxy-2,3-dihydrobenzo[b]thiepin-5-yl)phenoxy)pyrrolidin-1-yl)-N,N-dimethylbut-2-enamide

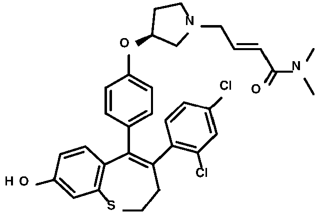

### Step 1: (S)-5-(4-(Pyrrolidin-3-yloxy)phenyl)-2,3-dihydrobenzo[b]thiepin-8-yl pivalate

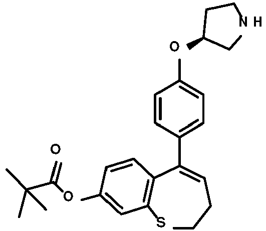

Step 1 of Example 27 was prepared following a similar procedure to that of Step 1 of Intermediate 12 from 5-(((trifluoromethyl)sulfonyl)oxy)-2,3-dihydrobenzo[b]thiepin-8-yl pivalate (prepared according to WO2018091153) and (3S)-3-[4-(4,4,5,5-tetramethyl-1,3,2-dioxaborolan-2-yl)phenoxy]pyrrolidine (prepared according to WO2017140669) to give 210 mg (62%) of (S)-5-(4-(pyrrolidin-3-yloxy)phenyl)-2,3-dihydrobenzo[b]thiepin-8-yl pivalate.

LC/MS (m/z, MH+): 424

### Step 2: (S)-4-Bromo-5-(4-(pyrrolidin-3-yloxy)phenyl)-2,3-dihydrobenzo[b]thiepin-8-yl pivalate

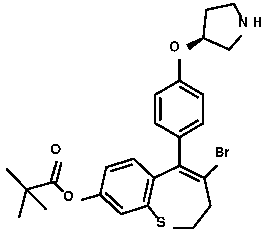

Step 2 of Example 27 was prepared following a similar procedure to that of Step 3 of Example 23 from (S)-5-(4-(pyrrolidin-3-yloxy)phenyl)-2,3-dihydrobenzo[b]thiepin-8-yl pivalate and pyridinium tribromide to give 300 mg (crude) of (S)-4-bromo-5-(4-(pyrrolidin-3-yloxy)phenyl)-2,3-dihydrobenzo[b]thiepin-8-yl pivalate.

LC/MS (m/z, MH+): 502

### Step 3: (S)-4-(2,4-Dichlorophenyl)-5-(4-(pyrrolidin-3-yloxy)phenyl)-2,3-dihydrobenzo[b]thiepin-8-yl pivalate

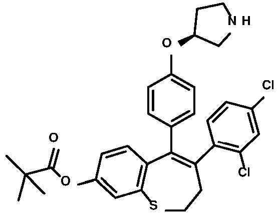

Step 3 of Example 27 was prepared following a similar procedure to that of Step 1 of Example 1 from (S)-4-bromo-5-(4-(pyrrolidin-3-yloxy)phenyl)-2,3-dihydrobenzo[b]thiepin-8-yl pivalate and 2,4-dichlorophenylboronic acid to give 83 mg (29%) of (S)-4-(2,4-dichlorophenyl)-5-(4-(pyrrolidin-3-yloxy)phenyl)-2,3-dihydrobenzo[b]thiepin-8-yl pivalate.

LC/MS (m/z, MH+): 568

### Step 4: (S,E)-4-(2,4-Dichlorophenyl)-5-(4-((1-(4-(dimethylamino)-4-oxobut-2-en-1-yl)pyrrolidin-3-yl)oxy)phenyl)-2,3-dihydrobenzo[b]thiepin-8-yl pivalate

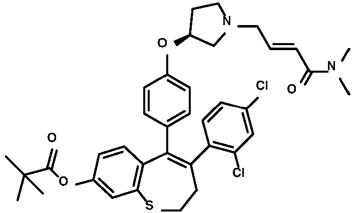

Step 4 of Example 27 was prepared following a similar procedure to that of Step 3 of Intermediate 4 (S)-4-(2,4-dichlorophenyl)-5-(4-(pyrrolidin-3-yloxy)phenyl)-2,3-dihydrobenzo[b]thiepin-8-yl pivalate and (E)-4-bromo-N,N-dimethyl-but-2-enamide (Intermediate 2) to give 61 mg (61%) of (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]thiepin-8-yl pivalate.

LC/MS (m/z, MH+): 679

### Step 5: (S,E)-4-(3-(4-(4-(2,4-Dichlorophenyl)-8-hydroxy-2,3-dihydrobenzo[b]thiepin-5-yl)phenoxy)pyrrolidin-1-yl)-N,N-dimethylbut-2-enamide

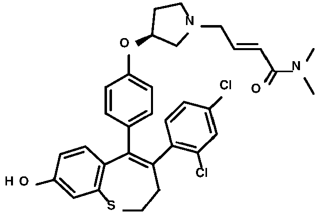

Step 5 of Example 27 was prepared following a similar procedure to that of Example 16 from (S,E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]thiepin-8-yl pivalate (Intermediate 2) to give 25 mg (47%) of (S,E)-4-(3-(4-(8-(2,4-dichlorophenyl)-3-hydroxy-6,7-dihydro-5H-benzo[7]annulen-9-yl)phenoxy)pyrrolidin-1-yl)-N,N-dimethylbut-2-enamide.

### Examples:

The compounds according to Table 1a above were subjected to pharmacological tests as presented below.

### Test: In vitro anti-proliferation assay

Said test involves measuring the *in vitro* anti-proliferation activity of the compounds of the Table 1a.

The measurements of the anti-proliferation activities were made using MCF7-WT (ATCC), MCF7-Y537S and HCC1428-LTED cells as described hereunder.

On Day 1, MCF7-WT and MCF7-Y537S (Mol Cancer Ther. 2021 20(2):250-262), and HCC1428-LTED (J Clin Invest. 2010;120(7):2406-2413) cells, were seeded at 2000 (WT and LTED) and 500 (Y537S) cells/well in phenol red free IMEM (Corning, 110-026-CV) with 5% Charcoal stripped-FBS and incubated overnight 37°C and 5% CO₂.

On day 2, compounds were tested in triplicate in 10-point dose response (1:3 serial dilutions with a top concentration starting 300nM); DMSO for upper limit (negative control) and staurosporine (1uM) for lower limit (positive control) were also included. MCF7-WT cells were then supplemented with final 0.1 nM b-estradiol. After treating the cells, the assay plates were transferred back in the incubator at 37°C and 5% CO₂.

On day 8, cell viability was measured per vendor recommendations (Cell Titer-Glo, Promega). Relative IC50 measurements were calculated as follow using a 4-parameter logistic derived from Levenburg Marquardt algorithm (IDBS XL Model 205 fit within Abase).

The Table 2 below indicates the anti-proliferation activity results for the compounds of Table 1a.

**Table 2:**

| | **Viability Screen Results** | | |
|---|---|---|---|
| **Compound No.** | **MCF7 WT IC50 (nM)** | **MCF7 Y537S IC50 (nM)** | **HCC 1428 IC50 (nM)** |
| 1 | 14 | 500 | 0.7 |
| 2 | 10 | 500 | 0.4 |
| 3 | 24 | 167 | 0.7 |
| 4 | 22 | 500 | 2 |
| 5 | 3 | 76 | 0.2 |
| 6 | 14 | 500 | 0.7 |
| 7 | 28 | 500 | 2 |
| 8 | 3 | 87 | 0.2 |
| 9 | 4 | 87 | 0.2 |
| 10 | 8 | 500 | 0.3 |
| 11 | 2 | 204 | 0.1 |
| 12 | 126 | 500 | 3 |
| 13 | 3 | 9 | 0.01 |
| 14 | 101 | 0.4 | 0.4 |
| 15 | 53 | 186 | 5 |
| 16 | 37 | 174 | 33 |
| 17 | 10 | 138 | 1 |
| 18 | 26 | 59 | 0.3 |
| 19 | 9 | 20.7 | 0.2 |
| 20 | 41 | 500 | 1 |
| 21 | 28 | 500 | 2.1 |
| 22 | 11 | 184 | 0.6 |
| 23 | 26 | 23 | 0.03 |
| 24 | 160 | 500 | 4.4 |
| 25 | 300 | 500 | 17 |
| 26 | 21 | 193 | 0.4 |
| 27 | 2.9 | 33 | 0.02 |

It is therefore apparent that the tested compounds have anti-proliferation activities, in both wild-type and mutated cell lines as tested above. As shown in Table 2, the compounds described herein display IC50 activities lower than 1 µM, typically 500 nM or lower activities.

The compounds of formula (I) can therefore be used for preparing medicaments, especially antiproliferative medicaments, as well as medicaments useful in the treatment of diseases wherein the estrogen receptor is involved.

Accordingly, also provided herein are medicaments which comprise a compound of the formula (I), or a pharmaceutically acceptable salt thereof.

Herein are also provided the compounds of formula (I) defined above, or pharmaceutically acceptable salts thereof, for use as medicines.

Herein are also provided the compounds of formula (I) defined above, or pharmaceutically acceptable salt thereof, for use in therapy, especially in diseases wherein the estrogen receptor is involved.

Herein are also provided the compounds of formula (I) defined above, or a pharmaceutically acceptable salts thereof, for use in the treatment of ovulatory dysfunction, cancer, endometriosis, osteoporosis, benign prostatic hypertrophy or inflammation.

A particular aspect is a compound of formula (I) defined above, or a pharmaceutically acceptable salt thereof, for use in the treatment of cancer.

In an embodiment, the cancer is a hormone dependent cancer.

In another embodiment, the cancer is an estrogen receptor dependent cancer, particularly the cancer is an estrogen receptor α dependent cancer.

In another embodiment, the cancer is selected from breast, ovarian, endometrial, prostate, uterine, cervical and lung cancer, or a metastasis thereof.

In another embodiment, the metastasis is a cerebral metastasis.

In another embodiment, the cancer is breast cancer. Particularly, the breast cancer is an estrogen receptor positive breast cancer (ERα positive breast cancer).

In another embodiment, the cancer is resistant to anti-hormonal treatment.

In a further embodiment, the compound of formula (I) is as used as single agent or in combination with other agents such as CDK4/6, mTOR or PI3K inhibitors.

Herein is also described the use of a compound of the formula (I), or a pharmaceutically acceptable salt thereof, for the manufacture of a medicament useful in treating any of the pathological conditions indicated above, more particularly useful in treating cancer.

Herein are also provided the pharmaceutical compositions comprising as active principle a compound of formula (I). These pharmaceutical compositions comprise an effective dose of at least one compound of formula (I), or a pharmaceutically acceptable salt thereof, and also at least one pharmaceutically acceptable excipient.

The said excipients are selected, in accordance with the pharmaceutical form and method of administration desired, from the customary excipients, which are known to a person skilled in the art.

In the pharmaceutical compositions for oral, sublingual, subcutaneous, intramuscular, intravenous, topical, local, intra-tracheal, intranasal, transdermal or rectal administration, the active principle of formula (I) above, or its base, acid, zwitterion or salt thereof, may be administered in a unit administration form, in a mixture with conventional pharmaceutical excipients, to animals and to human beings for the treatment of the above disorders or diseases.

The unit administration forms appropriate include oral forms such as tablets, soft or hard gel capsules, powders, granules and oral solutions or suspensions, sublingual, buccal, intra-tracheal, intra-ocular and intra-nasal administration forms, forms for inhalative, topical, transdermal, subcutaneous, intra-muscular or intravenous administration, rectal administration forms and implants. For topical application it is possible to use the compounds of formula (I) in creams, gels, ointments or lotions.

As an example, a unit administration form of a compound of formula (I) in tablet form may comprise the following components:

| | |
|---|---|
| Compound of formula (I) | 50.0 mg |
| Mannitol | 223.75 mg |
| Sodium croscarmellose | 6.0 mg |
| Corn starch | 15.0 mg |
| Hydroxypropylmethylcellulose | 2.25 mg |
| Magnesium stearate | 3.0 mg |

There may be particular cases in which higher or lower dosages are appropriate. According to usual practice, the dosage that is appropriate for each patient is determined by the doctor according to the mode of administration and the weight and response of the said patient.

## Claims

1. A compound of the formula (I) or a pharmaceutically acceptable salt thereof: 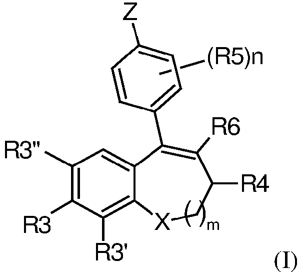 wherein:
- R3 represents a hydrogen atom, a -COOH group or a -OH group;
- R3' and R3" independently represent a hydrogen atom, a methyl group, a methoxy group, a chlorine atom, a fluorine atom or a cyano group;
- R4 represents a hydrogen atom, a (C₁-C₃)alkyl group or a cyclopropyl;
- R5 independently represents a (C₁-C₃)alkyl group such as a methyl group, a halogen atom, such as a fluorine atom, a cyano group, or a (C₁-C₃)fluoroalkyl group, such as a trifluoromethyl;
- R6 represents a group selected from:
▪ a phenyl group, said phenyl group being optionally substituted by 1 to 3 substituents independently selected from a halogen atom; a (C₁-C₆)alkyl group, optionally substituted with a cyano group or a -OH group; a (C₁-C₆)fluoroalkyl group; a (C₃-C₆)cycloalkyl group; a (C₁-C₆)alkoxy group; a (C₁-C₆)fluoroalkoxy group; a cyano group; a trifluoromethylsulfonyl group; a (C₁-C₄)alkylthio group; a (C₁-C₄)fluoroalkylthio group; a (C₁-C₄)alkylsulfonyl group and a -OH group;
▪ a fused phenyl group, selected from phenyl groups fused with a (C₃-C₆)cycloalkyl, which (C₃-C₆)cycloalkyl ring optionally comprises an unsaturation and, wherein the fused phenyl group is optionally substituted with 1 to 3 substituents independently selected from a (C₁-C₃)alkyl group, a hydroxy group, a halogen atom, a (C₁-C₆)fluoroalkyl group and a (C₁-C₃)alkoxy group;
▪ a bicyclic group comprising 5 to 12 carbon atoms, optionally comprising 1 to 2 unsaturations; optionally substituted with 1 to 4 substituents independently selected from: a fluorine atom, a -OH group, a (C₁-C₃)alkyl group, a (C₁-C₃)fluoroalkyl group, a (C₁-C₃)alkoxy group, a (C₁-C₃)fluoroalkoxy group and an oxo group;
▪ a heteroaryl group comprising 2 to 9 carbon atoms and comprising from 1 to 3 heteroatoms independently selected from oxygen, nitrogen and sulfur, and at least 5 atoms including carbon atoms and heteroatoms, such as a pyridyl group or a pyrrolyl group, said heteroaryl group being optionally substituted with 1 to 3 substituents independently selected from a halogen atom, a (C₁-C₆)alkyl group, a (C₁-C₆)fluoroalkyl group, a (C₁-C₆)alkoxy group, a (C₁-C₆)fluoroalkoxy group, a cyano group, a carbamoyl group and a -OH group;
▪ a cycloalkyl group comprising 3 to 7 carbon atoms, said cycloalkyl group being saturated or partially saturated and being optionally substituted with 1 to 4 substituents independently selected from:
∘ a fluorine atom, a -OH group, a (C₁-C₃)alkyl group, a (C₁-C₃)fluoroalkyl group, a (C₁-C₃)alkoxy group, a (C₁₋C₃)fluoroalkoxy group, an oxo group,
∘ a (C₃-C₆)cycloalkyl group, and a phenyl group, said (C₃-C₆)cycloalkyl or phenyl groups being optionally substituted with one or two halogen atom(s) or (C₁-C₃)alkyl group(s);
▪ a (C₃-C₆)cycloalkyl(C₁-C₃)alkyl group, optionally substituted on the cycloalkyl with 1 to 4 substituents independently selected from: a fluorine atom, a -OH group, a (C₁-C₄)alkyl group, a (C₁-C₃)fluoroalkyl group, a (C₁₋C₃)fluoroalkoxy group and an oxo group;
▪ a 4 to 7 membered-heterocycloalkyl group comprising 1 or 2 heteroatoms independently selected from oxygen, nitrogen and sulfur, such as a tetrahydropyranyl or a tetrahydrofuranyl group, said heterocycloalkyl group being saturated or partially saturated and being optionally substituted with 1 to 3 substituents independently selected from: a fluorine atom, a (C₁-C₃)alkyl group, a (C₁-C₃)fluoroalkyl group, a (C₁-C₃)fluoroalkoxy group, an oxo group, a (C₁-C₃)alkoxy group, and a -OH group;
▪ a (C₁-C₆)alkyl group, such as an isobutyl group or an ethylbutyl group, said alkyl group being optionally substituted with 1 to 4 substituents independently selected from: a fluorine atom, a (C₁-C₃)alkoxy group, a (C₁₋C₃)fluoroalkoxy group and a -OH group; and
▪ a phenyl(C₁-C₂)alkyl group, said phenyl group being optionally substituted with 1 to 3 substituents independently selected from a halogen atom; a (C₁-C₃)alkyl group; a (C₁-C₃)fluoroalkyl group; a (C₁-C₃)alkoxy group; a (C₁₋C₃)fluoroalkoxy group; a cyano group; and a -OH group;
- X represents -CH₂-, -O- or -S-;
- n is 0, 1 or 2;
- m is 0 or 1; and
- Z represents a group selected from:
▪ 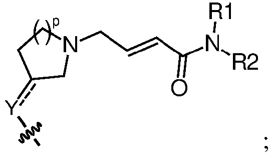 and
▪ 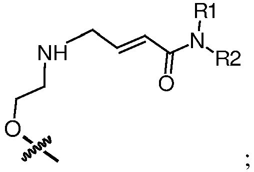 wherein:
∘ R1 and R2 independently represent a hydrogen atom, a -CH₃ group, a -CH₂CH₃ group, or a -CH₂CH₂OH group, or R1 and R2 forms a 4 to 6 membered-heterocycloalkyl group with the nitrogen atom to which R1 and R2, are attached, said heterocycloalkyl group optionally comprising an additional heteroatom selected from oxygen, nitrogen and sulfur;
∘ Y represents -CH₂-, -CH=, -CR7=, -O- or -NH-, wherein R7 represents a fluorine atom or a (C₁-C₃)alkyl group;
∘ 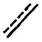 represents a single bond or a double bond; and
∘ p is 0 or 1.

2. The compound of formula (I) according to claim 1, or a pharmaceutically acceptable salt thereof, **characterized in that** it is of formula (I'):

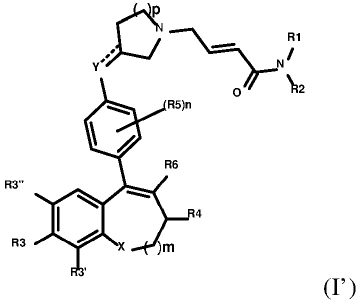

3. The compound of formula (I) or (I') according to claim 1 or 2, or a pharmaceutically acceptable salt thereof, **characterized in that** Y represents -CH₂-, -CH=, - O- or -NH-, and preferably Y represents -O-.

4. The compound of formula (I) or (I') according to any one of claim 1 to 3, or a pharmaceutically acceptable salt thereof, **characterized in that** 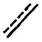 represents a single bond.

5. The compound of formula (I) or (I') according to any one of claim 1 to 4, or a pharmaceutically acceptable salt thereof, **characterized in that** p is 1.

6. The compound of formula (I) according to claim 1, or a pharmaceutically acceptable salt thereof, **characterized in that** it is of formula (I''):

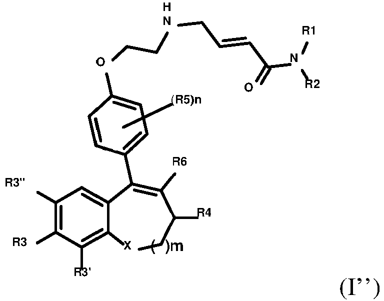

7. The compound of formula (I), (I') or (I") according to any one of claim 1 to 6, or a pharmaceutically acceptable salt thereof, **characterized in that** R1 and R2 are a -CH₃ group.

8. The compound of formula (I), (I') or (I") according to any one of claim 1 to 7, or a pharmaceutically acceptable salt thereof, **characterized in that** R3 is a -COOH group.

9. The compound of formula (I), (I') or (I") according to any one of claim 1 to 8, or a pharmaceutically acceptable salt thereof, **characterized in that** R3' and R3" represent a hydrogen atom.

10. The compound of formula (I), (I') or (I") according to any one of claim 1 to 9, or a pharmaceutically acceptable salt thereof, **characterized in that** R4 represents a hydrogen atom.

11. The compound of formula (I), (I') or (I") according to any one of claim 1 to 10, or a pharmaceutically acceptable salt thereof, **characterized in that** X represents -CH₂-.

12. The compound of formula (I), (I') or (I") according to any one of claim 1 to 11, or a pharmaceutically acceptable salt thereof, **characterized in that** n is 0.

13. The compound of formula (I), (I') or (I'') according to anyone of claims 1 to 12, or a pharmaceutically acceptable salt thereof, wherein m is 1.

14. The compound of formula (I), (I') or (I") according to any one of claim 1 to 13, or a pharmaceutically acceptable salt thereof, **characterized in that** R6 represents a phenyl group, said phenyl group being optionally substituted with 1 or 2 substituents independently selected from a chlorine atom, a fluorine atom and a methyl group.

15. The compound of formula (I), (I') or (I") according to any one of claim 1 to 13, or a pharmaceutically acceptable salt thereof, **characterized in that** R6 represents a pyridyl group, said pyridyl group being substituted by two substituents independently selected from a halogen atom and a (C₁-C₆)alkoxy group, and more particularly selected from a fluorine atom and a methoxy group.

16. The compound of formula (I), (I') or (I'') according to anyone of claims 1 to 15, or a pharmaceutically acceptable salt thereof, in particular hydrochloride salt thereof, **characterized in that** said compound is selected from the following compounds:
- (S,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7] annulene-3-carboxylic acid, (1)
- (S,E)-8-(2-chloro-4-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (2)
- (S,E)-8-(4-chloro-2-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (3)
- (S,E)-8-(2-chloro-3-fluorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (4)
- (S,E)-9-(4-((1-(4-(dimethylamino)-4-oxobut-2-en-1-yl)pyrrolidin-3-yl)oxy)phenyl)-8-(2-fluoro-4-methylphenyl)-6,7-dihydro-5H-benzo[7]annulene-3-carboxylic acid, (5)
- (S,E)-9-(4-((1-(4-(dimethylamino)-4-oxobut-2-en-1-yl)pyrrolidin-3-yl)oxy)phenyl)-8-phenyl-6,7-dihydro-5H-benzo[7]annulene-3-carboxylic acid, (6)
- (S,E)-8-(3-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (7)
- (S,E)-9-(4-((1-(4-(dimethylamino)-4-oxobut-2-en-1-yl)pyrrolidin-3-yl)oxy)phenyl)-8-(2,4-dimethylphenyl)-6,7-dihydro-5H-benzo[7]annulene-3-carboxylic acid, (8)
- (S,E)-8-(2-chlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (9)
- (S,E)-9-(4-((1-(4-(dimethylamino)-4-oxobut-2-en-1-yl)pyrrolidin-3-yl)oxy)phenyl)-8-(2-fluoro-6-methoxypyridin-3-yl)-6,7-dihydro-5H-benzo[7]annulene-3-carboxylic acid, (10)
- (S,E)-8-(2-chloro-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (11)
- (E)-8-(2,4-dichlorophenyl)-9-(4-((1-(4-(dimethylamino)-4-oxobut-2-en-1-yl)azetidin-3-yl)oxy)phenyl)-6,7-dihydro-5H-benzo[7]annulene-3-carboxylic acid, (12)
- (E)-8-(2,4-dichlorophenyl)-9-(4-((1-(4-(dimethylamino)-4-oxobut-2-en-1-yl)azetidin-3-yl)methyl)phenyl)-6,7-dihydro-5H-benzo[7]annulene-3-carboxylic acid compound with 2,2,2-trifluoroacetic acid, (13)
- (E)-8-(2,4-dichlorophenyl)-9-(4-((1-(4-(dimethylamino)-4-oxobut-2-en-1-yl)azetidin-3-ylidene)methyl)phenyl)-6,7-dihydro-5H-benzo[7]annulene-3-carboxylic acid, (14)
- (R,E)-8-(2,4-dichlorophenyl)-9-(4-((1-(4-(dimethylamino)-4-oxobut-2-en-1-yl)pyrrolidin-3-yl)oxy)phenyl)-6,7-dihydro-5H-benzo[7]annulene-3-carboxylic acid, (15)
- (S,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7]annulene-3-carboxylic acid, (16)
- (R,E)-8-(2,4-dichlorophenyl)-9-(4-((1-(4-(dimethylamino)-4-oxobut-2-en-1-yl)pyrrolidin-3-yl)amino)phenyl)-6,7-dihydro-5H-benzo[7] annulene-3-carboxylic acid, (17)
- (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 1, (18)
- (E)-8-(2,4-dichlorophenyl)-9-(4-((1-(4-(dimethylamino)-4-oxobut-2-en-1-yl)pyrrolidin-3-yl)methyl)phenyl)-6,7-dihydro-5H-benzo[7]annulene-3-carboxylic acid, Isomer 2, (19)
- 8-(2,4-dichlorophenyl)-9-(4-((Z)-(1-((E)-4-(dimethylamino)-4-oxobut-2-en-1-yl)pyrrolidin-3-ylidene)methyl)phenyl)-6,7-dihydro-5H-benzo[7]annulene-3-carboxylic acid, (20)
- (E)-8-(2,4-dichlorophenyl)-9-(4-((1-(4-(dimethylamino)-4-oxobut-2-en-1-yl)azetidin-3-yl)amino)phenyl)-6,7-dihydro-5H-benzo[7]annulene-3-carboxylic acid, (21)
- (E)-8-(2,4-dichlorophenyl)-9-(4-(2-((4-(dimethylamino)-4-oxobut-2-en-1-yl)amino)ethoxy)phenyl)-6,7-dihydro-5H-benzo[7]annulene-3-carboxylic acid, (22)
- (S,E)-4-(3-(4-(8-(2,4-dichlorophenyl)-3-hydroxy-6,7-dihydro-5H-benzo[7]annulen-9-yl)phenoxy)pyrrolidin-1-yl)-N,N-dimethylbut-2-enamide, (23)
- (S.E)-4-(2,4-dichlorophenyl)-5-(4-((1-(4-(dimethylamino)-4-oxobut-2-en-1-yl)pyrrolidin-3-yl)oxy)phenyl)-2,3-dihydrobenzo[b]oxepine-8-carboxylic acid, (24)
- (S,E)-6-(2,4-dichlorophenyl)-5-(4-((1-(4-(dimethylamino)-4-oxobut-2-en-1-yl)pyrrolidin-3-yl)oxy)phenyl)-7,8-dihydronaphthalene-2-carboxylic acid, (25)
- (S,E)-4-(3-(4-(8-(2,4-dichlorophenyl)-6,7-dihydro-5H-benzo[7]annulen-9-yl)phenoxy)pyrrolidin-1-yl)-N,N-dimethylbut-2-enamide, (26)
- (S,E)-4-(3-(4-(4-(2,4-dichlorophenyl)-8-hydroxy-2,3-dihydrobenzo[b]thiepin-5-yl)phenoxy)pyrrolidin-1-yl)-N,N-dimethylbut-2-enamide, (27).

17. A process for preparing a compound of formula (I) or (I') as defined in anyone of claims 1 to 5 and 7 to 16, or a pharmaceutically acceptable salt thereof, wherein a compound of formula 1F: 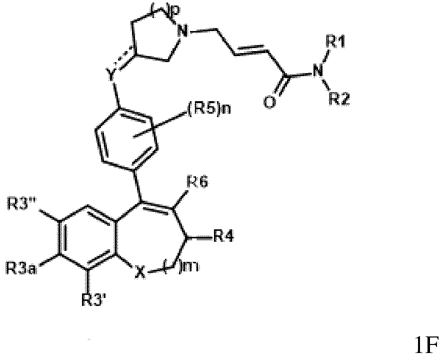 wherein R1, R2, R3', R3", R4, R5, R6, m, n, p, X, Y and 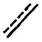 are as defined in any of claims 1, 3 to 5 and 7 to 15, and R3a is a hydrogen atom or a carboxylic ester such as -COOMe, - COOEt, or protected -OH such as O-pivaloyl for example, is converted to compound of formula (I) or (I'), in presence of a source of hydroxide ions, such as NaOH or LiOH in solution in THF or dioxane .

18. A process for preparing a compound of formula (I) or (I') as defined in anyone of claims 1 to 5 and 7 to 16, or a pharmaceutically acceptable salt thereof, wherein a compound of formula 1F': 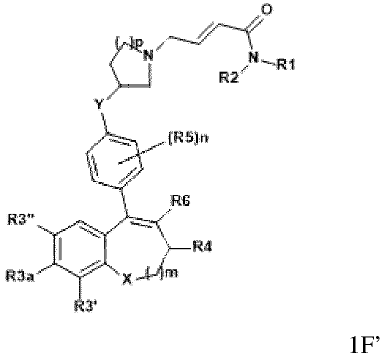 wherein R1, R2, R3', R3", R4, R5, R6, m, n, p, X and Y are as defined in any of claims 1, 3 to 5 and 7 to 15, and R3a is a hydrogen atom or a carboxylic ester such as -COOMe, -COOEt, or protected -OH such as O-pivaloyl for example, is converted to compound of formula (I) or (I'), in presence of a source of hydroxide ions, such as NaOH or LiOH in solution in THF or dioxane , said step being optionally preceded by a step for obtaining compound 1F', wherein a compound of formula 1T: 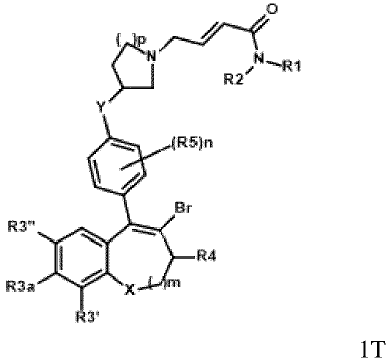
wherein R1, R2, R3', R3", R4, R5, m, n, p, X and Y are as defined in any of claims 1, 3 to 5 and 7 to 15 and R3a is as defined above,
is subjected to a Suzuki coupling with a boronic reagent R6B(OR')₂, wherein -B(OR')₂ is a boronic acid or a pinacolate ester and R6 is as defined in any of claims 1, 14 or 15.

19. Intermediate compounds selected from compounds of formula 1T, 1F, 1F' and 2B, or any of its pharmaceutically acceptable salt, 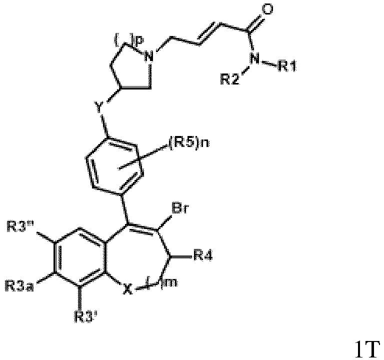 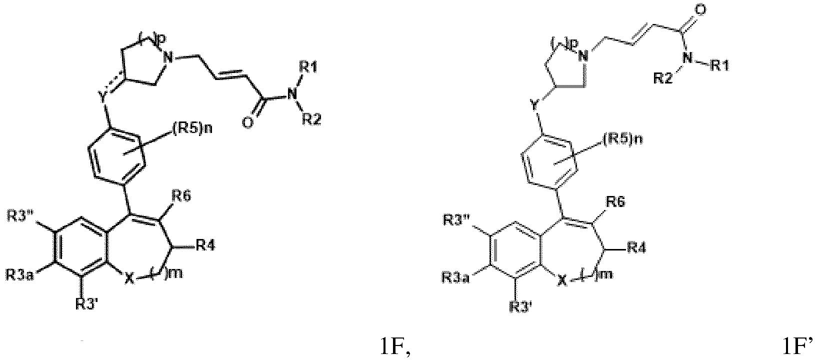 and 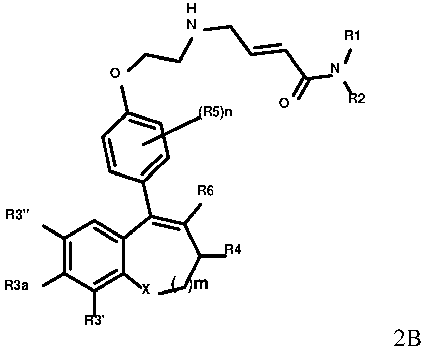 wherein R1, R2, R3', R3", R4, R5, R6, m, n, p, X, Y and 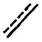 are as defined in any of claims 1, 3 to 5 and 7 to 15 and R3a is a hydrogen atom or a carboxylic ester such as -COOMe, - COOEt, or protected -OH such as O-pivaloyl.

20. Intermediate compound of formula 1E, or any of its pharmaceutically acceptable salt: 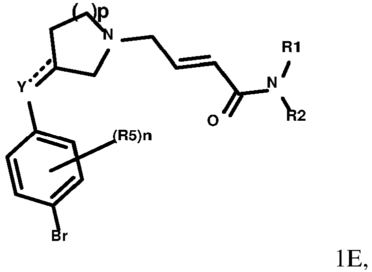 wherein R1, R2, R5, Y, n, p and 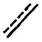 are as defined in any of claims 1, 3 to 5, 7 and 12.

21. A medicament, **characterized in that** it comprises a compound of formula (I) according to any of claims 1 to 16, or a pharmaceutically acceptable salt thereof.

22. A pharmaceutical composition, **characterized in that** it comprises a compound of formula (I) according to any of claims 1 to 16, or a pharmaceutically acceptable salt thereof, and at least one pharmaceutically acceptable excipient.

23. A compound of formula (I) according to any of claims 1 to 16, or a pharmaceutically acceptable salt thereof, for use in the treatment of ovulatory dysfunction, cancer, endometriosis, osteoporosis, benign prostatic hypertrophy or inflammation.

24. A compound of formula (I) for use according to claim 23, or a pharmaceutically acceptable salt thereof, for use in the treatment of cancer.

## Patentansprüche

1. Verbindung der Formel (I) oder ein pharmazeutisch akzeptables Salz davon: 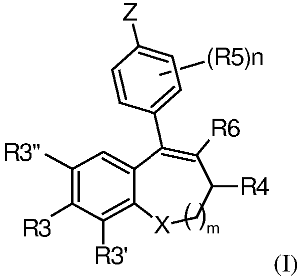 wobei:
- R3 für ein Wasserstoffatom, eine -COOH-Gruppe oder eine -OH-Gruppe steht;
- R3' und R3" unabhängig für ein Wasserstoffatom, eine Methylgruppe, eine Methoxygruppe, ein Chloratom, ein Fluoratom oder eine Cyanogruppe stehen;
- R4 für ein Wasserstoffatom, eine (C₁-C₃)-Alkylgruppe oder ein Cyclopropyl steht;
- R5 unabhängig für eine (C₁-C₃)-Alkylgruppe, wie eine Methylgruppe, ein Halogenatom, wie ein Fluoratom, eine Cyanogruppe oder eine (C₁-C₃)-Fluoralkylgruppe, wie ein Trifluormethyl, steht;
- R6 für eine Gruppe steht, die ausgewählt ist aus:
■ einer Phenylgruppe, wobei die Phenylgruppe gegebenenfalls durch 1 bis 3 Substituenten, die unabhängig aus einem Halogenatom; einer (C₁-C₆)-Alkylgruppe, die gegebenenfalls durch eine Cyanogruppe oder eine -OH-Gruppe substituiert ist; einer (C₁-C₆)-Fluoralkylgruppe; einer (C₃-C₆)-Cycloalkylgruppe; einer (C₁-C₆) -Alkoxygruppe; einer (C₁-C₆) -Fluoralkoxygruppe; einer Cyanogruppe; einer Trifluormethylsulfonylgruppe; einer (C₁-C₄)-Alkylthiogruppe; einer (C₁-C₄) - Fluoralkylthiogruppe; einer (C₁-C₄)-Alkylsulfonylgruppe und einer -OH-Gruppe ausgewählt sind, substituiert ist;
■ einer anellierten Phenylgruppe, ausgewählt aus Phenylgruppen, die mit einem (C₃-C₆)-Cycloalkyl anelliert sind, wobei der (C₃-C₆)-Cycloalkylring gegebenenfalls eine Ungesättigtheit enthält und wobei die anellierte Phenylgruppe gegebenenfalls durch 1 bis 3 Substituenten, die unabhängig aus einer (C₁-C₃)-Alkylgruppe, einer Hydroxylgruppe, einem Halogenatom, einer (C₁-C₆)-Fluoralkylgruppe und einer (C₁-C₃)-Alkoxygruppe ausgewählt sind, substituiert ist;
■ einer bicyclischen Gruppe mit 5 bis 12 Kohlenstoffatomen und gegebenenfalls 1 bis 2 Ungesättigtheiten; die gegebenenfalls durch 1 bis 4 Substituenten, die unabhängig aus einem Fluoratom, einer -OH-Gruppe, einer (C₁-C₃) -Alkylgruppe, einer (C₁-C₃)-Fluoralkylgruppe, einer (C₁-C₃)-Alkoxygruppe, einer (C₁-C₃)-Fluoralkoxygruppe und einer Oxogruppe ausgewählt sind, substituiert ist;
■ einer Heteroarylgruppe mit 2 bis 9 Kohlenstoffatomen und 1 bis 3 Heteroatomen, die unabhängig aus Sauerstoff, Stickstoff und Schwefel ausgewählt sind, und mindestens 5 Atomen einschließlich Kohlenstoffatomen und Heteroatomen, wie einer Pyridylgruppe oder einer Pyrrolylgruppe, wobei die Heteroarylgruppe gegebenenfalls durch 1 bis 3 Substituenten, die unabhängig aus einem Halogenatom, einer (C₁-C₆)-Alkylgruppe, einer (C₁-C₆) -Fluoralkylgruppe, einer (C₁-C₆)-Alkoxygruppe, einer (C₁-C₆)-Fluoralkoxygruppe, einer Cyanogruppe, einer Carbamoylgruppe und einer -OH-Gruppe ausgewählt sind, substituiert ist;
■ einer Cycloalkylgruppe mit 3 bis 7 Kohlenstoffatomen, wobei die Cycloalkylgruppe gesättigt oder teilweise gesättigt ist und gegebenenfalls durch 1 bis 4 Substituenten substituiert ist, die unabhängig ausgewählt sind aus:
∘ einem Fluoratom, einer -OH-Gruppe, einer (C₁-C₃)-Alkylgruppe, einer (C₁-C₃) -Fluoralkylgruppe, einer (C₁-C₃) -Alkoxygruppe, einer (C₁-C₃)-Fluoralkoxygruppe, einer Oxogruppe,
∘ einer (C₃-C₆)-Cycloalkylgruppe und einer Phenylgruppe, wobei die (C₃-C₆)-Cycloalkyl- oder Phenylgruppen gegebenenfalls durch ein oder zwei Halogenatome oder (C₁-C₃)-Alkylgruppen substituiert sind;
■ einer (C₃-C₆) -Cycloalkyl- (C₁-C₃) -alkylgruppe, die gegebenenfalls an dem Cycloalkyl durch 1 bis 4 Substituenten, die unabhängig aus einem Fluoratom, einer -OH-Gruppe, einer (C₁-C₄) -Alkylgruppe, einer (C₁-C₃)-Fluoralkylgruppe, einer (C₁-C₃)-Fluoralkoxygruppe und einer Oxogruppe ausgewählt sind, substituiert ist;
■ einer 4- bis 7-gliedrigen Heterocycloalkylgruppe mit 1 oder 2 Heteroatomen, die unabhängig aus Sauerstoff, Stickstoff und Schwefel ausgewählt sind, wie einer Tetrahydropyranyl- oder Tetrahydrofuranylgruppe, wobei die Heterocycloalkylgruppe gesättigt oder teilweise gesättigt ist und gegebenenfalls durch 1 bis 3 Substituenten, die unabhängig aus einem Fluoratom, einer (C₁-C₃) -Alkylgruppe, einer (C₁-C₃) -Fluoralkylgruppe, einer (C₁-C₃)-Fluoralkoxygruppe, einer Oxogruppe, einer (C₁-C₃)-Alkoxygruppe und einer -OH-Gruppe ausgewählt sind, substituiert ist;
■ einer (C₁-C₆)-Alkylgruppe, wie einer Isobutylgruppe oder einer Ethylbutylgruppe, wobei die Alkylgruppe gegebenenfalls durch 1 bis 4 Substituenten, die unabhängig aus einem Fluoratom, einer (C₁-C₃)-Alkoxygruppe, einer (C₁-C₃)-Fluoralkoxygruppe und einer - OH-Gruppe ausgewählt sind, substituiert ist; und
■ einer Phenyl- (C₁-C₂)-alkylgruppe, wobei die Phenylgruppe gegebenenfalls durch 1 bis 3 Substituenten, die unabhängig aus einem Halogenatom; einer (C₁-C₃)-Alkylgruppe; einer (C₁-C₃) -Fluoralkylgruppe; einer (C₁-C₃) -Alkoxygruppe; einer (C₁-C₃)-Fluoralkoxygruppe; einer Cyanogruppe und einer -OH-Gruppe ausgewählt sind, substituiert ist;
- X für -CH₂-, -O- oder -S- steht;
- n gleich 0, 1 oder 2 ist;
- m gleich 0 oder 1 ist; und
- Z für eine Gruppe steht, die ausgewählt ist aus:
▪ 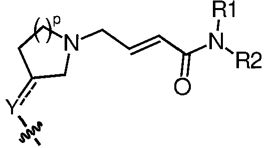 und
▪ 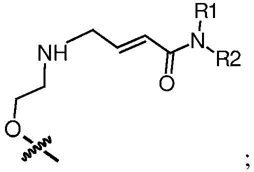 wobei:
∘ R1 und R2 unabhängig für ein Wasserstoffatom, eine - CH₃-Gruppe, eine -CH₂CH₃-Gruppe oder eine -CH₂CH₂OH-Gruppe stehen oder R1 und R2 mit dem Stickstoffatom, an das R1 und R2 gebunden sind, eine 4- bis 6-gliedrige Heterocycloalkylgruppe bilden, wobei die Heterocycloalkylgruppe gegebenenfalls ein zusätzliches Heteroatom, das aus Sauerstoff, Stickstoff und Schwefel ausgewählt ist, umfasst;
∘ Y für -CH₂-, -CH=, -CR7=, -O- oder -NH- steht, wobei R7 für ein Fluoratom oder eine (C₁-C₃)Alkylgruppe steht;
∘ 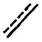 für eine Einfachbindung oder eine Doppelbindung steht; und
∘ p gleich 0 oder 1 ist.

2. Verbindung der Formel (I) nach Anspruch 1 oder ein pharmazeutisch akzeptables Salz davon, **dadurch gekennzeichnet, dass** es die Formel (I') aufweist:

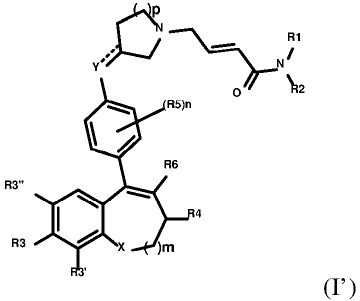

3. Verbindung der Formel (I) oder (I') nach Anspruch 1 oder 2 oder ein pharmazeutisch akzeptables Salz davon, **dadurch gekennzeichnet, dass** Y für -CH₂-, -CH=, -O- oder -NH- steht und vorzugsweise Y für -O- steht.

4. Verbindung der Formel (I) oder (I') nach einem der Ansprüche 1 bis 3 oder ein pharmazeutisch akzeptables Salz davon, **dadurch gekennzeichnet, dass** 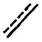 für eine Einfachbindung steht.

5. Verbindung der Formel (I) oder (I') nach einem der Ansprüche 1 bis 4 oder ein pharmazeutisch akzeptables Salz davon, **dadurch gekennzeichnet, dass** p gleich 1 ist.

6. Verbindung der Formel (I) nach Anspruch 1 oder ein pharmazeutisch akzeptables Salz davon, **dadurch gekennzeichnet, dass** es die Formel (I") aufweist:

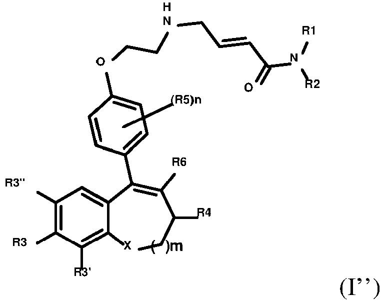

7. Verbindung der Formel (I), (I') oder (I") nach einem der Ansprüche 1 bis 6 oder ein pharmazeutisch akzeptables Salz davon, **dadurch gekennzeichnet, dass** R1 und R2 für eine -CH₃-Gruppe stehen.

8. Verbindung der Formel (I), (I') oder (I") nach einem der Ansprüche 1 bis 7 oder ein pharmazeutisch akzeptables Salz davon, **dadurch gekennzeichnet, dass** R3 für eine - COOH-Gruppe steht.

9. Verbindung der Formel (I), (I') oder (I") nach einem der Ansprüche 1 bis 8 oder ein pharmazeutisch akzeptables Salz davon, **dadurch gekennzeichnet, dass** R3' und R3" für ein Wasserstoffatom stehen.

10. Verbindung der Formel (I), (I') oder (I") nach einem der Ansprüche 1 bis 9 oder ein pharmazeutisch akzeptables Salz davon, **dadurch gekennzeichnet, dass** R4 für ein Wasserstoffatom steht.

11. Verbindung der Formel (I), (I') oder (I") nach einem der Ansprüche 1 bis 10 oder ein pharmazeutisch akzeptables Salz davon, **dadurch gekennzeichnet, dass** X für -CH₂- steht.

12. Verbindung der Formel (I), (I') oder (I") nach einem der Ansprüche 1 bis 11 oder ein pharmazeutisch akzeptables Salz davon, **dadurch gekennzeichnet, dass** n gleich 0 ist.

13. Verbindung der Formel (I), (I') oder (I") nach einem der Ansprüche 1 bis 12 oder ein pharmazeutisch akzeptables Salz davon, wobei m gleich 1 ist.

14. Verbindung der Formel (I), (I') oder (I") nach einem der Ansprüche 1 bis 13 oder ein pharmazeutisch akzeptables Salz davon, **dadurch gekennzeichnet, dass** R6 für eine Phenylgruppe steht, wobei die Phenylgruppe gegebenenfalls durch 1 oder 2 Substituenten, die unabhängig aus einem Chloratom, einem Fluoratom und einer Methylgruppe ausgewählt sind, substituiert ist.

15. Verbindung der Formel (I), (I') oder (I") nach einem der Ansprüche 1 bis 13 oder ein pharmazeutisch akzeptables Salz davon, **dadurch gekennzeichnet, dass** R6 für eine Pyridylgruppe steht, wobei die Pyridylgruppe durch zwei Substituenten, die unabhängig aus einem Halogenatom und einer (C₁-C₆)-Alkoxygruppe ausgewählt sind und spezieller aus einem Fluoratom und einer Methoxygruppe ausgewählt sind, substituiert ist.

16. Verbindung der Formel (I), (I') oder (I") nach einem der Ansprüche 1 bis 15 oder ein pharmazeutisch akzeptables Salz davon, insbesondere Hydrochloridsalz davon, **dadurch gekennzeichnet, dass** die Verbindung aus den folgenden Verbindungen ausgewählt ist:
- (S,E)-8-(2,4-Dichlorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (1)
- (S,E)-8-(2-Chlor-4-fluorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (2)
- (S,E)-8-(4-Chlor-2-fluorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (3)
- (S,E)-8-(2-Chlor-3-fluorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (4)
- (S,E)-9-(4-((1-(4-(Dimethylamino)-4-oxobut-2-en-1-yl)pyrrolidin-3-yl)oxy)phenyl)-8-(2-fluor-4-methylphenyl)-6,7-dihydro-5H-benzo[7]annulen-3-carbonsäure, (5)
- (S,E)-9-(4-((1-(4-(Dimethylamino)-4-oxobut-2-en-1-yl)pyrrolidin-3-yl)oxy)phenyl)-8-phenyl-6,7-dihydro-5H-benzo[7]annulen-3-carbonsäure, (6)
- (S,E)-8-(3-Chlor-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (7)
- (S,E)-9-(4-((1-(4-(Dimethylamino)-4-oxobut-2-en-1-yl)pyrrolidin-3-yl)oxy)phenyl)-8-(2,4-dimethylphenyl)-6,7-dihydro-5H-benzo[7]annulen-3-carbonsäure, (8)
- (S,E)-8-(2-Chlorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (9)
- (S,E)-9-(4-((1-(4-(Dimethylamino)-4-oxobut-2-en-1-yl)pyrrolidin-3-yl)oxy)phenyl)-8-(2-fluor-6-methoxypyridin-3-yl)-6,7-dihydro-5H-benzo[7]annulen-3-carbonsäure, (10)
- (S,E)-8-(2-Chlor-4-methylphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (11)
- (E)-8-(2,4-Dichlorphenyl)-9-(4-((1-(4-(dimethylamino)-4-oxobut-2-en-1-yl)azetidin-3-yl)oxy)phenyl)-6,7-dihydro-5H-benzo[7]annulen-3-carbonsäure, (12)
- (E)-8-(2,4-Dichlorphenyl)-9-(4-((1-(4-(dimethylamino)-4-oxobut-2-en-1-yl)azetidin-3-yl)methyl)phenyl)-6,7-dihydro-5H-benzo[7]annulen-3-carbonsäure, Verbindung mit 2,2,2-Trifluoressigsäure, (13)
- (E)-8-(2,4-Dichlorphenyl)-9-(4-((1-(4-(dimethylamino)-4-oxobut-2-en-1-yl)azetidin-3-yliden)methyl)phenyl)-6,7-dihydro-5H-benzo[7]annulen-3-carbonsäure, (14)
- (R,E)-8-(2,4-Dichlorphenyl)-9-(4-((1-(4-(dimethylamino)-4-oxobut-2-en-1-yl)pyrrolidin-3-yl)oxy)phenyl)-6,7-dihydro-5H-benzo[7]annulen-3-carbonsäure, (15)
- (S,E)-8-(2,4-Dichlorphenyl)-9-(4-((1-(4-(dimethylamino)-4-oxobut-2-en-1-yl)pyrrolidin-3-yl)amino)phenyl)-6,7-dihydro-5H-benzo[7]annulen-3-carbonsäure, (16)
- (R,E)-8-(2,4-Dichlorphenyl)-9-(4-((1-(4-(dimethylamino)-4-oxobut-2-en-1-yl)pyrrolidin-3-yl)amino)phenyl)-6,7-dihydro-5H-benzo[7]annulen-3-carbonsäure, (17)
- (E)-8-(2,4-Dichlorphenyl)-9-(4-((1-(4-(dimethylamino)-4-oxobut-2-en-1-yl)pyrrolidin-3-yl)methyl)phenyl)-6,7-dihydro-5H-benzo[7]annulen-3-carbonsäure, Isomer 1, (18)
- (E)-8-(2,4-Dichlorphenyl)-9-(4-((1-(4-(dimethylamino)-4-oxobut-2-en-1-yl)pyrrolidin-3-yl)methyl)phenyl)-6,7-dihydro-5H-benzo[7]annulen-3-carbonsäure, Isomer 2, (19)
- 8-(2,4-Dichlorphenyl)-9-(4-((Z)-(1-((E)-4-(dimethylamino)-4-oxobut-2-en-1-yl)pyrrolidin-3-yliden)methyl)phenyl)-6,7-dihydro-5H-benzo[7]annulen-3-carbonsäure, (20)
- (E)-8-(2,4-Dichlorphenyl)-9-(4-((1-(4-(dimethylamino)-4-oxobut-2-en-1-yl)azetidin-3-yl)amino)phenyl)-6,7-dihydro-5H-benzo[7]annulen-3-carbonsäure, (21)
- (E)-8-(2,4-Dichlorphenyl)-9-(4-(2-((4-(dimethylamino)-4-oxobut-2-en-1-yl)amino)ethoxy)phenyl)-6,7-dihydro-5H-benzo[7]annulen-3-carbonsäure, (22)
- (S,E)-4-(3-(4-(8-(2,4-Dichlorphenyl)-3-hydroxy-6,7-dihydro-5H-benzo[7]annulen-9-yl)phenoxy)pyrrolidin-1-yl)-N,N-dimethylbut-2-enamid, (23)
- (S,E)-4-(2,4-Dichlorphenyl)-5-(4-((1-(4-(dimethylamino)-4-oxobut-2-en-1-yl)pyrrolidin-3-yl)oxy)phenyl)-2,3-dihydrobenzo[b]oxepin-8-carbonsäure, (24)
- (S,E)-6-(2,4-Dichlorphenyl)-5-(4-((1-(4-(dimethylamino)-4-oxobut-2-en-1-yl)pyrrolidin-3-yl)oxy)phenyl)-7,8-dihydronaphthalin-2-carbonsäure, (25)
- (S,E)-4-(3-(4-(8-(2,4-Dichlorphenyl)-6,7-dihydro-5H-benzo[7]annulen-9-yl)phenoxy)pyrrolidin-1-yl)-N,N-dimethylbut-2-enamid, (26)
- (S,E)-4-(3-(4-(4-(2,4-Dichlorphenyl)-8-hydroxy-2,3-dihydrobenzo[b]thiepin-5-yl)phenoxy)pyrrolidin-1-yl)-N,N-dimethylbut-2-enamid, (27).

17. Verfahren zur Herstellung einer Verbindung der Formel (I) oder (I') gemäß einem der Ansprüche 1 bis 5 und 7 bis 16 oder eines pharmazeutisch akzeptablen Salzes davon, bei dem man eine Verbindung der Formel 1F: 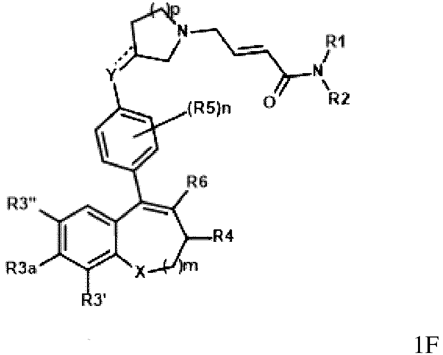 wobei R1, R2, R3', R3", R4, R5, R6, m, n, p, X, Y und 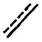 wie in einem der Ansprüche 1, 3 bis 5 und 7 bis 15 definiert sind und R3a ein Wasserstoffatom oder ein Carbonsäureester wie -COOMe, -COOEt oder geschütztes -OH wie beispielsweise O-Pivaloyl ist, in Gegenwart einer Quelle von Hydroxidionen wie NaOH oder LiOH in Lösung in THF oder Dioxan in eine Verbindung der Formel (I) oder (I') umwandelt.

18. Verfahren zur Herstellung einer Verbindung der Formel (I) oder (I') gemäß einem der Ansprüche 1 bis 5 und 7 bis 16 oder eines pharmazeutisch akzeptablen Salzes davon, bei dem man eine Verbindung der Formel 1F': 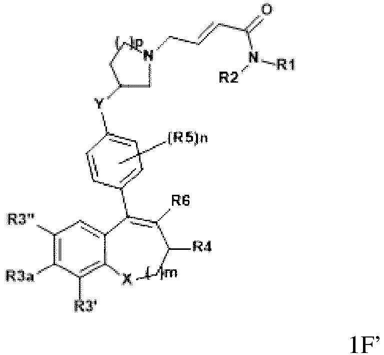 wobei R1, R2, R3', R3", R4, R5, R6, m, n, p, X und Y wie in einem der Ansprüche 1, 3 bis 5 und 7 bis 15 definiert sind und R3a ein Wasserstoffatom oder ein Carbonsäureester wie -COOMe, -COOEt oder geschütztes OH wie beispielsweise O-Pivaloyl ist, in Gegenwart einer Quelle von Hydroxidionen wie NaOH oder LiOH in Lösung in THF oder Dioxan in eine Verbindung der Formel (I) oder (I') umwandelt, wobei dem Schritt gegebenenfalls ein Schritt zum Erhalt von Verbindung 1F' vorausgeht, bei dem man eine Verbindung der Formel 1T: 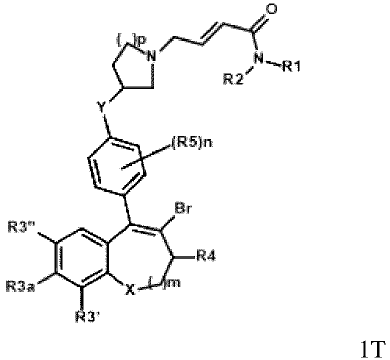
wobei R1, R2, R3', R3", R4, R5, m, n, p, X und Y wie in einem der Ansprüche 1, 3 bis 5 und 7 bis 15 definiert sind und R3a wie oben definiert ist,
einer Suzuki-Kupplung mit einem Boronsäure-Reagenz R6B(OR')₂ unterwirft, wobei -B(OR')₂ eine Boronsäure oder ein Pinacolatester ist und R6 wie in einem der Ansprüche 1, 14 oder 15 definiert ist.

19. Zwischenverbindungen, ausgewählt aus Verbindungen der Formel 1T, 1F, 1F' und 2B oder einem beliebigen pharmazeutisch akzeptablen Salz davon, 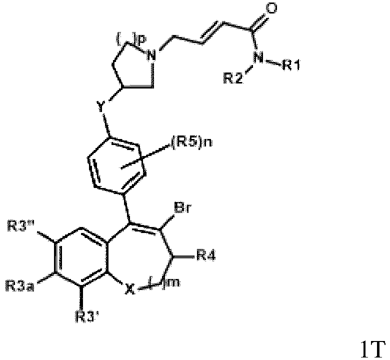 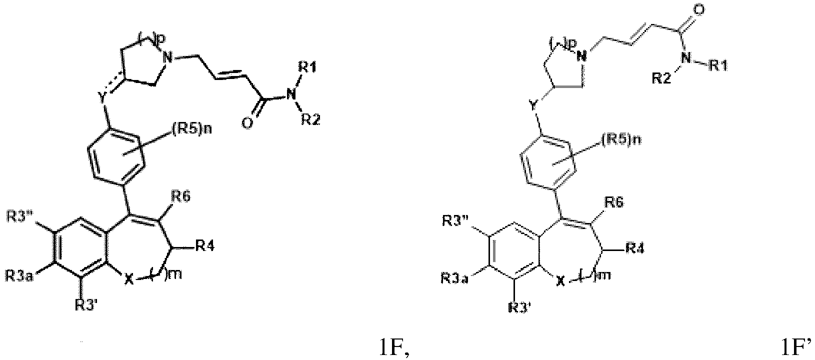 und 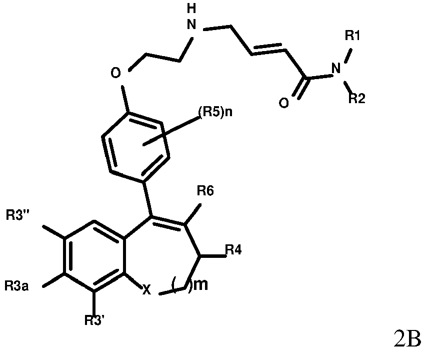 wobei R1, R2, R3', R3", R4, R5, R6, m, n, p, X, Y und 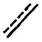 wie in einem der Ansprüche 1, 3 bis 5 und 7 bis 15 definiert sind und R3a ein Wasserstoffatom oder ein Carbonsäureester wie -COOMe, -COOEt oder geschütztes -OH wie O-Pivaloyl ist.

20. Zwischenverbindung der Formel 1E oder ein beliebiges pharmazeutisch akzeptables Salz davon: 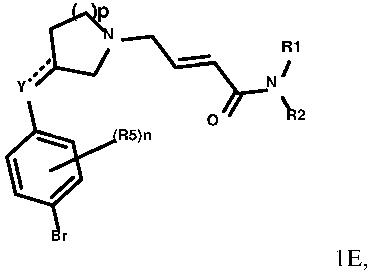 wobei R1, R2, R5, Y, n, p und 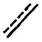 wie in einem der Ansprüche 1, 3 bis 5, 7 und 12 definiert sind.

21. Medikament, **dadurch gekennzeichnet, dass** es eine Verbindung der Formel (I) nach einem der Ansprüche 1 bis 16 oder ein pharmazeutisch akzeptables Salz davon umfasst.

22. Pharmazeutische Zusammensetzung, **dadurch gekennzeichnet, dass** sie eine Verbindung der Formel (I) nach einem der Ansprüche 1 bis 16 oder ein pharmazeutisch akzeptables Salz davon und mindestens einen pharmazeutisch akzeptablen Hilfsstoff umfasst.

23. Verbindung der Formel (I) nach einem der Ansprüche 1 bis 16 oder pharmazeutisch akzeptables Salz davon zur Verwendung bei der Behandlung von ovulatorischer Dysfunktion, Krebs, Endometriose, Osteoporose, benigner Prostatahypertrophie oder Entzündung.

24. Verbindung der Formel (I) zur Verwendung nach Anspruch 23 oder ein pharmazeutisch akzeptables Salz davon zur Verwendung bei der Behandlung von Krebs.

## Revendications

1. Composé de la formule (I) ou sel pharmaceutiquement acceptable correspondant : 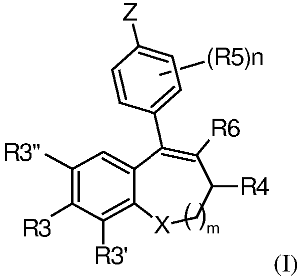
- R3 représentant un atome d'hydrogène, un groupe - COOH ou un groupe -OH ;
- R3' et R3" indépendamment représentant un atome d'hydrogène, un groupe méthyle, un groupe méthoxy, un atome de chlore, un atome de fluor, ou un groupe cyano ;
- R4 représentant un atome d'hydrogène, un groupe (C₁-C₃)alkyle ou un groupe cyclopropyle ;
- R5 indépendamment représentant un groupe (C₁-C₃)alkyle, tel qu'un groupe méthyle, un atome d'halogène, tel qu'un atome de fluor, un groupe cyano, ou un groupe (C₁-C₃)fluoroalkyle, tel qu'un trifluorométhyle ;
- R6 représentant un groupe choisi parmi :
■ un groupe phényle, ledit groupe phényle étant éventuellement substitué par 1 à 3 substituants indépendamment choisis parmi un atome d'halogène ; un groupe (C₁-C₆)alkyle, éventuellement substitué par un groupe cyano ou un groupe -OH ; un groupe (C₁-C₆) fluoroalkyle ; un groupe (C₃-C₆) cycloalkyle ; un groupe (C₁-C₆) alcoxy ; un groupe (C₁-C₆) fluoroalcoxy ; un groupe cyano ; un groupe trifluorométhylsulfonyle ; un groupe (C₁-C₄)alkylthio ; un groupe (C₁-C₄)fluoroalkylthio ; un groupe (C₁-C₄)alkylsulfonyle et un groupe -OH ;
■ un groupe phényle condensé, choisi parmi des groupes phényle condensés avec un (C₃-C₆)cycloalkyle, lequel cycle (C₃-C₆)cycloalkyle comprend éventuellement une insaturation et le groupe phényle condensé étant éventuellement substitué par 1 à 3 substituants indépendamment choisis parmi un groupe (C₁-C₃)alkyle, un groupe hydroxy, un atome d'halogène, un groupe (C₁-C₆)fluoroalkyle et un groupe (C₁-C₃) alcoxy ;
■ un groupe bicyclique comprenant 5 à 12 atomes de carbone, éventuellement comprenant 1 à 2 insaturations ; éventuellement substitué par 1 à 4 substituants indépendamment choisis parmi : un atome de fluor, un groupe -OH, un groupe (C₁-C₃)-alkyle, un groupe (C₁-C₃)fluoroalkyle, un groupe (C₁-C₃) alcoxy, un groupe (C₁-C₃)fluoroalcoxy et un groupe oxo ;
■ un groupe hétéroaryle comprenant 2 à 9 atomes de carbone et comprenant de 1 à 3 hétéroatomes indépendamment choisis parmi oxygène, azote et soufre, et au moins 5 atomes comprenant des atomes de carbone et des hétéroatomes, tel qu'un groupe pyridinyle ou un groupe pyrrolyle, ledit groupe hétéroaryle étant éventuellement substitué par 1 à 3 substituants indépendamment choisis parmi un atome d'halogène, un groupe (C₁-C₆) alkyle, un groupe (C₁-C₆)fluoroalkyle, un groupe (C₁-C₆) alcoxy, un groupe (C₁-C₆)fluoroalcoxy, un groupe cyano, un groupe carbamoyle et un groupe -OH ;
■ un groupe cycloalkyle comprenant 3 à 7 atomes de carbone, ledit groupe cycloalkyle étant saturé ou partiellement saturé et étant éventuellement substitué par 1 à 4 substituants indépendamment choisis parmi :
∘ un atome de fluor, un groupe -OH, un groupe (C₁-C₃) alkyle, un groupe (C₁-C₃) fluoroalkyle, un groupe (C₁-C₃) alcoxy, un groupe (C₁-C₃) fluoroalcoxy, un groupe oxo,
∘ un groupe (C₃-C₆) cycloalkyle et un groupe phényle, lesdits groupes (C₃-C₆) cycloalkyle ou phényle étant éventuellement substitués par un ou deux atomes d'halogène ou groupe (s) (C₁-C₃)alkyle ;
■ un groupe (C₃-C₆) cycloalkyl (C₁-C₃)alkyle, éventuellement substitué sur le cycloalkyle par 1 à 4 substituants indépendamment choisis parmi : un atome de fluor, un groupe -OH, un groupe (C₁-C₄)alkyle, un groupe (C₁-C₃)fluoroalkyle, un groupe (C₁-C₃)fluoroalcoxy et un groupe oxo ;
■ un groupe hétérocycloalkyle à 4 à 7 chaînons comprenant 1 ou 2 hétéroatomes indépendamment choisis parmi oxygène, azote et soufre, tel qu'un groupe tétrahydropyranyle ou un groupe tétrahydrofuranyle, ledit groupe hétérocycloalkyle étant saturé ou partiellement saturé et étant éventuellement substitué par 1 à 3 substituants indépendamment choisis parmi : un atome de fluor, un groupe (C₁-C₃)alkyle, un groupe (C₁-C₃) fluoroalkyle, un groupe (C₁-C₃)fluoroalcoxy, un groupe oxo, un groupe (C₁-C₃)alcoxy et un groupe -OH ;
■ un groupe (C₁-C₆)alkyle, tel qu'un groupe isobutyle ou un groupe éthylbutyle, ledit groupe alkyle étant éventuellement substitué par 1 à 4 substituants indépendamment choisis parmi : un atome de fluor, un groupe (C₁-C₃) alcoxy, un groupe (C₁-C₃) fluoroalcoxy et un groupe -OH ; et
■ un groupe phényl (C₁-C₂) alkyle, ledit groupe phényle étant éventuellement substitué par 1 à 3 substituants indépendamment choisis parmi un atome d'halogène ; un groupe (C₁-C₃)alkyle ; un groupe (C₁-C₃) fluoroalkyle ; un groupe (C₁-C₃)alcoxy ; un groupe (C₁-C₃) fluoroalcoxy ; un groupe cyano ; et un groupe -OH ;
- X représentant -CH₂-, -O- ou -S- ;
- n étant 0, 1 ou 2 ;
- m étant 0 ou 1 ; et
- Z représentant un groupe choisi parmi :
■ 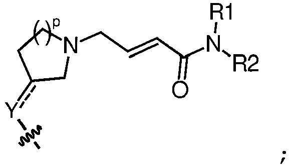 et
■ 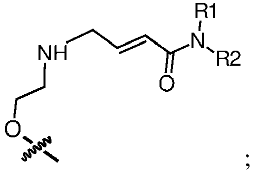
∘ R1 et R2 représentant indépendamment un atome d'hydrogène, un groupe -CH₃, un groupe -CH₂CH₃, un groupe -CH₂CH₂OH, ou R1 et R2 formant un groupe hétérocycloalkyle à 4 à 6 chaînons avec l'atome d'azote auquel sont fixés R1 et R2, ledit groupe hétérocycloalkyle comprenant éventuellement un hétéroatome supplémentaire choisi parmi oxygène, azote et soufre ;
∘ Y représentant -CH₂-, -CH=, -CR7=, -O- ou -NH-, R7 représentant un atome de fluor ou un groupe (C₁-C₃)alkyle
∘ 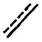 représentant une simple liaison ou une double liaison ; et
∘ p étant 0 ou 1.

2. Composé de formule (I) selon la revendication 1, ou sel pharmaceutiquement acceptable correspondant, **caractérisé en ce qu'**il est de formule (I') :

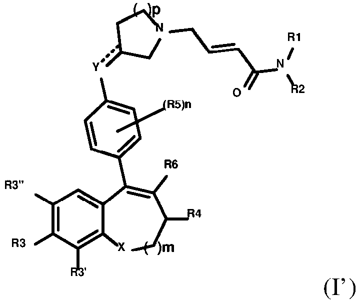

3. Composé de formule (I) ou (I') selon la revendication 1 ou 2, ou sel pharmaceutiquement acceptable correspondant, **caractérisé en ce que** Y représente -CH₂-, -CH=, -O- ou -NH-, et de préférence Y représente -O-.

4. Composé de formule (I) ou (I') selon l'une quelconque des revendications 1 à 3, ou sel pharmaceutiquement acceptable correspondant, **caractérisé en ce que** 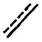 représente une simple liaison.

5. Composé de formule (I) ou (I') selon l'une quelconque des revendications 1 à 4, ou sel pharmaceutiquement acceptable correspondant, **caractérisé en ce que** p est 1.

6. Composé de formule (I) selon la revendication 1, ou sel pharmaceutiquement acceptable correspondant, **caractérisé en ce qu'**il est de formule (I'') :

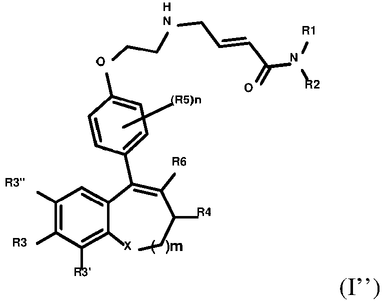

7. Composé de formule (I), (I') ou (I") selon l'une quelconque des revendications 1 à 6, ou sel pharmaceutiquement acceptable correspondant, **caractérisé en ce que** R1 et R2 sont un groupe -CH₃.

8. Composé de formule (I), (I') ou (I") selon l'une quelconque des revendications 1 à 7, ou sel pharmaceutiquement acceptable correspondant, **caractérisé en ce que** R3 est un groupe -COOH.

9. Composé de formule (I), (I') ou (I") selon l'une quelconque des revendications 1 à 8, ou sel pharmaceutiquement acceptable correspondant, **caractérisé en ce que** R3' et R3" représentent un atome d'hydrogène.

10. Composé de formule (I) , (I') ou (I") selon l'une quelconque des revendications 1 à 9, ou sel pharmaceutiquement acceptable correspondant, **caractérisé en ce que** R4 représente un atome d'hydrogène.

11. Composé de formule (I), (I') ou (I") selon l'une quelconque des revendications 1 à 10, ou sel pharmaceutiquement acceptable correspondant, **caractérisé en ce que** X représente -CH₂-.

12. Composé de formule (I), (I') ou (I") selon l'une quelconque des revendications 1 à 11, ou sel pharmaceutiquement acceptable correspondant, **caractérisé en ce que** n est 0.

13. Composé de formule (I), (I') ou (I'') selon l'une quelconque des revendications 1 à 12, ou sel pharmaceutiquement acceptable de celui-ci, dans lequel m est 1.

14. Composé de formule (I), (I') ou (I") selon l'une quelconque des revendications 1 à 13, ou sel pharmaceutiquement acceptable correspondant, **caractérisé en ce que** R6 représente un groupe phényle, ledit groupe phényle étant éventuellement substitué par 1 ou 2 substituants choisis indépendamment parmi un atome de chlore, un atome de fluor et un groupe méthyle.

15. Composé de formule (I), (I') ou (I") selon l'une quelconque des revendications 1 à 13, ou sel pharmaceutiquement acceptable correspondant, **caractérisé en ce que** R6 représente un groupe pyridinyle, ledit groupe pyridinyle étant substitué par deux substituants choisis indépendamment parmi un atome d'halogène, un groupe (C₁-C₆)alcoxy, et plus particulièrement choisi parmi un atome de fluor et un groupe méthoxy.

16. Composé de formule (I), (I') ou (I") selon l'une quelconque des revendications 1 à 15, ou sel pharmaceutiquement acceptable correspondant, en particulier sel de chlorhydrate correspondant, **caractérisé en ce que** ledit composé est choisi parmi les composés suivants :
- acide (S,E)-8-(2,4-dichlorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (1)
- acide (S,E)-8-(2-chloro-4-fluorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (2)
- acide (S,E)-8-(4-chloro-2-fluorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (3)
- acide (S,E)-8-(2-chloro-3-fluorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (4)
- acide (S,E)-9-(4-((1-(4-(diméthylamino)-4-oxobut-2-én-1-yl)pyrrolidin-3-yl)oxy)phényl)-8-(2-fluoro-4-méthylphényl)-6,7-dihydro-5H-benzo[7]annulène-3-carboxylique, (5)
- acide (S,E)-9-(4-((1-(4-(diméthylamino)-4-oxobut-2-én-1-yl)pyrrolidin-3-yl)oxy)phényl)-8-phényl-6,7-dihydro-5H-benzo[7]annulène-3-carboxylique, (6)
- acide (S,E)-8-(3-chloro-4-méthylphényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (7)
- acide (S,E)-9-(4-((1-(4-(diméthylamino)-4-oxobut-2-én-1-yl)pyrrolidin-3-yl)oxy)phényl)-8-(2,4-diméthylphényl)-6,7-dihydro-5H-benzo[7]annulène-3-carboxylique, (8)
- acide (S,E)-8-(2-chlorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (9)
- acide (S,E)-9-(4-((1-(4-(diméthylamino)-4-oxobut-2-én-1-yl)pyrrolidin-3-yl)oxy)phényl)-8-(2-fluoro-6-méthoxypyridin-3-yl)-6,7-dihydro-5H-benzo[7]annulène-3-carboxylique, (10)
- acide (S,E)-8-(2-chloro-4-méthylphényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (11)
- acide (E)-8-(2,4-dichlorophényl)-9-(4-((1-(4-(diméthylamino)-4-oxobut-2-én-1-yl)azétidin-3-yl)oxy)phényl)-6,7-dihydro-5H-benzo[7]annulène-3-carboxylique, (12)
- acide (E)-8-(2,4-dichlorophényl)-9-(4-((1-(4-(diméthylamino)-4-oxobut-2-én-1-yl)azétidin-3-yl)méthyl)phényl)-6,7-dihydro-5H-benzo[7]annulène-3-carboxylique composé avec l'acide 2,2,2-trifluoroacétique, (13)
- acide (E)-8-(2,4-dichlorophényl)-9-(4-((1-(4-(diméthylamino)-4-oxobut-2-én-1-yl)azétidin-3-ylidène)méthyl)phényl)-6,7-dihydro-5H-benzo[7]annulène-3-carboxylique, (14)
- acide (R,E)-8-(2,4-dichlorophényl)-9-(4-((1-(4-(diméthylamino)-4-oxobut-2-én-1-yl)pyrrolidin-3-yl)oxy)phényl)-6,7-dihydro-5H-benzo[7]annulène-3-carboxylique, (15)
- acide (S,E)-8-(2,4-dichlorophényl)-9-(4-((1-(4-(diméthylamino)-4-oxobut-2-én-1-yl)pyrrolidin-3-yl)amino)phényl)-6,7-dihydro-5H-benzo[7]annulène-3-carboxylique, (16)
- acide (R,E)-8-(2,4-dichlorophényl)-9-(4-((1-(4-(diméthylamino)-4-oxobut-2-én-1-yl)pyrrolidin-3-yl)amino)phényl)-6,7-dihydro-5H-benzo[7]annulène-3-carboxylique, (17)
- acide (E)-8-(2,4-dichlorophényl)-9-(4-((1-(4-(diméthylamino)-4-oxobut-2-én-1-yl)pyrrolidin-3-yl)méthyl)phényl)-6,7-dihydro-5H-benzo[7]annulène-3-carboxylique, isomère 1, (18)
- acide (E)-8-(2,4-dichlorophényl)-9-(4-((1-(4-(diméthylamino)-4-oxobut-2-én-1-yl)pyrrolidin-3-yl)méthyl)phényl)-6,7-dihydro-5H-benzo[7]annulène-3-carboxylique, isomère 2, (19)
- acide 8- (2,4-dichlorophényl)-9-(4-((Z)-(1-((E)-4-(diméthylamino)-4-oxobut-2-én-1-yl)pyrrolidin-3-ylidène)méthyl)phényl)-6,7-dihydro-5H-benzo[7]annulène-3-carboxylique, (20)
- acide (E)-8-(2,4-dichlorophényl)-9-(4-((1-(4-(diméthylamino)-4-oxobut-2-én-1-yl)azétidin-3-yl)amino)phényl)-6,7-dihydro-5H-benzo[7]annulène-3-carboxylique, (21)
- acide (E)-8-(2,4-dichlorophényl)-9-(4-(2-((4-(diméthylamino)-4-oxobut-2-én-1-yl)amino)éthoxy)phényl)-6,7-dihydro-5H-benzo[7]annulène-3-carboxylique, (22)
- (S,E)-4-(3-(4-(8-(2,4-dichlorophényl)-3-hydroxy-6,7-dihydro-5H-benzo[7]annulén-9-yl)phénoxy)pyrrolidin-1-yl)-N,N-diméthylbut-2-énamide, (23)
- acide (S,E)-4-(2,4-dichlorophényl)-5-(4-((1-(4-(diméthylamino)-4-oxobut-2-én-1-yl)pyrrolidin-3-yl)oxy)phényl)-2,3-dihydrobenzo[b]oxépine-8-carboxylique, (24)
- acide (S,E)-6-(2,4-dichlorophényl)-5-(4-((1-(4-(diméthylamino)-4-oxobut-2-én-1-yl)pyrrolidin-3-yl)oxy)phényl)-7,8-dihydronaphthalène-2-carboxylique, (25)
- (S,E)-4-(3-(4-(8-(2,4-dichlorophényl)-6,7-dihydro-5H-benzo[7]annulén-9-yl)phénoxy)pyrrolidin-1-yl)-N,N-diméthylbut-2-énamide, (26)
- (S,E)-4-(3-(4-(4-(2,4-dichlorophényl)-8-hydroxy-2,3-dihydrobenzo[b]thiépin-5-yl)phénoxy)pyrrolidin-1-yl)-N,N-diméthylbut-2-énamide, (27).

17. Procédé de préparation d'un composé de formule (I) ou (I') tel que défini selon l'une quelconque des revendications 1 à 5 et 7 à 16, ou d'un sel pharmaceutiquement acceptable de celui-ci, dans lequel un composé de formule 1F : 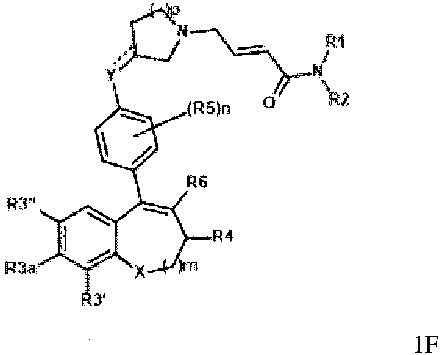 dans laquelle R1, R2, R3', R3", R4, R5, R6, m, n, p, X, Y et 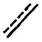 sont tels que définis dans l'une quelconque des revendications 1, 3 à 5 et 7 à 15, et R3a est un atome d'hydrogène ou un ester carboxylique tel que -COOMe, - COOEt, ou -OH protégé tel que O-pivaloyle par exemple, est converti en composé de formule (I) ou (I'), en présence d'une source d'ions hydroxydes tel que NaOH ou LiOH en solution dans du THF ou du dioxane.

18. Procédé de préparation d'un composé de formule (I) ou (I') tel que défini selon l'une quelconque des revendications 1 à 5 et 7 à 16, ou d'un sel pharmaceutiquement acceptable de celui-ci, dans lequel un composé de formule 1F' : 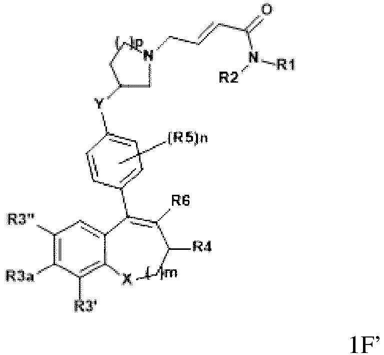 dans laquelle R1, R2, R3', R3", R4, R5, R6, m, n, p, X et Y sont tels que définis dans l'une quelconque des revendications 1, 3 à 5 et 7 à 15 et R3a est un atome d'hydrogène ou un ester carboxylique tel que -COOMe, - COOEt, ou OH protégé tel que O-pivaloyle par exemple, est converti en un composé de formule (I) ou (I'), en présence d'une source d'ions hydroxyde, telle que NaOH ou LiOH en solution dans le THF ou le dioxane, ladite étape étant éventuellement précédée par une étape pour obtenir un composé 1F', dans lequel un composé de formule 1T: 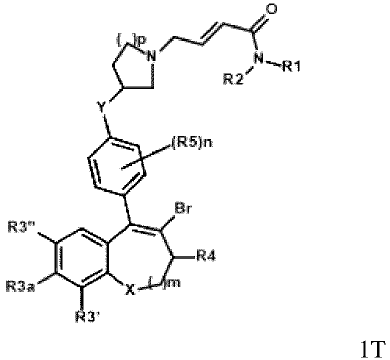
dans laquelle R1, R2, R3', R3", R4, R5, m, n, p, X et Y sont tels que définis dans l'une quelconque des revendications 1, 3 à 5 et 7 à 15 et R3a est tel que défini ci-dessus,
est soumis à un couplage de Suzuki avec un réactif boronique R6B(OR')₂, -B(OR')₂ étant un acide boronique ou un ester de pinacolate et R6 étant tel que défini dans l'une quelconque des revendications 1, 14 ou 15.

19. Composés intermédiaires choisis parmi les composés de formule 1T, 1F, 1F' et 2B, ou l'un quelconque de son sel pharmaceutiquement acceptable, 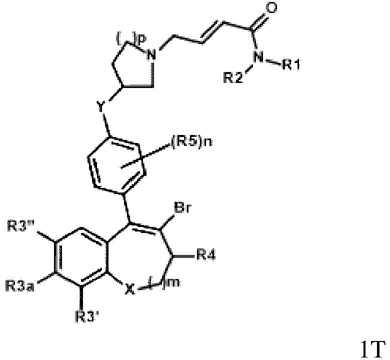 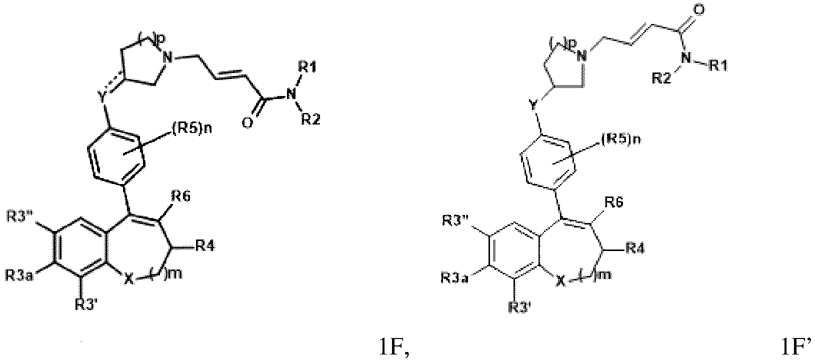 et 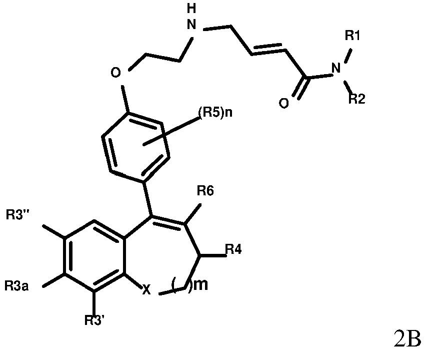 dans lesquelles R1, R2, R3', R3", R4, R5, R6, m, n, p, X, Y et 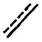 sont tels que définis dans l'une quelconque des revendications 1, 3 à 5 et 7 à 15 et R3a est un atome d'hydrogène ou un ester carboxylique tel que -COOMe, - COOEt, ou -OH protégé tel que O-pivaloyle.

20. Composé intermédiaire de formule 1E, ou l'un quelconque de son sel pharmaceutiquement acceptable : 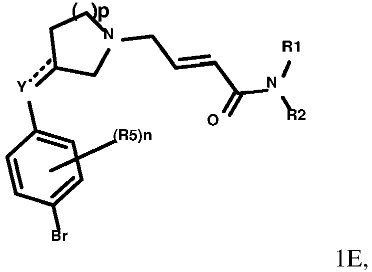 dans laquelle R1, R2, R5, Y, n, p et 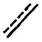 sont tels que définis dans l'une quelconque des revendications 1, 3 à 5, 7 et 12.

21. Médicament, **caractérisé en ce qu'**il comprend un composé de formule (I) selon l'une quelconque des revendications 1 à 16, ou un sel pharmaceutiquement acceptable correspondant.

22. Composition pharmaceutique, **caractérisée en ce qu'**elle comprend un composé de formule (I) selon l'une quelconque des revendications 1 à 16, ou un sel pharmaceutiquement acceptable correspondant, et au moins un excipient pharmaceutiquement acceptable.

23. Composé de formule (I) selon l'une quelconque des revendications 1 à 16, ou sel pharmaceutiquement acceptable correspondant, pour une utilisation dans le traitement d'un dysfonctionnement ovulatoire, d'un cancer, de l'endométriose, de l'ostéoporose, d'une hypertrophie prostatique bénigne ou d'une inflammation.

24. Composé de formule (I) pour une utilisation selon la revendication 23, ou sel pharmaceutiquement acceptable de celui-ci, pour une utilisation dans le traitement d'un cancer.
